# FASCICULE DE BREVET EUROPEEN

(11) **EP 2 234 620 B1**
(45) Date de publication et mention de la délivrance du brevet: **09.03.2016**
(21) Numéro de dépôt: 08873368.8
(22) Date de dépôt: 31.12.2008
(51) Int. Cl.: A61K 31/675, A61K 31/22, A61K 45/06, A61P 31/18

(54) **TRITHERAPIE UTILISABLE LORS DU TRAITEMENT D'UN PATIENT INFECTE PAR LE VIH**
TRITHERAPIE VERWENDBAR FÜR DIE BEHANDLUNG EINES HIV-PATIENTEN
TRITHERAPY USED TO TREAT A PATIENT INFECTED BY HIV

(30) Priorité: 03.01.2008 US 18688 P
(43) Date de publication de la demande: 06.10.2010
(73) Titulaire: Université d'Aix-Marseille, 13284 Marseille Cedex 07 (FR)
(72) Inventeur: CAU, Pierre, F-13540 Puyricard (FR); BOURGEOIS, Patrice, F-13127 Vitrolles (FR); BONNIOL, Vincent, F-13001 Marseille (FR); LEVY, Nicolas, F-13008 Marseille (FR)
(74) Mandataire: Novagraaf Technologies
(86) Numéro de dépôt international: PCT/FR2008/001844
(87) Numéro de publication internationale: WO 2009/115652

(56) Documents cités:
- EP-A- 1 127 573
- WO-A-02/00168
- WO-A-2004/024165
- WO-A-2004/050077
- WO-A-2005/021001
- WO-A-2008/003864
- WO-A-2009/112653
- US-A1- 2003 105 121
- US-A1- 2005 020 517
- US-A1- 2006 078 531
- MALLON PATRICK W G ET AL: "Effect of pravastatin on body composition and markers of cardiovascular disease in HIV-infected men--a randomized, placebo-controlled study." AIDS (LONDON, ENGLAND) 24 APR 2006, vol. 20, no. 7, 24 avril 2006 (2006-04-24), pages 1003-1010, XP002557095 ISSN: 0269-9370
- TODD T. BROWN,: "Selected Endocrine Topics in HIV: Osteoporosis and Adrenal Insufficiency" THE PRN NOTEBOOK,, [Online] vol. 12, décembre 2007 (2007-12), pages 1-7, XP002557096 Extrait de l'Internet: URL:http://www.prn.org/images/pdfs/85_brow n_todd.pdf>
- BAULCH-BROWN ET AL: "Inhibitors of the mevalonate pathway as potential therapeutic agents in multiple myeloma" LEUKEMIA RESEARCH, NEW YORK,NY, US, vol. 31, no. 3, 12 janvier 2007 (2007-01-12), pages 341-352, XP005828123 ISSN: 0145-2126
- VARELA IGNACIO ET AL: "Combined treatment with statins and aminobisphosphonates extends longevity in a mouse model of human premature aging." NATURE MEDICINE JUL 2008, vol. 14, no. 7, juillet 2008 (2008-07), pages 767-772, XP002557097 ISSN: 1546-170X cité dans la demande
- STEVEN GERARD CLARKE: 'HIV protease inhibitors and nuclear lamin processing: Getting the right bells and whistles' PNAS, [en ligne] 28 Août 2007, pages 13857 - 13858, XP055067094 Extrait de l'Internet: <URL:http://www.pnas.org/content/104/35/138 57.full.pdf#page=1&view=FitH> [extrait le 2013-06-18]
- RAMIREZ C L ET AL: "Human progeroid syndromes, aging and cancer: new genetic and epigenetic insights into old questions", CMLS CELLULAR AND MOLECULAR LIFE SCIENCES, BIRKHAUSER VERLAG, HEIDELBERG, DE, vol. 64, 1 January 2007 (2007-01-01), pages 155-170, XP002495855, ISSN: 1420-682X, DOI: 10.1007/S00018-006-6349-3 [retrieved on 2006-11-27]

## Description

### Demandes relatives

La présente demande est relative à la demande de brevet français N° FR 08/50019 déposée le 3 janvier 2008. Elle est également relative à la demande de brevet français N° FR 06/06097 déposée le 5 juillet 2006 et à sa demande PCT correspondante déposée le 5 juillet 2007 ayant pour titre « Médicament destiné au traitement des maladies avec persistance et/ou accumulation de protéines prénylées.»

### Description

### Domaine technique

L'invention se rapporte à une composition comprenant :
- au moins un inhibiteur de l'hydroxyméthylglutaryl-coenzyme A (HMG-CoA) réductase ou l'un de ses sels physiologiquement acceptable, ledit inhibiteur étant une molécule de la famille des statines ou l'un de ses sels physiologiquement acceptables
- au moins un inhibiteur de la farnésyl-pyrophosphate synthase ou de l'un de ses sels physiologiquement acceptable, ledit inhibiteur étant une molécule de la famille des aminobiphosphonates (NBP) ou l'un de ses sels physiologiquement acceptables, et
- au moins un agent anti-VIH, ledit agent anti-VIH étant un inhibiteur de protéase ou un inhibiteur de la transcriptase inverse.

La présente invention trouve une application dans le traitement des effets secondaires générés par un agent anti-VIH, par exemples le vieillissement précoce, la lipodystrophie qui peuvent être générés par les inhibiteurs de protéase ou les inhibiteurs de la transcriptase inverse.

Dans la description ci-dessous, les références entre parenthèses (X) renvoient à la liste de références à la fin des exemples. Les références entre parenthèse avec le nom de l'auteur et la date renvoient également à cette liste de références.

### Art antérieur

Le noyau des cellules eucaryotes est délimité par une double membrane percée de pores, l'enveloppe nucléaire, qui contrôle les échanges moléculaires entre les deux compartiments nucléaire et cytoplasmique. Cette enveloppe isole partiellement le contenu du noyau, c'est-à-dire le matériel génétique et toute la machinerie enzymatique nécessaire aux fonctions du génome nucléaire.

L'enveloppe nucléaire est constituée de 2 membranes concentriques, la membrane externe, en continuité avec le réticulum endoplasmique, et la membrane interne. Cette dernière est bordée sur sa face interne par un maillage fibrillaire dense appelé *lamina* nucléaire. Il s'agit d'un réseau protéique essentiellement composé de polymères de lamines et de protéines associées. Chez les vertébrés, on distingue deux sous-classes de lamines : les lamines de type A (lamines A et C), et de type B (lamines B1, B2 et B3) qui participent toutes à l'élaboration de la *lamina.* Cette dernière est maintenue en place par l'association avec d'autres protéines, fixées à la membrane interne de l'enveloppe nucléaire (pour revue, Gruenbaum & al. 2005 **(19)).**

Les lamines sont des protéines en forme de filament appartenant à la famille des filaments intermédiaires (type V) qui ont toutes une structure commune : un court segment globulaire N-terminal (tête) séparé d'un autre segment globulaire C-terminal (queue) par un long domaine central organisé en plusieurs hélices *alpha (rod domain).* La queue globulaire contient notamment un signal de localisation nucléaire (*NLS*) permettant l'adressage au noyau après la synthèse. Le domaine central permet l'association de deux molécules de lamine parallèles et leur organisation en filaments par association des dimères "tête-bêche". Cette structure leur confère des propriétés mécaniques très résistantes.

Seules la lamine A et les lamines B subissent une maturation après la synthèse d'un précurseur (pour revue, Gruenbaum & al. 2000 **(20)).** La lamine C est directement synthétisée sous sa forme mature.

Le précurseur de la lamine A et des lamines B se termine par un motif *CaaX* caractéristique (C est une cystéine, a un acide aminé à chaîne aliphatique non chargée et *X* un acide aminé quelconque, ici une méthionine, pour revue, Lévy & Cau 2003 **(29)).**

Le motif *CaaX* C-terminal permet la fixation d'un acide gras (en général, un acide gras en C15, farnésyle) grâce à une farnésyl-transférase. Cette prénylation (le motif farnésyle dérive d'une unité aliphatique de base en C5 appelée isoprène) permet aux prélamines de venir s'insérer dans la membrane du réticulum endoplasmique après leur synthèse dans le cytosol. Elles y subissent l'action d'une endoprotéase elle-même insérée dans la membrane d'enveloppe du réticulum et dont le site actif est cytosolique. L'endoprotéase spécifique de la prélamine A est Face1 (ou ZMPSTE24, *Zinc Metallo-Protease* homologue de STE24 de levure), tandis que Face2 (ou Rce1, *ras-converting enzyme*) est spécifique des prélamines B. Ces enzymes catalysent l'hydrolyse de la liaison peptidique entre la cystéine et l'aminoacide suivant (aliphatique), raccourcissant les prélamines de 3 acides aminés. L'extrémité carboxyle de la cystéine farnésylée est ensuite reconnue par une isoprénylcystéine-carboxyméthyl transférase (ICMT) qui vient y fixer un groupement méthyle par estérification.

Seule la maturation de la prélamine A se poursuit avec un deuxième clivage endoprotéolytique par Face1, qui libère un farnésyl-peptide de 15 acides aminés et la lamine A mature. Cette lamine A, qui ne comporte plus l'acide gras, devient soluble, est importée dans le noyau grâce à son signal de localisation nucléaire, et se localise dans la *lamina* nucléaire elle-même ainsi que dans le reste du compartiment nucléaire, constituant un véritable squelette nucléaire (Gruenbaum & al. 2005 **(19)).** La lamine B mature en revanche possède toujours à l'extrémité C-terminale sa cystéine farnésylée et méthylestérfiée. Elle reste donc insérée dans la membrane d'enveloppe du réticulum, puis dans la face nucléoplasmique de l'enveloppe nucléaire, d'où sa localisation exclusive à la *lamina* nucléaire, sous la membrane interne de l'enveloppe nucléaire où elle est ancrée.

Sous le terme de prénylation on entend la fixation au groupe thiol d'une cystéine soit d'une chaîne farnésyle de 15 atomes de carbone, on parle alors de farnésylation, soit d'une chaîne géranyl-géranyle de 20 atomes de carbone, on parle alors de géranyl-géranylation (Reid & al. 2004 **(39)**), soit encore de tout autre dérivé de l'isoprène.

La farnésylation, catalysée par la farnésyl-transférase (FTase) qui reconnaît la séquence consensus C-terminale (*CaaX*), fixe préférentiellement un groupement farnésyle sur le résidu cystéine du motif.

La géranyl-géranylation est la fixation par la géranylgéranyl-transférase (GGTase) d'un groupement géranyl-géranyle sur le résidu cystéine du motif.

Ces acides gras sont issus de la voie de biosynthèse, qui, à partir de l'hydroxyméthyl-glutaryl-Coenzyme A, est utilisée par les cellules pour fabriquer notamment le cholestérol, les stéroïdes, l'hème de l'hémoglobine et les ubiquinones (Hampton & al. 1996 **(20)**).

La famille des protéines prénylées comporte environ 300 membres dans le génome humain, dont la majorité est identifiable par le motif C-terminal *CaaX* (Reid & al. 2004 **(39)**). Les protéines des familles *Ras, Rho, Rab* (Leung & al. 2006 **(28)**) certaines protéines assurant une fonction d'import vers la mitochondrie (HDJ2), certaines protéines mitotiques (CENPE, CENPF) sont notamment prénylées (Winter-Vann & Casey 2005 **(51)**). En général, si dans le motif *CaaX* X est une sérine, une méthionine, une cystéine, une alanine ou un glutamate, l'isoprénoïde préférentiellement greffé est le farnésyle. Si X est une leucine, la reconnaissance du motif *CaaL* se fera préférentiellement par la GGTase, qui catalysera le transfert d'un groupement géranyl-géranyle (Basso & al. 2006 **(1)**). Il est probable que d'autres groupements dérivés de l'isoprène peuvent aussi être fixés sur cette cystéine, bien que cela ne soit pas décrit dans la littérature.

Chez l'homme, il existe trois gènes de lamines. Le gène *LMNA,* situé en 1q21.2-q21.3 (Wydner & al. 1996 **(52)**), donne par épissage alternatif les lamines A et C. Le gène *LMNA* est composé de 12 exons. Le début de l'exon 1 code l'extrémité globulaire N-terminale commune aux lamines A et C ; la fin de l'exon 1 et jusqu'au début de l'exon 7 codent la partie hélicoïdale centrale ; enfin, les autres exons codent l'extrémité globulaire C-terminale (Lévy & Cau 2003 **(29)**).

En fait, le gène code pour 4 produits épissés différemment, dont les lamines C et la prélamine A sont les 2 principaux (Lin & Worman 1993 **(31)**). La production différentielle des lamines A et C se fait par l'utilisation d'un site d'épissage alternatif au niveau de l'exon 10 du pré-messager, de telle sorte que la lamine C est codée par les exons de 1 à 10 et la lamine A est codée par les exons de 1 à 9, les 90 premières paires de bases de l'exon 10, et les exons 11 et 12 (lamine A-spécifiques).

Par conséquent, les peptides prélamine A et lamine C sont identiques au niveau des premiers 566 acides aminés, les extrémités C-terminales des lamines C et de la prélamine A contenant ensuite respectivement 6 et 98 acides aminés spécifiques.

Les lamines de type B comprennent trois protéines différentes (Shelton & al. 1981 **(43)**) : les lamines B1, B2 (les deux isoformes les plus représentées) et B3. Le gène *LMNB1* est situé en 5q23.3-q31.1 et comporte 11 exons codant la lamine B1 (Lin & Worman 1995 **(30)**). Le gène *LMNB2* est localisé en 19p13.3 et code pour les lamines B2 et B3 par un mécanisme d'épissage alternatif (Biamonti & al. 1992 **(2)**).

Les lamines de type B sont exprimées constitutivement dans toutes les cellules à partir des premiers stades de développement, tandis que les lamines de type A sont en général absentes dans les cellules souches embryonnaires (Stewart et al. 1987 **(45)**) et sont exprimées dans toutes les cellules somatiques différenciées. Leur expression est sujette à des régulations selon le tissu et au cours de la vie (Duque & al 2006 **(9)**). Il semble que leur expression ne soit pas nécessaire, puisque des souris chez lesquelles on a bloqué spécifiquement l'expression de lamine A, mais qui expriment tout de même la lamine C et les autres lamines, n'ont pas de phénotype apparent (Fong & al 2006 **(14)**).

Les lamines interagissent avec un nombre très élevé de partenaires protéiques ayant des fonctions très diverses; elles sont par conséquent impliquées dans un grand nombre de processus nucléaires, incluant la réplication et la réparation de l'ADN, le contrôle de la transcription et de l'épissage, l'organisation de la structure chromatinienne (pour revue, voir Shumaker & al. 2003 **(44)**, Zastrow & al. 2004 **(54)**, Hutchison & al. 2004 **(26)**, Gruenbaum & al. 2005 **(19)**). Les altérations de la structure de la *lamina* sont à l'origine de nombreuses pathologies héréditaires humaines. Elles sont dues à des mutations des gènes codant les lamines, ou d'autres protéines de la *lamina.* On a regroupé ces pathologies sous le terme générique de laminopathies (Broers & al. 2006. **(5),** Mattout & al 2006 **(33)**). Récemment, des mutations dans les gènes des enzymes responsables de la maturation des lamines (Face1 notamment), ont été identifiées, donnant lieu à des pathologies appartenant également au groupe des laminopathies (Navarro & al 2004 **(36)** et 2005 **(35)**).

A ce jour, la seule pathologie chez l'homme associée à des mutations des gènes *LMNA1* ou 2 est une leucodystrophie causée par une duplication complète du gène *LMNB1* (Padiath & al 2006 **(37)**). Un doute subsiste sur l'implication potentielle de variations de séquences trouvées dans *LMNB2* chez des patients atteints du syndrome de Barraquer-Simon (Hegele & al 2006 **(22)**). Cependant, il a été démontré *in vitro* par des expériences de RNAi (RNA-interférence) (Harborth & al. 2001 **(21)**), ainsi que chez le modèle murin (Vergnes & al. 2004 **(50),** que les lamines de type B sont essentielles pour le développement et l'intégrité cellulaire. En effet, la déficience en lamine B1 entraîne chez la souris une létalité périnatale. Par ailleurs, les noyaux des fibroblastes embryonnaires des mêmes souris *LMNB1* déficientes montrent des altérations remarquables de la morphologie nucléaire, proches de celles observées chez les patients porteurs de mutations du gène *LMNA.* De plus, il a été montré récemment que les lamines B sont nécessaires à la formation du fuseau de division pendant la mitose, ce qui tend à prouver que leur rôle est dynamique et multiple au cours du cycle cellulaire, et pas uniquement restreint au maintien de l'architecture du noyau (Tsai & al. 2006 **(48)**). Sur ce dernier rôle, un article récent constitue la démonstration de la fonction structurale des lamines B : des cellules artificiellement privées de lamines B1 ont un noyau "flottant" dans la cellule, qui tourne sur lui-même (Liu & al. 2007 **(45)**). La redondance fonctionnelle existant entre les deux lamines B1 et B2 est sans doute aussi le reflet direct de leur indispensabilité, exerçant une forte pression de sélection et masquant l'effet de mutations éventuelles de la séquence des gènes correspondants.

Les altérations fonctionnelles des lamines A/C, dues à des mutations du gène *LMNA,* sont à l'origine d'au moins 15 désordres regroupant des pathologies très diverses dans un spectre clinique allant de formes peu sévères, affectant un seul tissu de façon isolée, à des formes systémiques létales en période périnatale.

Nombre de mutations du gène *LMNA* modifient de façon notable l'assemblage des protéines dans l'enveloppe nucléaire et en perturbent le fonctionnement. Dans les cellules de divers tissus, la morphologie des noyaux est altérée : ils présentent souvent des hernies qui extrudent du matériel génétique dans le cytoplasme (Goldman & al. 2004 **(18)**).

Les protéines habituellement associées à l'enveloppe nucléaire, les lamines B, certaines protéines des pores nucléaires et les protéines LAP2, sont absentes du pourtour de ces hernies.

Ces anomalies morphologiques sont suivies d'altérations fonctionnelles, qui finissent par entraîner la mort cellulaire. Parmi l'ensemble des pathologies regroupées sous la dénomination de laminopathies, seules celles liées à l'accumulation anormale d'une forme prénylée de protéine sont concernées par la présente invention.

Ce sont principalement le syndrome de Hutchinson-Gilford, ou Progéria (De Sandre-Giovannoli & al. 2003 **(7)**, Eriksson & al. 2003 **(11)**), et la dermopathie restrictive (Navarro & al. 2004 **(36)**). Dans ces 2 syndromes, la cause physiopathologique est une accumulation et une persistance dans les cellules des malades de prélamine farnésylée non maturée.

La dermopathie restrictive, létale autour de la période natale, se caractérise par des signes cliniques qui sont presque tous la conséquence d'un déficit cutané qui restreint les mouvements *in utero*. Cette pathologie est très rare. La peau est rigide et tendue, elle cède par endroits, provoquant par exemple des déchirures au niveau des aisselles ou du cou. Les cils, les sourcils et le duvet cutané sont absents ou très clairsemés. Un hydramnios est souvent présent, et la diminution des mouvements foetaux est signalée dès le 6ème mois de grossesse. Au niveau du squelette, la radiographie révèle des contractures de toutes les articulations, des pieds en piolet, des clavicules fines, dysplasiques et bi-partites, des côtes en ruban, des os longs tubulaires des bras et une déminéralisation au niveau du crâne. La transmission de la dermopathie restrictive létale est récessive autosomique.

Des mutations de LMNA et de ZMPSTE24/Face1 ont été rapportées pour cette pathologie (Navarro & al. 2004 **(36)**). Dans les 2 cas, le mécanisme physiopathologique est le même : la prélamine A ne peut pas maturer (mutation nulle de Face1 ou disparition du site de clivage par mutation de la prélamine A), et reste farnésylée, et donc insérée dans la membrane nucléaire. L'accumulation et la persistance dans les cellules de ces précurseurs anormaux, qui empêchent probablement l'interaction normale des lamines B et C avec leurs partenaires, entraîne la mort des cellules et, à brève échéance, du patient. Il a été clairement démontré que c'est bien la persistance du groupement farnésyle, et non l'absence de lamine A mature, comme on pourrait le penser de prime abord, qui est responsable de la toxicité cellulaire (Fong & al. 2004 **(16)**).

En avril 2003, à partir d'un recoupement des symptômes communs à la dysplasie acromandibulaire et à certaines maladies se traduisant par un vieillissement prématuré, les inventeurs ont montré que la Progéria, la forme la plus typique et la plus grave de vieillissement précoce, résulte d'une mutation du gène *LMNA* (De Sandre-Giovannoli & al. 2003 **(7)**). Les enfants atteints par cette maladie, aussi nommée syndrome de Hutchinson-Gilford, souffrent d'un vieillissement accéléré, jusqu'à dix fois plus rapide que celui d'un individu normal, et ont une espérance de vie qui ne dépasse pas 13 ans. En Europe, un enfant sur environ six millions est touché. Les symptômes sont un vieillissement cutané, une calvitie, une réduction de la taille de la mâchoire et des problèmes liés à la vieillesse, par exemple, une raideur des articulations et des troubles cardio-vasculaires. Ces derniers, tels l'infarctus du myocarde ou l'athérosclérose, sont souvent la cause de la mort.

La mutation incriminée, située dans l'exon 11 du gène *LMNA,* active un site cryptique d'épissage du pré-ARNm, conduisant à un ARNm délété de 150 nucléotides (De Sandre-Giovannoli & al. 2003 **(7)**, Eriksson & al. 2003 **(11)**). Cet ARNm délété est traduit en une prélamine A anormale, la progérine, qui ne peut pas être maturée en lamine A normale : l'absence de 50 acides aminés de l'exon 11 comportant le site de reconnaissance de la protéase bloque le 2^{e} clivage de la progérine dont l'extrémité C-terminale conserve son groupement famésyle. Elle reste donc insérée dans la face nucléoplasmique de l'enveloppe nucléaire, qui présente les altérations caractéristiques, hernies du nucléoplasme dans le cytosol et anomalies de la répartition de l'hétérochromatine périphérique (Goldman & al. 2004 **(18)**). Ici encore, c'est la persistance du groupe famésyle, par ailleurs nécessaire pour l'ancrage à la membrane d'enveloppe du réticulum dans laquelle sont localisées certaines des enzymes responsables de la maturation (clivages, méthylation) qui est responsable de la toxicité cellulaire de la progérine (Fong & al. 2004 **(16)**).

Ces pathologies systémiques présentent la particularité d'être associées à l'apparition précoce de signes habituellement liés au vieillissement. Leur caractéristique physiopathologique commune est de générer une lamine prénylée, avec les conséquences décrites.

Deux études récentes ont montré qu'une réduction de l'accumulation intranucléaire de la prélamine farnésylée, tronquée ou non, prévient efficacement l'apparition du phénotype cellulaire. La première a été réalisée sur le modèle murin progéroïde déficient en protéase Face1 (Pendas & al. 2002 **(38)**). Lorsqu'elles sont croisées avec des souris exprimant moitié moins de Lamine A (souris *Lmna* +/-), les effets de l'absence de Face1 sont amoindris (Varela & al. 2005 **(49)**). La deuxième étude montre que le traitement de cellules de patients HGPS par des *morpholino* (oligonucléotides antisens) ciblant le site d'épissage cryptique abolit le phénotype mutant (Scaffidi & Misteli 2005 **(43)**).

Plusieurs études récentes (voir Scaffidi & Mistelli 2006 **(42)**) montrent l'implication de la lamine A dans le processus de vieillissement physiologique. En particulier, il a été démontré qu'au cours du vieillissement physiologique, la progérine est synthétisée par les cellules en l'absence de toute mutation du gène *LMNA* en raison de l'utilisation à bas bruit du site cryptique d'épissage de l'exon 11. Cette progérine se localise dans la *lamina,* à la périphérie du noyau des cellules. Le noyau de cellules de patients âgés «normaux» peut présenter des hernies caractéristiques d'une laminopathie causée par ces événements d'épissage accidentel, qui entraînent des anomalies des fonctions cellulaires et sont probablement au moins en partie responsables de leur vieillissement.

Dans la peau *in vivo,* la progérine est aussi synthétisée par une sous-population de fibroblastes dermiques et de kératinocytes, cellules dans lesquelles elle s'accumule avec l'âge. La progérine pourrait donc être un marqueur du vieillissement cutané (McClintock et al. 2007 **(34)**).

Il apparaît que des mécanismes moléculaires identiques sont d'une part responsables des signes de vieillissement prématuré des individus atteint de Progéria et d'autre part, à un niveau beaucoup plus faible, interviennent dans le vieillissement physiologique d'individus non porteurs de mutations.

Il existe dans l'art antérieur deux approches thérapeutiques décrites pour améliorer le phénotype cellulaire causé par la production pathologique de progérine. La première de ces solutions est tout simplement d'empêcher l'utilisation par le spliceosome de ce site d'épissage cryptique dans l'exon 11, en le "masquant" par traitement avec un oligonucléotide antisens (Scaffidi Misteli 2005 **(41)**), ou avec un rétrovirus produisant un siARN (Huang & al. 2005 **(25)**). Les résultats sont prometteurs *in vitro,* mais il s'agit ici de thérapie "génique", et le développement d'un médicament autour de cette approche est immanquablement long et compliqué, avec tous les inconvénients liés à la vectorisation des OAS pour obtenir un effet *in vivo.* La deuxième solution consiste à inhiber la farnésyl-transférase, l'enzyme qui catalyse le transfert du groupement famésyle sur les prélamines à partir de farnésyle-pyrophosphate. Lorsque de tels inhibiteurs (FTI) sont utilisés, une enveloppe nucléaire «normale» n'est que partiellement restaurée sur des cellules HGPS (Progéria) en culture, et la survie de souris RD (KO ZMPSTE24) est améliorée (Glynn & Glover 2005 **(17),** Capell & al. 2005 **(6),** Toth & al. 2005 **(47)**, Fong & al. 2006 **(15)**).

Cependant, le blocage de la farnésylation peut induire une géranyl-géranylation compensatoire (Bishop & al. 2003 **(3),** Varela & al. 2008 **(54 bis)**).

D'autre part, il a été rapporté récemment que les FTI induisent un arrêt du cycle cellulaire en bloquant le protéasome (Demyanets & al. 2006 **(8)**, Efuet & Keyomarsi 2006 **(10)**). Ainsi, le traitement induit sans doute une accumulation dans le nucléoplasme de progérine probablement ubiquitinylée, non dégradée par le protéasome.

Par ailleurs, des travaux récents rapportent que la diminution du taux de farnésylation de la progérine *in vivo* est très faible, de l'ordre de 5 % (Young & al. 2006 **(53)**), ce qui ne suffit pas à expliquer la restauration de la morphologie nucléaire observée *in vitro*.

Finalement, les FTI sont spécifiques d'une seule des voies de prénylation protéique, et ne peuvent être envisagés comme inhibiteurs globaux des prénylations post-traductionnelles.

Par ailleurs, il est rapporté que l'absence totale d'une des enzymes de cette voie, la mévalonate-kinase, est létale durant l'enfance (mutation homozygote perte de fonction du gène codant pour cette enzyme, syndrome rapporté par Hoffmann & al. 2003 **(24)**).

### Traitements anti-VIH et effets secondaires

1. Les patients infectés par le HIV et soumis à un traitement anti-rétroviral présentent les signes cliniques et biologiques d'un vieillissement accéléré comparable à celui présenté par les patients porteurs d'un syndrome progéroïde d'origine génétique.

Les traitements antirétroviraux, inhibiteurs de la transcriptase inverse, nucléosidiques (NRTI) ou non (NNRTI), inhibiteurs de la protéase virale (PI) ont permis un allongement de la durée de vie des patients infectés par le virus du SIDA chez lesquels apparaissent les conséquences du vieillissement « physiologique » (Casau, 2005; Levy et al., 2003).

Toutefois, l'infection elle-même et les traitements anti-rétroviraux font apparaître les mêmes signes cliniques et biologiques que ceux présentés par les patients atteints par un syndrome de vieillissement accéléré d'origine génétique (pour une revue récente de ces syndromes, voir (Navarro et al., 2006).

*Certaines des manifestations semblent directement liées à infection virale :*
Par exemple, l'hélicase mutée dans le syndrome de Werner (OMIM 277700), un syndrome de vieillissement prématuré associé à une prédisposition au cancer et à l'athérosclérose, recrute des cofacteurs protéiques cellulaires indispensables à la transactivation du LTR de HIV-1 et à la réplication du virus. La moindre disponibilité de l'hélicase dans les cellules infectées pourrait conduire au vieillissement et à l'immunosuppression (Sharma et al., 2007).

Par exemple encore les modifications du transport du cholestérol dans les macrophages. La protéine virale Nef inhibe les perméases de la famille ABC responsables de l'efflux du cholestérol (Bukrinsky and Sviridov, 2006; Mujawar et al., 2006; Wang and Rader, 2007). L'accumulation du cholestérol dans les macrophages les transforme en cellules spumeuses (foam cells) impliquées dans la constitution des plaques d'athérome dans les parois vasculaires (Pennings et al., 2006). Les médicaments anti-rétroviraux inhibent aussi l'efflux de cholestérol des macrophages et contribuent à la formation de la plaque d'athérome (Azzam et al., 2006; Dressman et al., 2003; Wang et al., 2007).

*De très nombreuses manifestations cliniques et biologiques semblent aussi* être *la conséquence des traitements anti-rétroviraux :*
Elles reproduisent les signes observés au cours des syndromes progéroïdes d'origine génétique, par exemple la progeria de Hutchinson-Gilford (OMIM 176670, voir (Hennekam, 2006), la dysplasie acromandibulaire (OMIM 248370) et la forme létale néonatale, la dermopathie restrictive (OMIM 275210) liées à des mutations dans le gène LMNA codant pour les lamines A et C ou à des mutations dans la protéase ZMPSTE24 (FACE1) responsable du clivage de la prélamine A au cours de sa maturation en lamine A :
   - L'alopécie (Torres et al., 2007; Wiwanitkit, 2004), indépendante des manifestations infectieuses dermatologiques (Maurer, 2005).
   - Les anomalies du système squelettique, en particulier l'ostéoporose (Brown and Qaqish, 2006; Thomas and Doherty, 2003) dont il a été proposé la correction par la vitamine D et un biphosphonate (Mondy et al., 2005)
   - La fonte musculaire (Restrepo et al., 2006; Restrepo et al., 2004; Tehranzadeh et al., 2004a; Tehranzadeh et al., 2004b), en relation avec le système ubiquitine-protéasome (Costelli and Baccino, 2003) ou les calpaïnes (Bartoli and Richard, 2005; Costelli et al., 2005), deux systèmes protéolytiques inhibés par certains traitements anti-rétroviraux (voir plus bas). Les mutations de la calpaïne 3 musculaire sont responsables d'une forme de dystrophie musculaire des ceintures (LGMD2A, OMIM 253600; (Richard et al., 1995) dont la myosite à éosinophiles pourrait être l'un des premiers signes (Krahn et al., 2006).
   - Une cardiomyopathie (Barbaro, 2003; Restrepo et al., 2006), indépendante des complications cardio-vasculaires (voir ci-dessous), en relation avec la toxicité mitochondriale des NRTI (Lewis, 2003).
   - Les anomalies cardiovasculaires (Mondy and Tebas, 2007) avec troubles du métabolisme lipidique (Hui, 2003; Jones et al., 2005; Moyle, 2007), athérome (de Saint Martin et al., 2006; Thomas and Smart, 2007; van Wijk et al., 2006), lésions des cellules endothéliales (Chen et al., 2005; Jiang et al., 2006; Zhong et al., 2002) et anomalies de la différenciation des cellules adipeuses (Kim et al., 2006; Roche et al., 2002). La dyslipidémie a pu être traitée par des statines (Benesic et al., 2004; Liang et al., 2006; Mallon et al., 2006) ou par un inhibiteur de l'absorption intestinale du cholestérol (Negredo et al., 2006). A noter que la pravastatine qui corrige en partie certains des paramètres altérés dans le syndrome métabolique (Yamagishi et al., 2006) induit une augmentation du tissu adipeux sous-cutané sans amélioration notable de la cholestérolémie (Gharakhanian et al., 2006; Mallon et al., 2006).
   - Des manifestations cliniques liées à une hypoandrogénie sont fréquemment observées chez les hommes (Cohan, 2006) et les cas de ménopause précoce chez les femmes séropositives font l'objet de plusieurs publications (Cohan, 2006; Ferreira et al., 2007).

*Les inhibiteurs de la protéase du virus (PI) présentent plusieurs cibles cellulaires dont des protéases :*
- L'inhibition du protéasome (Piccinini et al., 2005) a des conséquences très variées en raison du rôle de cet ensemble protéolytique sur de nombreuses fonctions cellulaires, turn-over des protéines, contrôle du cycle cellulaire, apoptose, transcription de gènes, transduction de signaux, sénescence, réponse au stress... (Naujokat et al., 2007). Par exemple, l'une des voies de blocage par les PI de la différenciation adipocytaire passe par la non-production du facteur de régulation de la transcription NFkB qui contrôle la transcription du gène codant pour la métalloprotéase (à zinc) MMP9 impliquée dans la différenciation adipocytaire (Bourlier et al., 2005; De Barros et al., 2007).
- Un autre exemple est la voie de signalisation mise en jeu par l'insuline (Rudich et al., 2005; Schutt et al., 2004). Les PI inhibent l'activité de l'insulin-degrading enzyme (Hamel et al., 2006), bloquent les canaux potassiques responsables de la sécrétion d'insuline (Neye et al., 2006), interagissent avec les transporteurs du glucose Glut4 (Hertel et al., 2004) et empêchent leur insertion à la membrane plasmique (Hruz, 2006; Parker et al., 2005).
- De même les PI bloquent la voie de signalisation *via* la kinase Akt mise en jeu par l'activation de récepteurs à activité tyrosine-kinase (Gupta et al., 2005), dont celui de l'IGF1. Or IGF1 contrôle directement l'atrophie ou l'hypertrophie musculaire (Glass, 2003).
- Les PI exercent un effet anti-apoptotique par inhibition des calpaines, protéases cytosoliques Ca⁺⁺-dépendantes (Ghibelli et al., 2003; Lichtner et al., 2006), et qui contrôlent l'équilibre apoptose-autophagie par le clivage de ATG5, indispensable à la formation de la vacuole autophagique (Yousefi et al., 2006).
- Deux autres systèmes enzymatiques sont bloqués par les PI : certaines cytochromes P450 de la sous-famille 3A, intestinaux ou hépatiques (Granfors et al., 2006), alors que d'autres cytochrome P450 de la sous-famille 2A sont induites (Yeh et al., 2006) en même temps que d'autres transporteurs (Dixit et al., 2007; Yeh et al., 2006) ; la glucuronoconjugaison dans le RE en particulier de la bilirubine (Zhang et al., 2005).
- L'inhibition de plusieurs activités enzymatiques dont le protéasome induit un stress du réticulum endoplasmique, le déclenchement de l'UPR (Unfolded Protein Response) et la mise en jeu du mécanisme de signalisation du réticulum au noyau avec l'apparition de facteurs de régulation de la transcription (Zhou et al., 2006). De nombreuses publications démontrent l'augmentation de la quantité des isoformes de SREBP (Sterol Response Element Binding Protein), qui contrôlent l'activation de gènes régulant le métabolisme lipidique dont celui du cholestérol (Colgan et al., 2007; Miserez et al., 2002; Nguyen et al., 2000; Williams et al., 2004; Zhou et al., 2006; Zhou et al., 2005). L'induction par les PI de la transcription de SREBP a aussi été montrée par une l'étude du transcriptome (puces, PCR quantitative) d'adipocytes en cours de différenciation (Pacenti et al., 2006) et l'effet est augmenté par l'absence de dégradation de SREBP par le système ubiquitine-protéasome.
   Les PI induisent l'accumulation nucléaire de SREBP dans les hépatocytes et les adipocytes et ses conséquences sur le métabolisme lipidique (synthèse augmentée d'acides gras et de cholestérol) (Hui, 2003; Riddle et al., 2001).
- Trois articles de la même équipe parisienne analysant la différenciation *in vitro* d'adipocytes montrent successivement que les PI entraînent un syndrome de résistance à l'insuline, la localisation anormale de SREBP dans le nucléoplasme, en relation avec une anomalie de la localisation de la lamine A (Caron et al., 2001) ; l'inhibition du clivage de SREBP (par les protéases golgiennes S-1P et S-2P, voir (Seidah et al., 2006), et ses conséquences sur la synthèse d'enzymes du métabolisme lipidique, sur la maturation anormale de la lamine A, alors que la maturation de la lamine B n'est pas modifiée (Caron et al., 2003) ; la similitude entre le stress mitochondrial observé au cours des lipodystrophies liées à une mutation du gène *LMNA* et celle résultant du traitement par les PI (Caron et al., 2007).
   L'un des intérêts de ces travaux est de suggérer fortement que certains PI bloquent la protéase (FACE1 ou ZMPSTE24) impliquée dans la maturation de la prélamine A, donnée qui vient d'être confirmée par une équipe américaine (Coffinier et al., 2007). Ces PI au contraire sont sans effet sur l'activité de la protéase FACE2 (ou Rce1, Ras converting enzyme 1), responsable du clivage des prélamines B mais aussi de celui de la protéine G monomérique Ras, clivage nécessaire à sa maturation (Wright and Philips, 2006). Une étude récente de RT-PCR quantitative montre que les PI induisent une diminution de la quantité des ARNm codant pour la lamine A sans modifier la quantité des ARNm codant pour la lamine C (Miranda et al., 2007).
- Les PI inhibent les protéases mitochondriales responsables du clivage des signaux d'adressage à la mitochondrie, du renouvellement des protéines mitochondriales, du contrôle de certaines GTPases mitochondriales (OPA1) impliquées dans la fusion des mitochondries et l'apoptose (Mukhopadhyay et al., 2002; Roehl White and Lauring, 2007).
- Indépendamment de leur action sur les calpaïnes (voir plus haut), les PI exercent un effet anti-apoptique pour les lymphocytes T en bloquant, *via* la protéine UCP2, la dépolarisation de la membrane interne induite par des stimuli pro-apoptotiques (Matarrese et al., 2003; Matarrese et al., 2005).
- Il est donc à noter que les PI inhibent donc des enzymes différentes de l'aspartyl-protéase spécifique du virus du SIDA (Dunn et al., 2002). Bien que la littérature soit à ce jour muette sur ce point, les PI pourraient aussi inhiber certaines des aspartyl-protéases eucaryotes (voir le site « Degradome » : http://www.uniovi.es/degradome/) comme la préséniline, la signal peptide peptidase, ou Ddi1 (DNA damage inducible protein), capable de fixer à la fois le protéasome et des protéines ubiquitinylées en vue de la dégradation de ces dernières (Sirkis et al., 2006).

*Les nucléosides inhibiteurs de la transcriptase inverse du virus (NRTI) ont aussi plusieurs cibles cellulaires dont la mitochondrie est l'une des plus importantes :*
- Les non-nucléosides inhibiteurs de la transcriptase inverse du virus, moins étudiés, semblent avoir une action plus spécifique sur l'enzyme virale tout en induisant l'apoptose d'une lignée de cellules T (Pilon et al., 2002).
- Les NRTI sont incorporés dans l'ADN nucléaire (Olivero et al., 1999) et leur effet mutagène peut entraîner un blocage du cycle cellulaire (Olivero et al., 2005). Il a été rapporté une mutation de l'ADN polymérase gamma (Yamanaka et al., 2007), responsable de la réplication et de la réparation de l'ADN mitochondrial (Hudson and Chinnery, 2006).
- Un petit nombre de publications montrent que les NRTI inhibent la N-glycosylation (dans le réticulum endoplasmique), la O-glycosylation et la modification des arborisations sucrées (dans le Golgi), en interférant avec les nucléotides transporteurs dans le Golgi des précurseurs des arborisations sucrées (Lizzi et al., 2007). Plusieurs dystrophies musculaires sont liées à des anomalies génétiques de la glycosylation (Muntoni et al., 2004; Percival and Froehner, 2007).
- Les NRTI sont transportés dans la matrice mitochondriale par une famille de transporteurs spécialisés dans celui des désoxynucléosides diphosphates, qui sont phosphorylés par une kinase mitochondriale avant d'être incorporés dans l'ADN mitochondrial par l'ADN polymérase gamma (Palmieri, 2004).
- La génotoxicité des NRTI a pour conséquence une augmentation du taux de mutations de l'ADN mitochondrial et une diminution du nombre de ses copies dans les mitochondries (Kohler and Lewis, 2007; Olivero, 2007).
- Les anomalies de l'ADN mitochondrial et celles du fonctionnement de l'ADN polymérase gamma ont des conséquences sur la synthèse des 13 protéines de la membrane interne constituant, avec des protéines codées par le génome nyucléaire et importées, les complexes de la chaîne respiratoire et l'ATP synthase. Les perturbations de l'ADN mitochondrial et de sa polymérase entraînent, par exemple, la diminution de la quantité de cytochrome oxydase Il (Vidal et al., 2006), la production d'espèces réactives de l'oxygène (ROS) (Jiang et al., 2007) avec des conséquences sur les hépatocytes, les adipocytes, les cardiomyocytes et les cellules endothéliales participant à la lipodystrophie et aux complications cardiovasculaires.
- L'augmentation du taux de l'acide lactique plasmatique est l'un des critères du dysfonctionnement mitochondrial induit par les traitements anti-rétroviraux (2007; John et al., 2001).
- Les NRTI inhibent par ailleurs l'activité de la télomérase et induisent un raccourcissement des télomères (Olivero, 2007; Yamaguchi et al., 2001).

En conclusion, les traitements anti-rétroviraux ont un impact sur des mécanismes et des voies métaboliques regroupés dans plusieurs des théories cellulaires tenant d'expliquer le vieillissement normal ou accéléré :
- la théorie « mitochondriale »,
- la théorie « noyau et lamine », apparue en 2003 avec la découverte que le gène LMNA est responsable de la progeria,
- la théorie « télomérique »,
- la théorie « régulation de la transcription des gènes » avec en particulier les protéines p53, NF-κkB
- la théorie « métabolique » avec implication de voies de signalisation dont certaines activées par des récepteurs membranaires.
- Plusieurs modèles animaux de vieillissement prématuré ou au contraire d'augmentation de la durée de vie font apparaître des recouvrements partiels entre les voies métaboliques mises en jeu dans chacune de ces théories et les interrelations, à l'échelle cellulaire, entre le cytosol et en particulier les mécanismes de dégradation des protéines (Grillari et al., 2006), la mitochondrie, le noyau et la membrane plasmique (Irminger-Finger, 2007; Kenyon, 2005; Martin and Loeb, 2004; Quarrie and Riabowol, 2004).

Plusieurs modèles animaux de vieillissement prématuré ou au contraire d'augmentation de la durée de vie font apparaître des recouvrements partiels entre les voies métaboliques mises en jeu dans chacune de ces théories et les interrelations, à l'échelle cellulaire, entre le cytosol et en particulier les mécanismes de dégradation des protéines (Grillari et al., 2006), la mitochondrie, le noyau et la membrane plasmique (Irminger-Finger, 2007; Kenyon, 2005; Martin and Loeb, 2004; Quarrie and Riabowol, 2004).

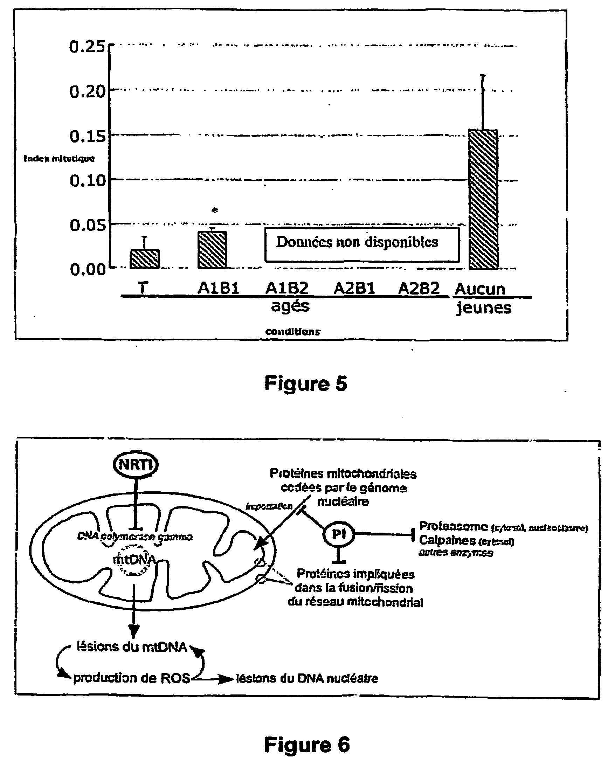

### 2. La théorie « mitochondriale » du viellissement (Figure 6 annexée).

Les trois principales composantes de la théorie mitochondriale du vieillissement sont i/ l'augmentation de la production d'espèces réactives de l'oxygène (ROS) par les complexes I et II de la chaîne respiratoire, ii/ les dysfonctionnements progressifs de celle ci et iii/ l'accumulation de lésions de l'ADN mitochondrial (Balaban et al., 2005; Meissner, 2007; Wallace, 2005).

L'ADN mitochondrial est plus sensible que l'ADN nucléaire aux effets des ROS qui ciblent l'ADN polymérase gamma (Graziewicz et al., 2002; Richter et al., 1988). Deux modèles murins exprimant une ADN polymérase gamma défective dans sa fonction proof-reading montrent un vieillissement accéléré avec mutations de l'ADN mitochondrial (Kujoth et al., 2005; Trifunovic et al., 2004). La toxicité mitochondriale des ROS peut être abolie par la surexpression de la catalase peroxysomale adressée à la mitochondrie dans un modèle murin transgénique, dont la durée de vie est augmentée (Schriner et al., 2005).

Chez l'homme, le vieillissement s'accompagne d'une baisse des fonctions mitochondriales, dont celles de la chaîne respiratoire (Short et al., 2005; Trifunovic et al., 2005), d'une augmentation de délétions dans l'ADN mitochondrial, tout particulièrement observées au cours de maladies neurodégénératives (Bender et al., 2006; Kraytsberg et al., 2006).

Un mécanisme de signalisation, encore trop mal connu, de la mitochondrie au noyau, fait intervenir la protéine p32, en particulier au cours du vieillissement ou des pathologies induites par les ROS (Jiang et al., 1999; Storz, 2006). Cette protéine, codée par le génome nucléaire puis importée dans la matrice mitochondriale, est réexportée vers le nucléoplasme (Brokstad et al., 2001) où elle contrôle la transcription (Chattopadhyay et al., 2004) et l'épissage (Petersen-Mahrt et al., 1999) d'ARNm. Elle interagit d'autre part avec le récepteur de la lamine B, localisé dans la face nucléoplasmique de l'enveloppe nucléaire (Mylonis et al., 2004; Simos and Georgatos, 1994).

Le mécanisme de signalisation du noyau à la mitochondrie fait intervenir les environ 1500 protéines codées par le génome nucléaire, qui sont synthétisées dans le cytosol puis importées dans la mitochondrie (Calvo et al., 2006; Truscott et al., 2003).

Parmi ces protéines, p53 est impliquée dans le vieillissement accéléré dans un modèle murin (Tyner et al., 2002) et participe aux dérégulations dans le cadre de la théorie « nucléaire » (voir ci-dessous). La protéine p53 contrôle la respiration mitochondriale (Matoba et al., 2006), exerce des effets pro- et anti-oxydants grâce au contrôle de la transcription de gènes nucléaires (Bensaad and Vousden, 2005; Sablina et al., 2005). Elle maintient la stabilité de l'ADN mitochondrial en interagissant avec l'ADN polymérase gamma (Achanta et al., 2005).

Enfin, les mécanismes de fission (division) et de fusion des mitochondrie, dans lesquels interviennent plusieurs GTPases des membranes mitochondriales externes (Drp1, les mitofusines...) et internes (OPA1) et plusieurs protéines associées (Fis1...) modulent la sénescence cellulaire : l'élongation du réseau mitochondrial par fusion permanente induit une sénescence cellulaire avec diminution de la différence de potentiel entre les deux faces de la membrane interne, augmentation de la production de ROS et lésions de l'ADN. Ces modifications phénotypiques sont neutralisées par la fragmentation du réseau mitochondrial (Lee et al., 2007).

### 3. Les mécanismes des syndromes de vieillissement accéléré d'origine génétique dans le cadre de la théorie « noyau et lamine »

Les lamines sont les protéines constitutives des filaments intermédiaires localisés exclusivement dans le nucléoplasme. Le gène LMNA code principalement pour les lamines A et C, produites par épissage alternatif. Deux autres gènes codent pour les lamines B1 et B2.

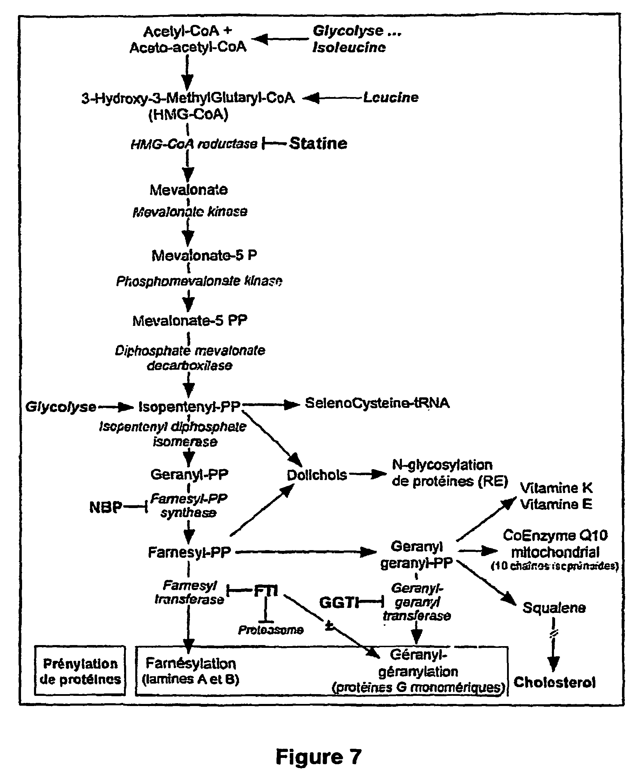

Les Lamines A et B sont synthétisées dans le cytosol sous la forme d'un précurseur qui subit plusieurs étapes de maturation. Les 3 protéines possèdent à leur extrémité C-terminale la boite *CaaX* (une cystéine, deux acides aminés aliphatiques, un acide aaminé indifférent). Cette séquence *CaaX* permet la farnésylation des lamines A et B (et d'environ 300 autres protéines du génome humain). Le résidu farnésyl (isoprénoïde de 15 atomes de carbone) est synthétisé à une étape intermédiaire de la voie de synthèse du cholestérol (Figure 7 annexée). Une enzyme cytosolique, la farnésyl-transférase, fixe le résidu farnésyl sur la cystéine.

La présence du résidu acide gras permet l'ancrage des prélamines A et B dans le feuillet cytosolique de la membrane d'enveloppe du réticulum endoplasmique (RE). Les prélamines A et B sont alors clivées par FACE1 (ZMPSTE24) ou FACE2 (Rce1) respectivement et perdent leurs trois derniers acides aminés C-terminaux aaX. Les protéines toujours ancrées dans la membrane d'enveloppe du RE où elles subissent l'action d'une deuxième enzyme, ICMT, qui fixe un groupement méthyl sur la cystéine déjà farnésylée.

La maturation des lamines B se termine à cette étape. Ces protéines glissent dans le plan de la membrane d'enveloppe du RE, puis de la face cytosolique de l'enveloppe nucléaire, traversent le pore nucléaire et se localisent dans le feuillet nucléoplasmique de l'enveloppe nucléaire où elles entrent dans la constitution de la *lamina* nucléaire et où elles interagissent avec plusieurs protéines, dont le récepteur de la lamine B. L'ancrage à l'enveloppe a pour conséquence l'absence de lamines B dans la matrice nucléaire, à l'intérieur du nucléoplasme et à distance de l'enveloppe.

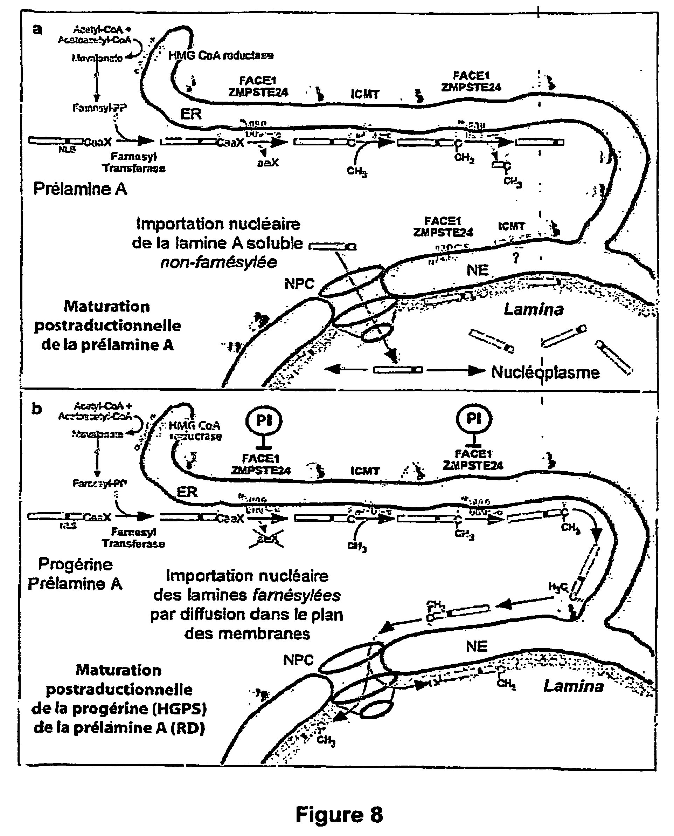

La prélamine A farnésylée et carboxyméthylée sur la même cystéine C-terminale subit une dernière étape de maturation par clivage protéolytique des 15 derniers acides aminés par FACE1 (et peut-être par Rce1). La lamine A perd son ancrage au RE, devient soluble, et est importée dans le nucléoplasme (comme la lamine C) au travers du pore nucléaire, comme toutes les protéines solubles, grâce à son signal de localisation nucléaire qui recrute le complexe d'importation nécessaire (Figure 8 annexée).

Dans le nucléoplasme, la lamine A (et la lamine C) se localise dans la *lamina* nucléaire, sous l'enveloppe nucléaire, où elle interagit avec de nombreuses protéines insérées dans le feuillet nucléoplasmique de l'enveloppe. Les lamines A et C (à la différence des lamines B) sont aussi dispersées dans le reste du nucléoplasme où elles entrent dans la constitution de la matrice nucléaire ou nucléeosquelette (Figure 6 annexée).

La matrice nucléaire contrôle le fonctionnement du génome nucléaire: réplication, réparation de l'ADN, transcription des ARN, épissage des ARNm, maturation des autres ARN... et ses constituants, dont les lamines A et C, interagissent avec de très nombreuses protéines importées dans le nucléoplasme (p53, SREBP...) comme l'ont montré les anomalies observées au cours des laminopathies d'origine génétique (Broers et al., 2006; Vlcek et al., 2001; Vlcek and Foisner, 2006). Le versant nucléoplasmique de l'enveloppe nucléaire et ses protéines transmembranaires ainsi que les lamines de la *lamina* contribue au stockage des facteurs de régulation de la transcription, avant leur utilisation et à la régulation de l'expression des gènes (Heessen and Fornerod, 2007; Shaklai et al., 2007).

Les premières laminopathies découvertes à partir de 1999 se présentaient comme des maladies prédominant dans un tissu donné, muscle squelettique, muscle cardiaque..., alors que les lamines sont des protéines ubiquistes (Vlcek and Foisner, 2007a; Vlcek and Foisner, 2007b; Worman and Bonne, 2007). Les lipodystrophies associent des anomalies du métabolisme des lipides et une insulino-résistance avec une trouble de la répartition corporelle du tissu adipeux, à rapprocher de la lipodystrophie observée au cours des traitements anti-rétroviraux (Capeau et al., 2005).

Les laminopathies « systémiques » ont été découvertes après 2002 : dysplasie acromandibulaire (OMIM 248370 ; (Agarwal et al., 2003; Novelli et al., 2002) ; progeria de Hutchinson-Gilford (OMIM 176670 ; (De Sandre-Giovannoli et al., 2003; Eriksson et al., 2003) ; dermopathie restrictive (OMIM 275210 ; (Navarro et al., 2005; Navarro et al., 2004). Elles intéressent l'ensemble des tissus de l'organisme des patients, peau et phanères, muscles squelettique et cardiaque, tissu osseux et cartilagineux, tissu adipeux et ses conséquences métaboliques, mais surtout elles s'accompagnent d'un vieillissement accéléré, dont le maximum est atteint avec la dermopathie restrictive, létale à la naissance. Ces trois maladies sont liées soit à une mutation dans le gène *LMNA* codant pour les lamines A et C, soit dans le gène *FACE1* (*ZMPSTE24*) codant pour la protéase du RE qui clive à deux reprises la prélamine A. La mutation du gène LMNA a pour conséquence de faire disparaître le site de reconnaissance par FACE1 de la prélamine A : la protéine n'est pas clivée, conserve donc son résidu farnésyl, est importée dans le nucléoplasme par glissement le long des membranes d'enveloppe comme le sont les lamines B (Pan et al., 2007). La prélamine A farnésylée reste ancrée dans la face nucléoplasmique de l'enveloppe nucléaire où elle perturbe l'organisation de la *lamina* et les interactions entre la *lamina* et les protéines membranaires de l'enveloppe, ce qui se traduit par des déformations nucléaires caractéristiques (Goldman et al., 2004).

Le reste de la matrice nucléaire est dépourvue de lamine A, avec des conséquences fonctionnelles multiples, par exemple, l'activation des voies en aval de p53 (Varela et al., 2005), des anomalies de la mitose (Cao et al., 2007; Dechat et al., 2007), de la réparation de l'ADN (Liu et al., 2005; Liu et al., 2006; Liu et al., 2007b)... conduisant à la sénescence cellulaire (Kudlow et al., 2007). L'analyse du transcriptome de cellules de patients progeria a montré des anomalies de l'expression de gènes impliqués dans l'athérosclérose (Csoka et al., 2004).

Il semble probable que le vieillissement accéléré présenté par les patients traités avec certains antirétroviraux, en particulier les PI qui inhibent ZMPSTE24 (FACE1), obéisse aux mêmes mécanismes.

La mutation du gène LMNA responsable de la progeria démasque un site cryptique d'épissage qui conduit à la synthèse d'un ARNm délété de 150 nucléotides, codant pour la progérine, une prélamine A délétée de 50 acides aminées et qui conserve son groupement farnésyl. Cette même protéine est produite au cours du vieillissement physiologique, en l'absence de toute mutation du gène *LMNA,* à la suite d'une erreur, liée à l'âge, de la machinerie d'épissage (Scaffidi and Misteli, 2006). La progerine s'accumule dans des fibroblastes dermiques localisés sous la lame basale, ainsi que dans des kératinocytes. Elle pourrait représenter un marqueur de la sénescence cutanée (McClintock et al., 2007).

Les modifications nucléaires (hernies du nucléoplasme dans le cytosol, effraction de l'enveloppe, irrégularités du contour nucléaire... ) observées dans les cellules de patients atteints de progeria ressemblent par ailleurs aux modifications transitoires induites par la protéine virale Vpr qui facilite l'entrée, par effraction transitoire de l'enveloppe nucléaire et de la *lamina,* du complexe de préintégration du virus du SIDA, complexe trop volumineux pour être importé dans le nucléoplasme via les pores nucléaires (de Noronha et al., 2001). Pour une revue récente sur ces mécanismes d'importation nucléaire (Suzuki and Craigie, 2007).

Il a été montré d'autre part que plusieurs protéines de la face nucléoplasmique de l'enveloppe nucléaire, dont l'émerine, mutée dans une forme de dystrophie musculaire d'Emery-Dreiffus, sont indispensables à la pénétration dans le nucléoplasme du virus du SIDA et à l'infection de cellules qui ne se divisent pas (Jacque and Stevenson, 2006).

Le terme de laminopathie génétique regroupe donc les pathologies résultant de mutations dans les gènes codant pour les lamines, tout particulièrement LMNA codant les lamines A et C, mais aussi celles provoquées par des mutations de protéines associées, dont celles qui participent à la maturation des lamines.

Les anomalies induites par les PI. au niveau de la matrice nucléaire représentent donc une des laminopathies, acquise, iatrogène. Qu'elles soient d'origine génétique ou acquises, ces anomalies de la matrice nucléaire ont pour conséquences une instabilité génomique et le vieillissement cellulaire (Mehta et al., 2007; Oberdoerffer and Sinclair, 2007).

### 4. La théorie « télomérique » du vieillissement

Le vieillissement cellulaire est contrôlé par une « horloge » mesurant la longueur des télomères, qui sont raccourcis à chaque mitose, un phénomène qui peut être contrebalancé par l'activité de la sous-unité catalytique de la télomérase (Gilson and Geli, 2007; Stewart and Weinberg, 2006).

Avant l'apparition de la PCR quantitative qui permet la mesure directe de la longueur de télomères en particulier dans les cellules sanguines mononucléées (Cawthon, 2002; Gil and Coetzer, 2004; Njajou et al., 2007), la mesure de l'activité de la télomérase (TRAP assay) a été réalisée sur les cellules de patients traités par aantirétroviraux, avec des résultats variables, en particulier selon le sous-type de lymphocyte T analysé (Effros, 2000).

Les NRTI inhibent l'activité de la télomérase (Olivero, 2007; Yamaguchi et al., 2001).

Les anomalies de la lamine A entraînent une baisse de l'activité de la télomérase, conduisant à un raccourcissement accéléré des télomères. Ces effets sont observés dans les cellules de patients atteints de progeria, que les cellules aient été ou non transfectées pour surexprimer la sous-unité catalytique de la télomérase (Wallis et al., 2004). Des résultats comparables ont été obtenus dans des fibroblastes de sujets sains surexprimant après transfection la lamine A, normale, mutée ou délétée comme dans la progeria (Huang et al., 2008). La matrice nucléaire et les protéines de l'enveloppe nucléaire qui lui sont associées au niveau de la *lamina* contrôlent donc directement ou indirectement l'activité de la télomérase (Pandita et al., 2007).

### 5. Certains facteurs de régulation de la transcription, en particulier p53 et NF-κB, sont impliqués dans le phénomène de vieillissement ou sa régulation.

Deux familles de protéines, p53 (Zafon, 2007) et NF-kB (Hayden and Ghosh, 2004), contrôlent la transcription de très nombreux gènes et sont impliquées en particulier dans le phénomène de vieillissement. Ces protéines participent aussi aux mécanismes décrits dans les autres théories du vieillissement et les relient entre eux.

A côté de ses fonctions bien caractérisées dans le contrôle du cycle cellulaire, la réponse aux stress, l'oncogenèse, la réparation des lésions de l'ADN (Fuster et al., 2007; Helton and Chen, 2007; Sengupta and Harris, 2005), p53 intervient dans le vieillissement cellulaire (voir par exemple : (Bauer and Helfand, 2006; Ben-Porath and Weinberg, 2005; Papazoglu and Mills, 2007; Sharpless and DePinho, 2002; Tyner et al., 2002). La protéine p53 est impliquée aussi dans les anomalies induites par les laminopathies (Varela et al., 2005), dans le fonctionnement de l'hélicase dont la mutation est responsable du syndrome de Werner (Brosh et al., 2001; Sommers et al., 2005), la régulation des télomères (Wynford-Thomas, 1996), le contrôle de la respiration mitochondriale (Matoba et al., 2006) indépendamment du rôle de p53 dans le déclanchement de l'apoptose par son interaction avec les molécules apoptogènes de la membrane externe (Manfredi, 2003; Mihara et al., 2003; Moll et al., 2005). p53 régule aussi la voie métabolique « insuline-IGF1-klotho » (voir ci-dessous).

NF-κB est aussi au carrefour de très nombreuses voies métaboliques. Son inactivation par ARN interférence dans des fibroblastes en culture les protège de la sénescence (Hardy et al., 2005). De même le blocage inductible de l'expression de NF-κB dans les cellules épidermiques de la souris ralentit leur vieillissement *via* les variations de l'expression de nombreux gènes analysés par microarray (Adler et al., 2007).

### 6. La théorie « métabolique » du vieillissement via la restriction calorique et les voies de signalisation de l'insuline, de l'IGF1 et de l'hormone klotho relie la membrane plasmique, le noyau et la mitochondrie

La restriction calorique induit l'augmentation de la durée de vie chez les rongeurs (Bordone and Guarente, 2005). Elle active des sirtuin déacétylases, qui ciblent à la fois les histones (avec un remodelage de la chromatine) (Vijg and Suh, 2006), de nombreuses autres protéines nucléaires (des facteurs de transcription dont p53, des protéines participant à la réparation de l'ADN...) mais aussi cytosoliques (tubuline) et mitochondriales (Guarente and Picard, 2005; North and Verdin, 2004; Porcu and Chiarugi, 2005).

La restriction calorique provoque la biogenèse des mitochondries via l'expression de la NO-synthase endothéliale, eNOS (Nisoli and Carruba, 2006; Nisoli et al., 2005), avec pour résultat l'augmentation de la production de ROS (Gredilla and Barja, 2005).

Les voies de signalisation de l'insuline et de l'IGF, impliquées dans le vieillissement (Bartke, 2005; Russell and Kahn, 2007) sont inhibées par la restriction calorique (Holzenberger et al., 2004; Masoro, 2004).

La restriction calorique inhibe aussi les voies de signalisation régulées par l'hormone klotho (Kurosu et al., 2005; Unger, 2006), dont les mutations s'accompagnent d'un vieillissement prématurée chez la souris (Kuro-o et al., 1997). Klotho est un régulateur de la réponse induite par le récepteur FGF-23 (Kurosu et al., 2006). Les mutations de klotho et de FGF-23 induisent le même phénotype de vieillissement prématuré, dont on sait qu'il est causé par une hypervitaminose D et qu'il est corrigé par la délétion d'un gène augmentant l'effet de la vitamine D (Razzaque and Lanske, 2006). Klotho bloque par ailleurs la réponse des récepteurs Wnt (Liu et al., 2007a), ce qui montre la diversité des voies métaboliques impliquées dans le vieillissement. Enfin, l'insuline stimule, *via* la phosphatidyl-inositol 3 kinase, le clivage du précurseur transmembranaire de klotho par des métalloprotéases de la famille ADAM et la libération de klotho dans le milieu extracellulaire (Chen et al., 2007).

La protéine p53 intervient aussi dans la régulation de ces voies métaboliques (Campisi, 2004; de Oliveira, 2006; Kim et al., 2007; Schmid et al., 2007).

La protéine p66^{shc} représente l'un des signaux de communication entre les récepteurs de la membrane plasmique pour l'insuline et pour l'IGF1, le noyau et la mitochondrie (Martin and Friedman, 2004). Les souris KO p66^{shc-/-} montrent une plus grande résistance au stress et une augmentation de leur durée de vie (Migliaccio et al., 1999). p66^{shc} est une cible de p53, localisée dans la mitochondrie dont elle contrôle le métabolisme et la production de ROS (Migliaccio et al., 2006; Nemoto et al., 2006; Orsini et al., 2004; Trinei et al., 2002). Une délétion de p66^{shc} inhibe le vieillissement des cellules souches cardiaques et prévient les accidents cardiaques, deux phénomènes induits par le diabète (Rota et al., 2006). p66^{shc} est aussi une enzyme redox. En réponse à des stress cellulaires, p66^{shc} est importé dans la mitochondrie et se localise dans l'espace intermembranaire où il synthétise H₂O₂ (soit 30% de l'H₂O₂ cellulaire, le reste étant produit par le peroxysome) en utilisant des électrons cédés par le cytochrome C (Giorgio et al., 2005). H₂O₂ pourrait représenter un signal intracellulaire de régulation de la durée de vie (Giorgio et al., 2007).

### Exposé de l'invention

Après de longues recherches, les inventeurs ont montré que l'association d'un inhibiteur de l'hydroxyméthylglutaryl-coenzyme A réductase (famille des statines) et d'un inhibiteur de la farnésyl-pyrophosphate synthase (famille des amino-biphosphonates, NBP), ou de l'un de leurs sels physiologiquement acceptable, est un traitement efficace des situations, pathologiques ou non, liées à l'accumulation et/ou la persistance dans les cellules de protéines prénylées, en ce sens qu'elle agit sur l'ensemble de la voie de prénylation des protéines, tant en C15 qu'en C20 ou dans les formes non caractérisées. Les inventeurs ont en outre constaté que l'association d'un inhibiteur de l'hydroxyméthylglutaryl-coenzyme A (HMG-CoA) réductase et d'un inhibiteur de la farnésyl-pyrophosphate synthase présente un effet additif dans la restauration du phénotype normal chez des fibroblastes de patients atteints de Progéria et chez des souris présentant un modèle de dermopathie restrictive. L'effet de l'association est nettement supérieur à ce qu'est l'effet de l'un ou l'autre des inhibiteurs utilisé individuellement (Varela et al., 2008 **(54 bis)**).

L'utilisation de l'association sur des cellules de patients atteints de Progéria conduit à une inhibition de la prénylation des protéines, donc à l'apparition de prélamine A non farnésylée, à l'amélioration des symptômes nucléaires et à la correction partielle des anomalies de la réparation de l'ADN. L'inhibition de la prénylation est aussi observée chez des souris présentant un modèle de dermopathie restrictive et elle restaure en partie la durée de vie des souris (Varela et al., 2008 **(54 bis)**).

Les traitements anti-rétroviraux ciblent plusieurs voies métaboliques et plusieurs compartiments cellulaires, le cytosol, le nucléoplasme, la mitochondrie.

Les effets secondaires de ces traitements reproduisent certains des signes cliniques et biologiques observés chez les pateints atteints de laminopathies génétiques causés par des mutations dans le gène *LMNA* ou dans le gène *ZMPSTE24.* Le point commun de ces mutations est de conserver le groupement farnésyl fixé à la lamine A ou à la prelamine A. Ce groupement farnésyl ancre la lamine A à la face nucléoplasmique de l'enveloppe nucléaire au niveau de la *lamina,* alors que le reste du nucléoplasme est dépourvu de lamine A soluble.

Ces deux anomalies de répartition de la lamine A entraînent des conséquences délétères au niveau de plusieurs voies métaboliques nucléaires (réplication et réparation de l'ADN, transcription de gènes, raccourcissement des télomères...), ont aussi des conséquences délétères dans d'autres compartiments cellulaires, dont la mitochondrie. Le dysfonctionnement général de la cellule cause son vieillissement accéléré et la réduction de sa durée de vie.

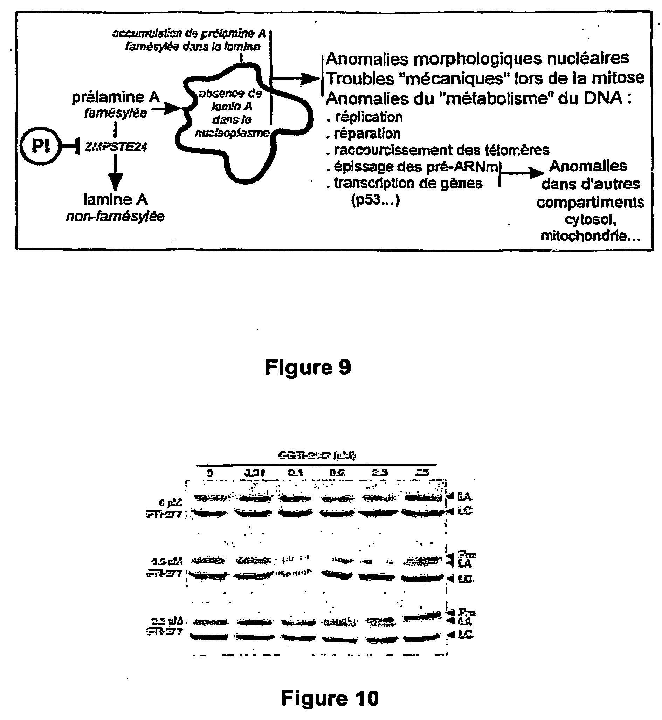

Une famille de traitements anti-rétroviraux, les inhibiteurs de protéase, inhibent ZMPSTE24, bloque la maturation de la prélamine A et provoque la persistance dans la cellule de prélamine A farnésylée. La figure 9 annexée représente schématiquement la théorie du vieillissement développée par les inventeurs de la présente et basée sur les anomalies de la maturation de la prélamine A et leurs conséquences fonctionnelles.

D'autre part, en plus les effets indirects des inhibiteurs de la protéase virale sur la mitiochondrie, une deuxième famille de médicaments anti-rétroviraux, les nucléosides inhibiteurs de la réverse transcriptase virale, perturbent directement la mitochondrie, un organite dont on sait que les anomalies de son fonctionnement sont associées au vieillissement.

L'objectif est donc d'analyser les mécanismes par lesquels ces deux types de traitement anti-rétroviral entraînent le vieillissement accéléré des patients infectés par le virus du SIDA et de vérifier que ces mécanismes sont comparables à ceux provoquant le viellissement accéléré des patients atteints de laminopathie génétique.

Les inventeurs de la présente en ont déduit que les effets secondaires décrits ci-dessus des traitements anti-rétroviraux peuvent être minimisés grâce à une composition comprenant ou un traitement combinant :
- Au moins un inhibiteur de l'hydroxyméthylglutaryl-coenzyme A (HMG-CoA) réductase ou de l'un de ses sels physiologiquement acceptable, et
- au moins un inhibiteur de la farnésyl-pyrophosphate synthase ou de l'un de ses sels physiologiquement acceptable.

La présente invention se rapporte donc à une composition comprenant :
- au moins un inhibiteur de l'hydroxyméthylglutaryl-coenzyme A (HMG-CoA) réductase ou l'un de ses sels physiologiquement acceptable, ledit inhibiteur étant une molécule de la famille des statines ou l'un de ses sels physiologiquement acceptables
- au moins un inhibiteur de la farnésyl-pyrophosphate synthase ou de l'un de ses sels physiologiquement acceptable, ledit inhibiteur étant une molécule de la famille des aminobiphosphonates (NBP) ou l'un de ses sels physiologiquement acceptables, et
- au moins un agent anti-VIH, ledit agent anti-VIH étant un inhibiteur de protéase ou un inhibiteur de la transcriptase inverse.

Cette composition peut être destinée par exemple au traitement des situations, pathologiques ou non, liées à l'accumulation et/ou la persistance dans les cellules de protéines prénylées. En particulier cette composition est utile pour le traitement d'un patient infecté par le VIH.

La présente décrit également à une composition dans laquelle l'agent anti-VIH est un agent anti-rétroviral ou un mélange d'agents anti-rétroviraux.

La présente invention se rapporte à une composition, dans laquelle l'agent anti-VIH est un inhibiteur de protéase ou un inhibiteur de la transcriptase inverse.

La présente invention se rapporte également à une composition selon l'invention, dans laquelle l'agent anti-VIH est un inhibiteur de protéase choisi dans le groupe comprenant fosamprenavir, lopinavir, ritonavir, amprenavir, atazanavir et indinavir.

La présente invention se rapporte également à une composition selon l'invention, dans laquelle l'agent anti-VIH est un inhibiteur de la transcriptase inverse choisi dans le groupe comprenant zidovudine, lamivudine, didanosine et epzicom.

Dans la composition décrite l'agent anti-VIH peut être une association d'une ou de plusieurs antiprotéases du virus et/ou d'un ou de plusieurs inhibiteurs de la transcriptase inverse du virus et/ou d'un ou de plusieurs inhibiteurs de l'entrée du virus dans les cellules et/ou d'un ou de plusieurs inhibiteurs de l'intégrase et/ou ou de tout autre traitement possédant un effet anti-viral, en particulier de tout traitement reconnu par les institutions réglementaires nationales et/ou internationales et la communauté scientifique.

La présente invention se rapporte également à une composition selon l'invention, dans laquelle on utilise des composés qui sont à la fois des inhibiteurs de l'hydroxyméthylglutaryl-coenzyme A (HMG-CoA) réductase et des inhibiteurs de la farnésyl-pyrophosphate synthase.

Très particulièrement, la composition selon l'invention est destinée au traitement d'un patient infecté par le VIH et développant des effets secondaires liés à l'accumulation et/ou la persistance dans les cellules de progérine ; encore plus particulièrement, au traitement des situations liées à l'accumulation et/ou la persistance dans les cellules de prélamine A farnésylée, que celle-ci soit ou non tronquée ou modifiée.

Dans la présente, des inhibiteurs de la farnésyl-pyrophosphate synthase, ou l'un de ses sels physiologiquement acceptables, peut être utilisé dans la préparation de la composition sont décrits.

Les sels physiologiquement acceptables décrits peuvent être par exemple des sels formés avec les acides chlorhydrique, bromhydrique, nitrique, sulfurique, phosphorique, les acides carboxyliques tels que par exemple les acides acétique, formique, propionique, benzoïque, maléique, fumarique, succinique, tartrique, citrique, oxalique, glyoxylique, aspartique, alcanes sulfoniques tels que les acides méthane ou éthane sulfoniques, arylsulfoniques tels que les acides benzène ou paratoluène sulfoniques.

Selon l'invention, l'inhibiteur de la farnésyl-pyrophosphate synthase est un des membres de la famille des aminobiphosphonates (NBP), ou l'un de ses sels physiologiquement acceptables.

Les polyphosphonates sont des molécules synthétiques très utilisées dans le traitement de l'ostéoporose et de la régénération osseuse.

Le terme de phosphonate s'applique à des molécules très semblables au phosphate :

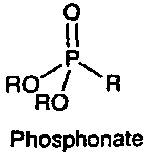

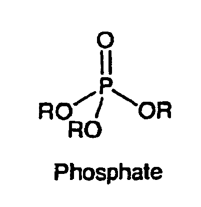

Le coeur des biphosphonates (BP) est l'équivalent d'une liaison PO-P comme dans l'ATP par exemple, mais où l'oxygène est remplacé par un carbone. Ceci confère une stabilité tout à fait particulière à ces molécules.

Un biphosphonate simple serait équivalent à l'ADP, les 2 groupes phosphates (O3P-) étant remplacés par le groupe biphosphonate.

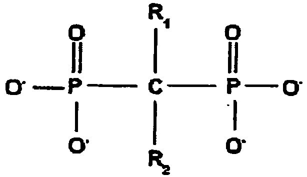

Le carbone central, à la différence de l'oxygène des phosphates, peut encore être impliqué dans 2 liaisons, et c'est la nature des groupements greffés sur ce carbone qui fait la spécificité des biphosphonates.

Lorsque les chaînes "latérales" (R1 et R2) comportent une fonction amine (NH), ou plus généralement un ou plusieurs atomes d'azote, on parle d'amino-biphosphonate, ou NBP.

Bien sûr, d'autres substituants peuvent être fixés sur les oxygènes.

L'acide pyrophosphorique, ou pyrophosphate en solution (PPi) 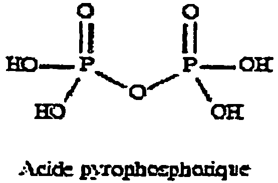 est utilisé dans de nombreuses réactions métaboliques comme transporteur de substrats, et il est restitué à la fin de la réaction. L'une des voies métaboliques utilisant des molécules couplées au pyrophosphate est celle de la prénylation protéique.

Le greffage d'un isopentènyl-PP (unité de base en C5) sur un géranyl-PP (C10) pour donner du famésyl-PP, réaction catalysée par l'enzyme farnésyl-pyrophosphate synthase (FPS), libère un PPi.

C'est cette étape qui est spécifiquement inhibée par les NBP.

A cet égard et à titre d'exemple, l'aminobiphosphonate (inhibiteur de la farnésyl-pyrophosphate synthase) peut être choisi parmi
- l'acide alendronique ou sa forme ionique, l'alendronate ;
- l'acide clodronique ou sa forme ionique, le clodronate ;
- l'acide étidronique ou sa forme ionique, l'étidronate ;
- l'acide ibandronique, ou sa forme ionique, l'ibandronate ;
- l'acide médronique ou sa forme ionique le médronate ;
- l'acide néridronique ou sa forme ionique, le néridronate ;
- l'acide olpadronique ou sa forme ionique, l'olpadronate ;
- l'acide pamidronique ou sa forme ionique, le pamidronate ;
- l'acide risédronique ou sa forme ionique, le risédronate ;
- l'acide tiludronique ou sa forme ionique le tiludronate;
- l'acide zolédronique ou sa forme ionique le zolédronate ;
- l'acide 4-N,N-diméthylaminométhane diphosphonique ou sa forme ionique le diméthylaminométhanediphosphonate ;
- l'α-amino-(4-hydroxybenzylidène) diphosphonate.

Préférentiellement selon l'invention, on préfère utiliser l'acide zolédronique (aussi appelé acide zolendronique) ou sa forme ionique, le zolédronate (aussi appelé zolendronate).

Dans la présente des inhibiteurs de HMG-CoA réductase, ou l'un de ses sels physiologiquement acceptables, peut être utilisé dans la préparation de la composition sont décrits.

Selon l'invention l'inhibiteur de HMG-CoA réductase est une molécule de la famille des statines, qu'elle soit liposoluble ou hydrosoluble, ou l'un de ses sels physiologiquement acceptables.

Les statines ont été mises en évidence chez des champignons. Elles ont une activité inhibitrice de l'HMG-CoA réductase, enzyme clé de la biosynthèse du cholestérol et des stéroïdes, qui catalyse la réduction de l'hydroxyméthyl-glutarate couplé au Coenzyme A en acide mévalonique (mévalonate en solution). Cette inhibition est assurée par leur analogie structurale avec le squelette de l'hydroxyméthylglutarate. La voie métabolique impliquée est certes celle de la biosynthèse du cholestérol, mais c'est aussi celle de la synthèse des groupes prényles, des polymères de l'unité de base à 5 carbones isoprène utilisés pour modifier environ 300 protéines dans les cellules et leur attacher une queue lipophile, permettant notamment leur ancrage dans les membranes.

Les principaux polyprènes, tous issus du pyruvate et de l'HMG-CoA, sont le géranyle (C10), le famésyle (C15) et le géranyl-géranyle (C20).

Toutes les statines sont globalement hépatosélectives, mais toutes n'ont pas le même mode d'entrée dans les cellules. En effet, pravastatine et rosuvastatine sont toutes 2 hydrophiles, donc hydrosolubles, au contraire de toutes les autres qui sont lipophiles, donc peuvent diffuser librement à travers les membranes plasmiques (bicouches lipidiques), ce qui explique sans doute leur plus haute toxicité. Les statines hydrosolubles ont besoin d'un transporteur spécifique pour entrer dans la cellule, *Organic Anion Transporter 3,* ou OAT3, ouSLC22A8 (Takedaa & al. 2004 **(46)**).

Elles sont très utilisées pour le traitement de l'hypercholestérolémie, et leurs effets secondaires, rares, sont bien caractérisés. Ce sont notamment des cas de rhabdomyolyse (1 à 7% des cas selon la molécule utilisée, Evans & al. 2002 **(12)**), dont le signe avant-coureur, des douleurs musculaires chez le patient traité, entraîne l'arrêt immédiat du traitement.

A cet égard et à titre d'exemple, une statine peut être choisie parmi l'atorvastatine, la simvastatine, la pravastatine, la rivastatine, la mévastatine (ou compactine), la fluindostatine, la vélostatine, la fluvastatine, la dalvastatine, la cérivastatine, la pentostatine, la rosuvastatine, la lovastatine, la pitavastatine, ou de l'un de leurs sels physiologiquement acceptables.

La lovastatine, la pravastatine et la simvastatine sont les molécules dérivées de métabolites fongiques, tandis que les autres, (atorvastatine, cérivastatine, fluvastatine, pitavastatine et rosuvastatine) sont entièrement synthétiques. Préférentiellement selon l'invention on utilise la pravastatine, une statine semi-naturelle hydrosoluble.

Bien entendu il est possible selon l'invention d'utiliser un, deux ou plusieurs inhibiteur(s) de la farnésyl-pyrophosphate synthase associés à un, deux ou plusieurs inhibiteur(s) de HMG-CoA réductase.

Selon une forme particulière de l'invention, la composition peut être destinée au traitement des effets secondaires du traitement anti-VIH, par exemple le vieillissement de la peau, la perte de poils et/ou de cheveux, l'ostéoporose et la lipodystrophie.

La présente invention a également pour objet la composition selon l'invention pour son application comme médicament dans le traitement des effets secondaires de vieillissement précoce généré(s) chez un patient par un traitement anti-VIH.

Selon l'invention, l'inhibiteur de la farnésyl-pyrophosphate synthase et l'inhibiteur de la HMG-CoA réductase sont avantageusement présents dans la composition à des doses physiologiquement efficaces.

De manière générale, les quantités à administrer peuvent être adaptées en fonction du patient, de la pathologie, du mode d'administration, etc. Il est entendu que des applications répétées peuvent être réalisées, éventuellement en combinaison avec d'autres ingrédients actifs ou tout véhicule.

En général la dose journalière des inhibiteurs sera la dose minimum pour obtenir l'effet souhaité.

Selon l'invention, l'inhibiteur de l'hydroxyméthylglutaryl-coenzyme A (HMG-CoA) réductase, l'inhibiteur de la farnésyl-pyrophosphate synthase, et l'agent anti-VIH peuvent être utilisés dans la composition, en mélange avec un ou plusieurs excipients ou véhicules inertes, c'est à dire physiologiquement inactifs et non toxiques. On peut citer par exemple les ingrédients habituellement utilisés dans les médicaments destinés au traitement de patients infectés par le VIH et accompagnant l'agent anti-VIH.

La composition de la présente invention peut également comprendre au moins un autre ingrédient actif, particulièrement un autre ingrédient thérapeutiquement actif, par exemple pour une utilisation simultanée, séparée ou étalée dans le temps suivant la formulation galénique utilisée. Cet autre ingrédient peut être par exemple un ingrédient actif utilisé par exemple dans le traitement de maladies opportunes qui peuvent se développer chez un patient infecté par le VIH.

La composition de la présente invention est une composition utilisable à la fois pour traiter un patient infecté par le VIH et prévenir et/ou traiter des désordres cutanés engendrés par l'utilisation de l'agent anti-VIH. Cette composition peut être utilisée au sein d'une thérapie multiple d'un patient infecté par le VIH. L'agent anti-VIH peut être unique ou multiple (plusieurs agent anti-VIH en mélange). Dans le cas d'une thérapie multiple (par exemple di-, tri- ou quadri-thérapie), le mélange d'inhibiteurs peut accompagner l'un ou plusieurs des agents anti-VIH.

L'agent anti-VIH peut également être une association d'une ou de plusieurs antiprotéases du virus et/ou d'un ou de plusieurs inhibiteurs de la transcriptase inverse du virus et/ou d'un ou de plusieurs inhibiteurs de l'entrée du virus dans les cellules et/ou d'un ou de plusieurs inhibiteurs de l'intégrase et/ou ou de tout autre traitement possédant un effet anti-viral, en particulier de tout traitement reconnu par les institutions règlementaires nationales et/ou internationales et la communauté scientifique.

Un procédé de traitement d'un patient infecté par le VIH comprenant l'administration d'une composition selon l'invention est décrit. La composition de l'invention est telle que définie ci-dessus.

Selon l'invention, l'administration peut être réalisée suivant toutes les voies connues de l'homme du métier pour l'administration d'une composition anti-VIH. Il peut s'agir par exemple d'une administration réalisée par voie orale ou par injection.

Comme indiqué ci-dessus, la dose administrée de la composition de l'invention dépend des besoins du patient et est également déterminée en tenant compte de ce qui est physiologiquement acceptable par le patient.

La quantité d'inhibiteurs dans la composition de la présente invention peut être telle qu'elle permet, à titre d'exemple, l'administration d'une dose d'inhibiteur de l'hydroxyméthylglutaryl-coenzyme A (HMG-CoA) réductase de 0,01 à 2 mg/kg de poids corporel et une dose d'inhibiteur de la farnésyl-pyrophosphate synthase de 0,01 à 40 mg/kg de poids corporel.

Selon l'invention, à titre d'exemple, dans la composition de l'invention, le rapport de l'inhibiteur de la farnésyl-pyrophosphate synthase sur l'inhibiteur d'hydroxyméthyl glutaryl coenzyme A réductase, ou de l'un de leurs sels physiologiquement acceptables, peut être compris entre 0,01 et 0,2, de manière préférée de 0,05 à 0,35.

La quantité d'agent anti-VIH dans la composition de la présente invention est déterminée de manière classique suivant les connaissances actuelles de l'homme du métier dans le traitement de patients infectés par le VIH. Elle peut être choisie par exemple parmi celles classiquement utilisées chez les patients infectés par le VIH.

Des exemples de concentration d'agent anti-VIH pour chacun des exemples d'agent anti-VIH décrit décrit dans la présente et chacun des mélanges ou association d'agents anti-VIH décrits dans la présente, sont donnés dans le Dictionnaire VIDAL (marque déposée), par exemple l'Edition 2007. Les concentrations indiquées dans ce Dictionnaire sont en outre celles authorisées pour l'humain.

La présente invention se rapporte également à un procédé de traitement des effets secondaires de vieillissement précoce et/ou de lipodystrophie généré(s) chez un patient soumis à un traitements anti-VIH, ledit procédé comprenant l'adiminstration d'un mélange comprenant au moins un inhibiteur de l'hydroxyméthylglutaryl-coenzyme A (HMG-CoA) réductase et au moins un inhibiteur de la farnésyl-pyrophosphate synthase est décrit.

La présente invention a également pour objet une composition selon l'invention pour son application comme médicament dans le traitement des effets secondaires de vieillissement précoce généré(s) chez un patient par un traitement anti-VIH.

Les conditions, quantités et voies d'administration peuvent être telles que décrites dans la présente, par exemple ci-dessus. L'agent anti-VIH est par exemple un angent anti-VIH ou une association tels que définis ci-dessus.

Une application du mélange d'inhibiteurs précités en tant qu'adjuvant de traitements ayant un effet iatrogène, par exemple un vieillissement anticipé, dont par exemple l'application "adjuvant aux thérapies anti-VIH comportant au moins une anti-protéase" qui particulièrement entraîne cet effet iatrogène est décrite. Ainsi, tout traitement engendrant les effets secondaires cités dans la présente est concerné par la présente.

La présente invention a également pour objet une composition selon l'invention pour son application comme médicament dans le traitement des effets secondaires de vieillissement précoce généré(s) chez un patient par un traitements anti-VIH utilisant un inhibiteur de protéase ou un inhibiteur de la transcriptase inverse.

La présente décrit également à un procédé de traitement d'un patient infecté par le VIH comprenant dans un ordre quelconque les étapes suivantes :
- administration d'un mélange comprenant au moins un inhibiteur de l'hydroxyméthylglutaryl-coenzyme A (HMG-CoA) réductase et au moins un inhibiteur de la farnésyl-pyrophosphate synthase,
- administration d'un agent anti-VIH,
dans lequel, les administrations sont concomitantes, successives ou alternatives.

La présente invention a également pour objet une composition pour son application comme médicament dans le traitement du VIH comprenant dans un ordre quelconque les étapes suivantes :
i. administration d'un mélange comprenant au moins un inhibiteur de l'hydroxyméthylglutaryl-coenzyme A (HMG-CoA) réductase, ledit inhibiteur étant une molécule de la famille des statines ou l'un de ses sels physiologiquement acceptables, et au moins un inhibiteur de la farnésyl-pyrophosphate synthase, ledit inhibiteur étant une molécule de la famille des aminobiphosphonates (NBP) ou l'un de ses sels physiologiquement acceptables
ii. administration d'un agent anti-VIH, ledit agent anti-VIH étant un inhibiteur de protéase ou un inhibiteur de la transcriptase inverse,
dans laquelle, les administrations sont concomitantes, successives ou alternatives.

Selon l'invention, ledit mélange et ledit agent anti-VIH peuvent être co-administrés. Cela rejoint le procédé de l'invention défini précédemment.

Selon l'invention, l'agent anti-VIH peut être tel que défini ci-dessus.

Selon l'invention, au moins une des administrations peut être réalisée par voie orale ou par injection. Les deux administrations peuvent être réalisées de la même manière ou différemment.

En d'autres termes, même si dans la présente description il est fait référence à une composition, on comprend bien que chacun des composés de la composition peut être administré concomittament aux autres composés (par exemple dans une seule composition ou dans deux compositions ou dans trois compositions, chacune de ces compositions comprenant un ou plusieurs des conmposants précités, le mode d'administration de chacun des composés ou composition(s) pouvant être identique ou différent), ou indépendamment les un des autres, par exemple successivement, par exemple administration indépendante d'un inhibiteur de l'hydroxyméthylglutaryl-coenzyme A (HMG-CoA) réductase, administration indépendante d'au moins un inhibiteur de la farnésyl-pyrophosphate synthase et adminstration indépendante d'un agent anti-VIH, ces administrations étant réalisées sur un même patient, concomittament ou successivement ou alternativement, dans un ordre qui est celui précité ou un autre ordre. Ces différentes administrations peuvent être réalisées, indépendamment l'une de l'autre ou de manière liée (composition ou co-administration), par un mode d'administration identique ou différent (injection, ingestion, application topique, etc.), une ou plusieurs fois par jour, pendant un ou plusieurs jours successifs ou non.

L'administration dudit mélange d'inhibiteurs peut être réalisée par exemple avec une dose d'inhibiteur de l'hydroxyméthylglutaryl-coenzyme A (HMG-CoA) réductase de 0,01 à 2 mg/kg de poids corporel et avec une dose d'inhibiteur de la farnésyl-pyrophosphate synthase de 0,01 à 40 mg/kg de poids corporel.

L'administration de l'agent anti-VIH peut être réalisée comme indiquée ci-dessus.

A titre d'exemple uniquement, des posologies sont décrites dans le tableau suivant pour la mise en oeuvre de l'un ou l'autre des procédés définis ci-dessus. Des exemples de quantités d'inhibiteurs et d'agent anti-VIH dans une composition conforme à la présente invention peuvent être déduites de ce tableau.

| | **Posologie 1** | **Posologie 2** | **voie** | **Fourchette large anti-VIH** |
|---|---|---|---|---|
| **Pravastatine** | 10 à 20 mg/j | 20 à 40mg/j | orale | 1 à 100 mg/J |
| **Simvastatine** | 10mg/j | 10 à 40mg/j | orale | 1 à 100mg/j |
| **Alendronate** | 10mg/j | 20 à 40mg/j | orale | 1 à 50mg/j |
| **Zolendronate** | 4mg/3s | soit 0,20mg/j | IV | 0,01 à 0,50 mg/j |
| **Pamidronate** | 15 à 90 mg/j | - | IV | 1 à 100mg/j |
| **clodronate** | 1600 mg/ | en deux fois | orale | 100 mg à 2g/j |

| | | | | |
|---|---|---|---|---|
| « IV » signifie par voie intraveineuse « j » signifie « jour ». Dose/j = dose/jour. | | | | |

Selon l'invention, les posologies concernant l'agent anti-VIH utilisables pour mettre en oeuvre la présente invention peuvent être celles connues de l'homme du métier. Il peut s'agir par exemple des posologies décrites dans le Dictionnaire VIDAL (marque déposée), par exemple dans l'Edition 2007. Pour chacun des exemples d'agent anti-VIH décrit ci-dessus et chacun des mélanges ou association d'agents anti-VIH décrits ci-dessus, on trouvera également, dans le Dictionnaire VIDAL (marque déposée), par exemple l'Edition 2007, des posologies utilisables pour mettre en oeuvre la présente invention.

D'autres avantages pourront encore apparaître à l'homme du métier à la lecture des exemples ci-dessous, donnés à titre illustratif et illustrés par les figures annexées.

### Brève description des figures

- La Figure 1 illustre les résultats obtenus en Western Blot sur des fibroblastes témoins "normaux" traités avec des doses croissantes d'une statine hydrosoluble (pravastatine P, 20 à 100 µM), et d'un aminobiphosphonate (NBP, zolédronate Z, 20 à 100 µM) (Pistes A à I, respectivement P20/Z20, P20/Z60, P20/Z100, P60/Z20. P60/Z60, P60/Z100, P100/Z20, P100/Z60, P100/Z100). La piste J est un contrôle positif de présence de prélamine A (fibroblastes de patients DR), la piste K est le contrôle négatif, traité avec le solvant seul (PBS).
- La Figure 2 illustre les résultats obtenus aux doses efficaces de chacun des produits.
- La Figure 3 illustre l'effet supérieur obtenu lors de l'administration des 2 produits ensemble.
- La Figure 4 illustre l'action de l'association des 2 produits sur des cellules âgées.
- La Figure 5 illustre la multiplication cellulaire des fibroblastes par mesure de l'index mitotique en fonction des conditions de culture cellulaire. L'index mitotique correspond au rapport entre le nombre de noyaux marqués (entrant en division) par rapport au nombre de noyaux totaux du champ observé en fonction de chaque traitement.
- Figure 6 : Représentation schématique de la théorie mitochondriale du vieillissement Les cibles mitochondriales des traitements antirétroviraux. Légende : NRTI : nucléosides inhibiteurs de la transcriptase inverse, PI : inhibiteur de la protéase, mtDNA : ADN mitochondrial et ROS : espèce réactive de l'oxygène
- Figure 7 : Représentation schématique de la voie de biosynthèse des isoprénoïdes et ses inhibiteurs.
   NBP : aminobiphosphonate
   FTI : inhibiteur de la farnésyl-transférase
   GGTI : inhibiteur de la géranyl-géranyl transférase
- Figure 8 : Représentation schématique de la maturation post-traductionnelle de la prélamine A, son importation nucléaire et sa localisation dans le nucléoplasme.
   **a** : prélamine A normale
   **b** : maturation de la prélamine A délétée dans la progeria (progérine) ou lors de mutation de la protéase ZMPSTE24 dans la dermopathie restrictive. Les inhibiteurs de la protéase du virus du SIDA (PI) inhibent ZMPSTE24 NPC :pore nucléaire
   NE : enveloppe nucléaire
   HGPS : Syndrome de la progéria d'Hutchinson-Gildford
   RD : dermopathie restrictive
- Figure 9 : Représentation schématique de la théorie du vieillissement basée sur les anomalies des lamines et leurs conséquences fonctionnelles. Légende : PI : inhibiteurs de la protéase virale.
- Figure 10: Western blot montrant que le blocage de la prénylation de la prélamine A nécessite à la fois l'inhibition de la farnésyl-transférase et de la géranyl-géranyl-transférase de type I. Détection de la lamine A/C dans des cellules HeLa traités par inhibiteurs de la farnésyl-transférase et/ou de la géranylgénamyl transférase de type I. LA = lamine A, LC = lamine C, Pre = prélamine A.

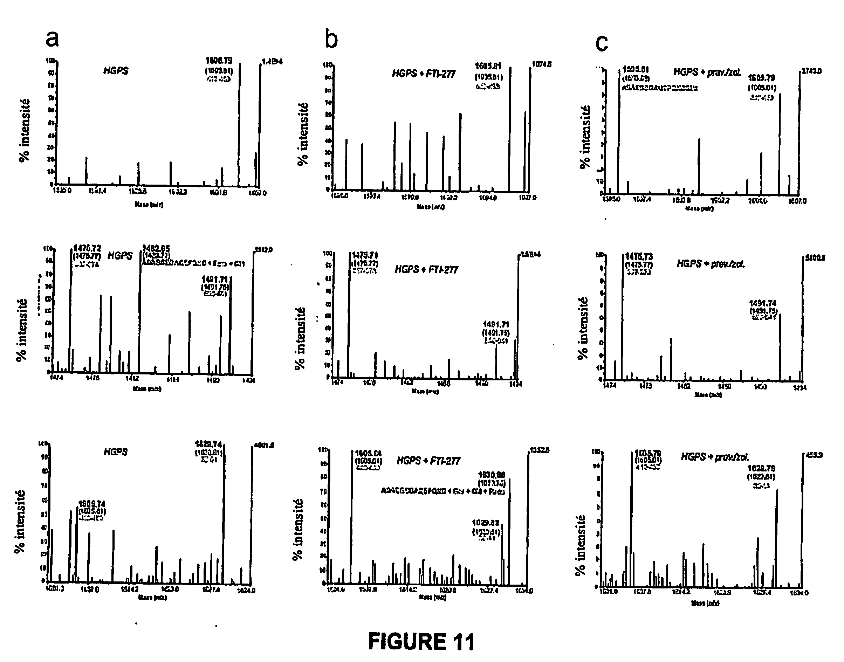

- Figure 11: Analyse par spectrométrie de masse de protéines extraites de l'enveloppe nucléaire de cellules non traitées (**a**), de cellules de patients progeria traitées par FTI (2,5 µM, **b**) ou traitées par le mélange pravastatine+zolédronate (1 µM chacun, **c**).

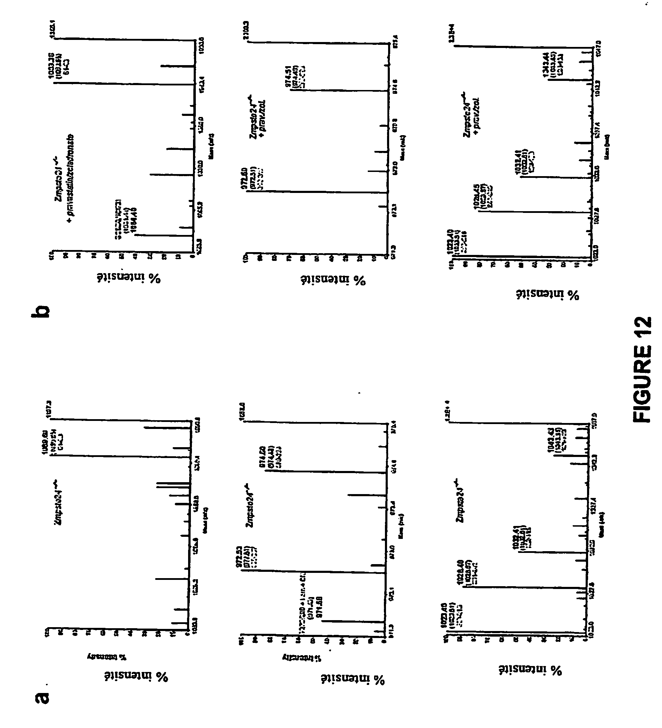

- Figure 12 : Analyse par spectrométrie de masse de protéines extraites de l'enveloppe nucléaire de fibroblastes non traitées (**a**) ou traitées par le mélange pravastatine+zolédronate (1 µM chacun, **b**) provenant de souris *Zmpste24*^{*-*/}*⁻.*

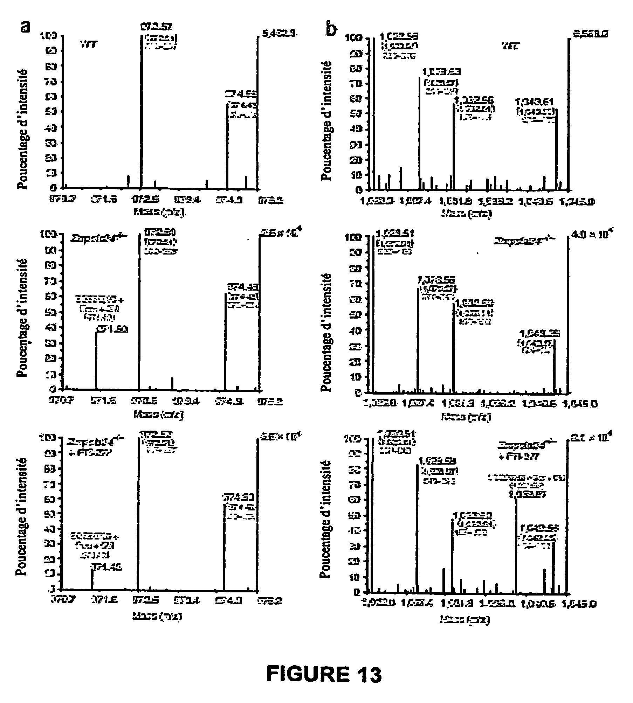

- Figure 13 : La lamine A (cellules témoins) et la prélamine A (cellules de souris *Zmpste24*^{*-*/*-*}) ont été analysées par spectrométrie de masse (MALDI-ToF). **a, b :** portions du spectre correspondant aux peptide tryptiques farnésylés (**a**) et géranylgéranylés (**b**).

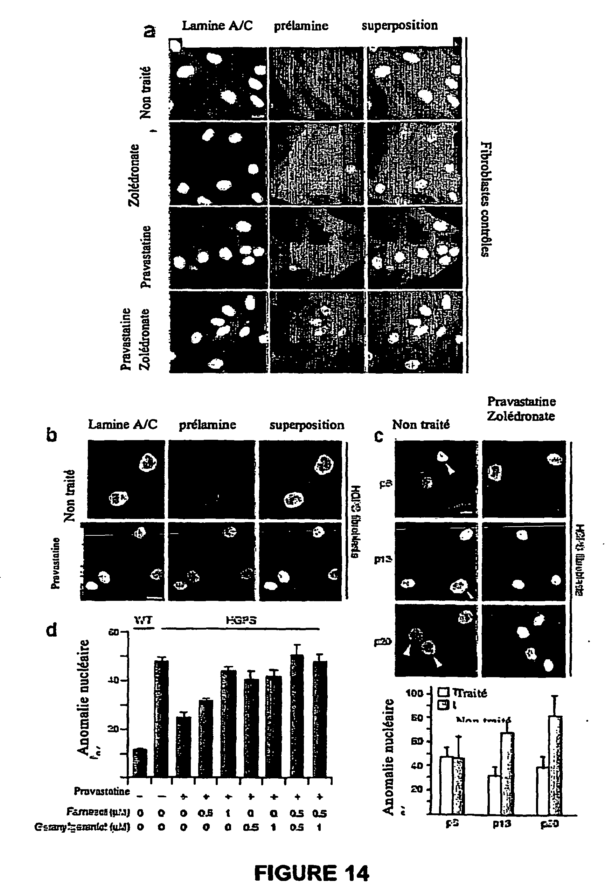

- Figure 14 : représentaions de résultats de différentes expérimetations démontrant l'effet synergique de la combinaison pravastatine+zolédronate sur l'accumulation de prélamine A dans des cellules témoins et de patients progeria : (**a**) Détection immunocytochimique de la lamine A/C et de la prélamine A dans des fibroblastes humains normaux, non traités, traités par pravastatine et/ou par zolédronate. (**b**) Détection immunocytochimique de la lamine A/C et de la prélamine A dans des fibroblastes humains normaux et de patients progeria traités par la combinaison pravastatine+zolédronate. (**c**) Analyse quantitative de l'effet du traitement pravastatine+zolédronate sur la morphologie nucléaire de cellules de patients progeria. (**d**) Analyse quantitative de l'effet du traitement pravastatine+zolédronate sur la morphologie nucléaire de cellules de patients progeria en présence de farnésol, de géranylgéraniol ou des deux composés. Barres d'erreur = moyenne ± erreur standard de la moyenne. Barre d'échelle = 10 µm.

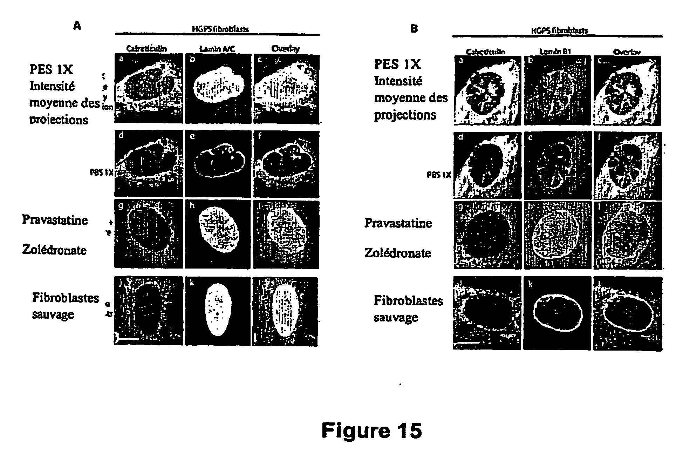

- Figure 15 : représentation de résultats de traitement par pravastatine+zolédronate montrant la correction de la morphologie nucléaire et l'induction d'une relocalisation partielle des isoformes de la lamine A/C et de la lamine B1 de la lamina nucléaire dans le nucléoplasme, dans des cellules de patients progeria. (**A**) Immunofluorescence et microscopie confocale. Les images a à c de chaque panneau sont des projections de l'intensité moyenne de 27 images de la pile et montrent les tubules du réticulum nucléaire marqués par la calréticuline dans les cellules progeria incubées avec le PBS. Images **d** à **I** : coupes confocales isolées de 0,2 µm d'épaisseur. Effet du traitement pravastatine+zolédronate (**g**) et (**h**). (**B**) Colocalisation de la lamine B1 et de la calréticuline. Barre d'échelle = 5 µm.

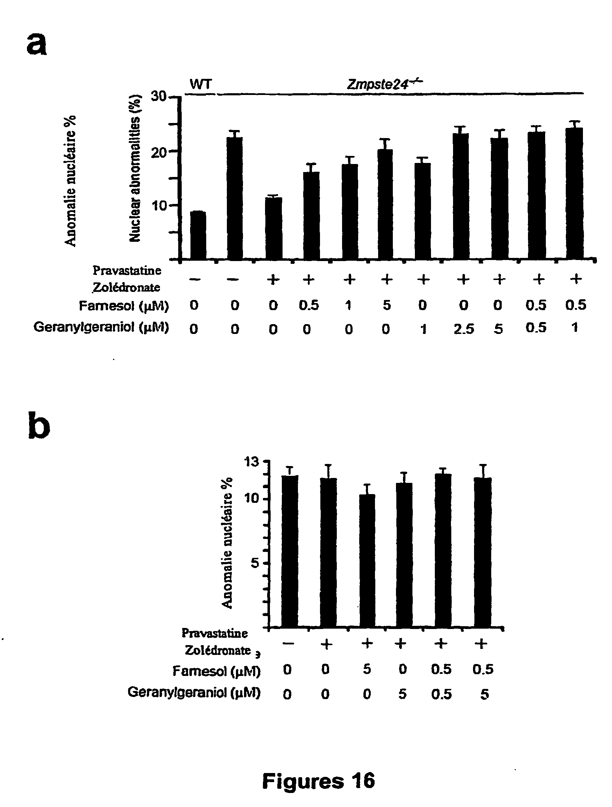

- Figure 16 : représentation de l'effet de la pravastatine et du zolédronate associés ou non sur la morphologie nucléaire de cellules de souris *Zmpste24*^{*-*/*-*} **(a)** et de souris témoins **(b)** en culture, en présence de farnésol, de géranylgéraniol ou des deux molécules.

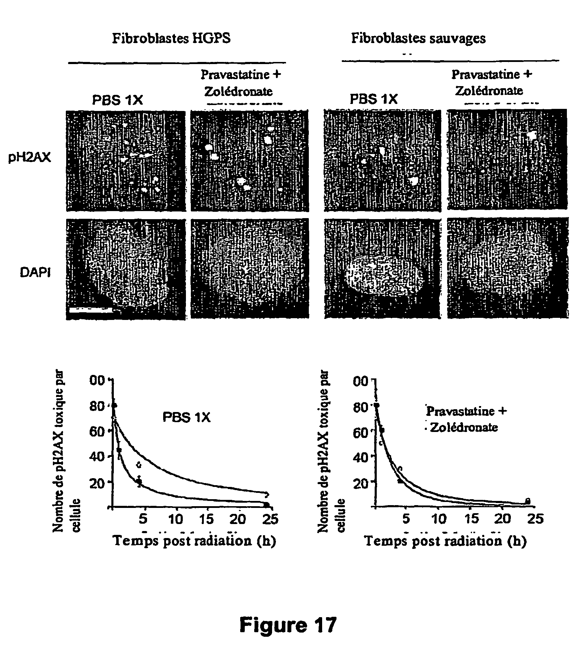

- Figure 17 : Effet du traitement pravastatine+zolédronate sur les anomalies de la réparation des cassures double-brin (DSB) de l'ADN dans les cellules de patients progeria. Immunodétection des foci de l'histone H2AX phosphorylée détectés 24 h après irradiation, foci correspondant aux cassures double brin non réparées (images du haut). Marquage nucléaire du DAPI (images du bas). Courbes du bas : évolution du nombre de foci d'histone H2AX phosphorylée en fonction du temps après irradiation dans les cellules témoins (carré plein) et les cellules progeria (cercle vide) incubées avec le PBS ou traités avec pravastatine+zolédronate. Chaque courbe représente la moyenne ± erreur standard de la moyenne d'au moins 3 expériences.

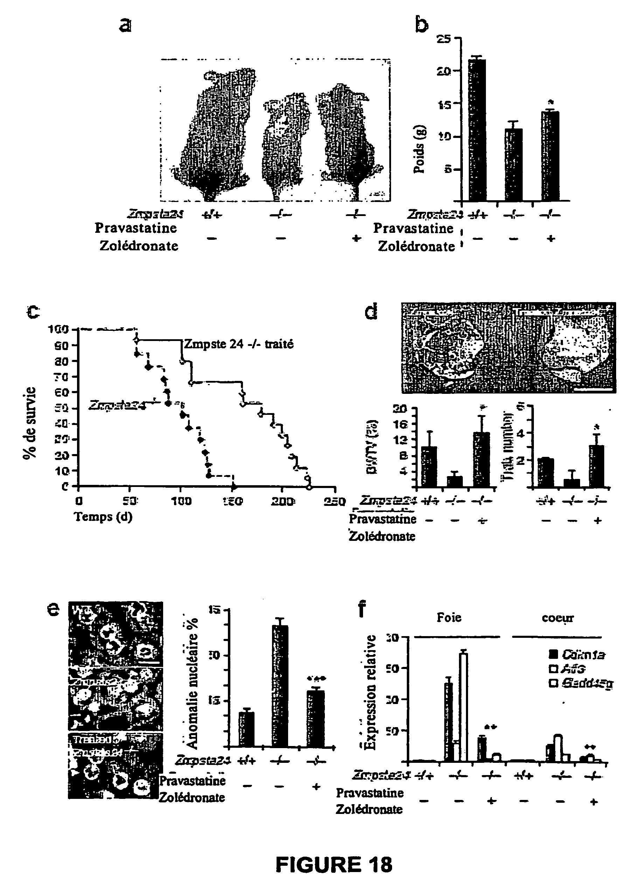

- Figure 18 : Effet du traitement pravastatine+zolédronate sur le phénotype progéroïde de souris *Zmpste24*^{*-*/}*⁻ :* **(a)** Photographies représentatives de souris âgées de 3 mois *Zmpste24*^{*+*/}*⁺, Zmpste24*^{*-*/*-*} et *Zmpste24*^{*-*/*-*} traitées par la combinaison pravastatine + zolédronate. Barre d'échelle = 1 cm. **(b)** Poids de souris âgées de 3 mois *Zmpste24*^{*+*/*+*} (n = 12), *Zmpste24*^{*-*/*-*} (n = 13) et *Zmpste24*^{*-*/*-*} traitées (n = 15). (c) Courbes de Kaplan-Meier montrant une augmentation significative de la dure de vie des souris *Zmpste24*^{*-*/*-*} traitées. **(d)** Représentation tridimensionnelle - par microtomographie informatisée du tibia de souris *Zmpsfe24*^{*-*/*-*} traitée et non traitée (image du haut). Le panneau du bas représente le volume osseux relatif et le nombre de trabécules osseux dans les souris *Zmpste24*^{*-*/*-*} non traitées et traitées. **(e)** Quantification des anomalies nucléaires des hépatocytes de souris *Zmpste24*^{*+*/}*⁺, Zmpste24*^{*-*/*-*} et *Zmpste24*^{*-*/*-*} traitées. Les flèches blanches montrent les noyaux anormaux. Barre d'échelle = 10 µm. **(f)** Expression relative des gènes cibles de la p53 dans le foie et le coeur de souris *Zmpste24*^{*+*/}*⁺, Zmpste24*^{*-*/*-*} et *Zmpste24*^{*-*/*-*} traitées, analysée par RT-PCR quantitative. *P < 0,05 ; ** P < 0,01 ; *** P < 0,001. Les barres d'erreur représentent la moyenne ± erreur standard de la moyenne.

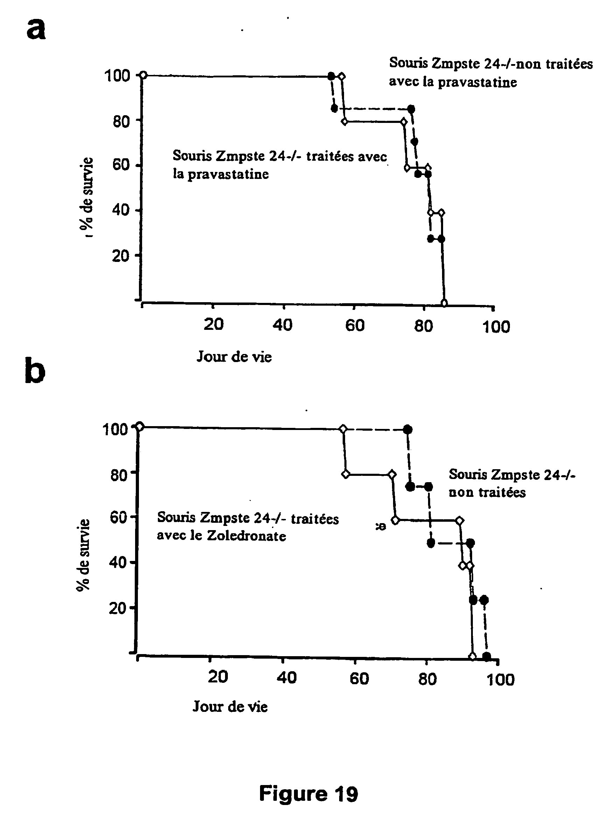

- Figure 19 : Effet de la pravastatine seule ou du zolédronate sur la durée de vie des souris Zmpste24^{-/-} : Courbes de Kaplan-Meier pravastine seule **(a)** et zolédronate seul **(b)** sur des souris *Zmpste2*^{*-*/*-*} traitées (diamant vide) et non traitées (cercles pleins).

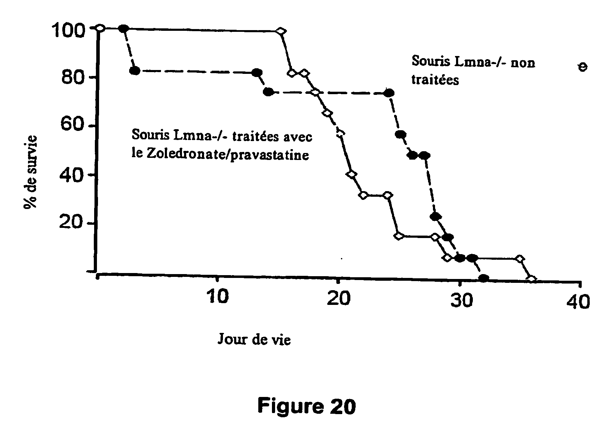

- Figure 20 : Effet d'un traitement pravastatine+zolédronate sur la durée de vie de souris *Lmna*^{*-*/*-*} : Courbes de Kaplan-Meier à partir de souris *Lmna*^{*-*/*-*} traitées par pravastine+zolédronate (n= 12, diamant vide), comparée à celle de souris non traitées (cercles pleins, n = 11).

### EXEMPLES

### EXEMPLE 1 : Effet additif de l'association d'un inhibiteur de l'hydroxyméthylglutaryl-coenzyme A (HMG-CoA) réductase hydrosoluble (une statine hydrosoluble : la pravastatine) et d'un inhibiteur de la farnésyl-pyrophosphate synthase (un aminobiphosphonate : le zolédronate) sur des cultures de cellules normales et progéroïdes

### A. PROTOCOLES

### A.1 Cellules et culture cellulaire

Les lignées de cellules sont soit des fibroblastes témoins AG16409 provenant du Coriell Institute, soit des fibroblastes issus de biopsies de patients atteints de Dermopathie Restrictive. Elles sont cultivées à 37°C sous 5% de CO₂ en salle P2.

Le milieu de culture complet usuel est
- RPMI (Invitrogen) complémenté avec
- Sérum de Veau Foetal 20% (Invitrogen)
- L-Glutamine 200 mM (Invitrogen)
- Mélange Pénicilline/Streptomycine/Fungizone 1 X (Stock 100 X, Cambrex)

### A.2 Récolte des cellules

La récolte des cellules est réalisée par trypsinisation de la façon suivante (protocole pour une grande flasque, 75 cm², BD Falcon) :
- Le milieu est aspiré ;
- Les cellules sont lavées avec 10 ml de PBS 1X (Invitrogen) :
- 5 ml d'une solution de Trypsine/EDTA 1X (Cambrex) sont ajoutés ;
- La flasque est incubée environ 6 min à 37°C, le temps que les cellules se décollent ;
- La trypsine est inhibée par dilution dans 15 ml de milieu complet ;
- Les cellules sont culottées par centrifugation 10 min à 1000 tours par minutes à 16°C ;
- Le culot est resuspendu dans 2 ml de PBS 1X, et re-centrifugé dans les mêmes conditions.

Les cellules obtenues sont soit congelées pour une utilisation ultérieure, soit repiquées à partir de ce culot lavé.

### A.3 Traitements

La solution de pravastatine (statine hydrosoluble) utilisée est préparée comme suit :
40 mg de pravastatine (Sigma Aldrich, P4498) sont repris dans de l'eau stérile pour constituer une solution stock à 10 mM.

Les concentrations finales testées ont été de 500 nM, 1, 5, 50 et 100 µM, obtenues en diluant la solution stock dans du milieu complet.

La solution de zolédronate (NBP) utilisée est préparée comme suit:
Une solution stock d'acide (1-hydroxy-2-imidazol-1-yl-1-phosphono-éthyl) phosphonique (0,8 mg/ml, Novartis) est ajustée à une concentration de 2 mM.

Les concentrations finales testées ont été de 500 nM, 1, 5, 50 et 100 µM, obtenues en diluant la solution stock dans du milieu complet.

### A.4 Western blot

### A.4.1 Préparation des cellules

Pour une expérience de Western blot, les cellules sont traitées comme suit :
Environ 7,5 x 105 cellules sont ensemencées dans une grande flasque et cultivées dans les conditions ci-dessus jusqu'à presque confluence (4 jours).

Au bout de 4 jours, les cellules sont lavées avec du PBS 1X, et reprises dans du milieu complet supplémenté avec le traitement

Les cellules sont incubées le temps du traitement (de 6 à 72h, séquentiellement ou simultanément) dans l'incubateur à 37°C.

A l'issue du traitement, les cellules sont trypsinisées (protocole ci-dessus) et le culot obtenu stocké à -80 °C jusqu'à extraction des protéines.

### A.4.2 Extraction des protéines pour Western blot

Le culot cellulaire est repris dans 300 µl de tampon de lyse

| | |
|---|---|
| Triton X100 | 1 % |
| SDS | 0,1 % |
| Désoxycholate de sodium | 0,5 % |
| NaCl | 50 mM |
| EDTA | 1 mM |
| TrisHCl pH 7,4 | 20 mM |
| Inhibiteur de Protéase (Roche 11697498001) | 1 pastille pour 50 ml |

Extemporanément, on ajoute

| | |
|---|---|
| Orthovanadate de sodium | 1 mM |
| PMSF | 1 mM |

Les cellules sont exposées à une sonication 2 fois 30 sec (*Brandson Sonifier Cell Disruptor B15*)*.*

Les débris cellulaires sont centrifugés pendant 10 min à 10 000 tours par minutes à 4 °C.

Le surnageant protéique est conservé à -80 °C jusqu'à utilisation.

Le dosage des protéines est effectué à la décongélation.

### A.4.3 Western blot

### Gel

Un gel d'acrylamide 8 % est classiquement utilisé pour détecter les différentes formes de lamines A/C.

| | |
|---|---|
| Acrylamide/bisacrylamide 37/1 | 8 % |
| TrisHCl pH 8,8 | 375 mM |
| SDS | 0,1 % |
| APS | 0,1 % |
| TEMED | 0,01 % |

Un gel de concentration est coulé sur le gel séparatif

| | |
|---|---|
| Acrylamide/bisacrylamide 37,5/1 | 3 % |
| TrisHCl pH 6.8 | 375 mM |
| SDS | 0,1 % |
| APS | 0,1 % |
| TEMED | 0,01 % |

La concentration en protéines des échantillons est dosée, et des aliquots sont ajustés à 50 µg par tube dans du tampon de lyse en qsp 15 µl.

5 µl de tampon de charge sont ajoutés à chaque échantillon

| | |
|---|---|
| SDS | 4 % |
| TrisHCl pH 6,8 | 100 mM |
| Glycérol | 20 % |
| β-mercaptoéthanol | 20 % |
| Bleu de bromophénol | traces |

Les échantillons sont dénaturés par chauffage 5 min à 95 °C et déposés dans les puits.

La migration a lieu à 50, puis 100 Volts, dans un tampon

| | |
|---|---|
| Tris-Base | 0,3 % |
| Glycine | 1,44 % |
| SDS | 0,1 % |

### ⇒ Transfert

La membrane de transfert (Hybon P, Amersham Biosciences) est préparée par trempage dans l'éthanol, suivi d'un bain de 5 min dans l'eau stérile, et de 10 min dans le tampon de transfert :

| | |
|---|---|
| Tris-Base | 12 mM |
| Glycine | 96 mM |
| Ethanol | 20 % |

Le gel est humidifié pendant 20 min dans le tampon de transfert, puis le sandwich est monté (système Miniprotéan, Biorad).

Le transfert a lieu en général sur la nuit, en chambre froide, à 10 Volts.

La membrane est rincée dans du PBS 1X, conservée à l'humidité, et utilisé telle quelle pour la détection.

### ⇒ Détection

La membrane est incubée 1 h à température ambiante dans une solution de saturation :

| | |
|---|---|
| Caséine | 10 % |
| Tween 20 | 0,1 % |
| PBS | 1 X |

On la rince 2 fois 10 min dans du tampon de lavage (Tween 20 0,1% / PBS 1X).

L'anticorps primaire est dilué dans le tampon de saturation (détails et dilution, voir ci-dessous immunomarquage).

La membrane est incubée avec les anticorps primaires 1 h à température ambiante sous agitation.

A l'issue, elle est rincée 3x avec du tampon de lavage, puis lavée 3x 15 min avec le même tampon.

L'anticorps secondaire (système couplé à la péroxydase, Jackson Immunoresearch) est dilué au 1/10000e dans du tampon de saturation.

La membrane est incubée avec cette solution 30 à 45 min à température ambiante sous agitation.

A l'issue, elle est rincée 3x avec du tampon de lavage, puis lavée 3x 15 min avec le même tampon.

La détection est effectuée avec le kit ECL Plus Western Blotting System d'Amersham Bioscience, selon les indications du fournisseur.

Après révélation, la membrane est exposée sur film Biomax MR (Kodak), le temps nécessaire pour avoir un signal satisfaisant.

### A.5 Immunomarquage

### A.5.1 Préparation des cellules

Une culture de cellules est trypsinisée, et les cellules comptées sur cellule de Neubauer.

Des puits de culture style Labtech (Nunc, réf. 177399) sont ensemencés, à raison de 5x104 cellules par puits.

Le milieu de culture complet est supplémenté par le ou les traitements (statine, NBP, les 2), et les cellules cultivées pendant le temps ad hoc.

A l'issue, le milieu de culture est aspiré, les puits démontés.

Les lames sont lavées dans du PBS 1X.

Les cellules sont fixées dans une solution de paraformaldéhyde 4% (dans PBS) pendant 10 minutes à température ambiante.

Un lavage de 10 min dans du PBS 1X est effectué.

Les cellules sont déshydratées par des bains successifs de 3 min dans des solutions de pourcentage éthanolique croissant (70, 90, 100%, ce dernier bain étant répété).

Après séchage, les lames sont stockées à -80°C jusqu'à utilisation.

### A.5.2 Marquage

Après décongélation, les cellules sont incubées 5 min à température ambiante en chambre humide dans 50 µl d'une solution de perméabilisation :

| | |
|---|---|
| PBS | 1 X |
| Triton X100 | 0,5 % |
| RNS | 5 % |
| (Rabbit Normal Serum, Vector S5000) | |
| Inhibiteur de Protéase | 1 pastille pour 50 ml |
| (Roche 11697498001) | |

3 bains de préincubation de 15 min chacun sont effectués dans 50 µl de la solution d'incubation :

| | |
|---|---|
| PBS | 1 X |
| RNS | 5 % |
| Inhibiteur de Protéase | 1 pastille pour 50 ml |
| (Roche 11697498001) | |

L'anticorps primaire est dilué au 1/100^{e} dans 50 µl de solution d'incubation et mis au contact des cellules pendant 1 h à température ambiante en chambre humide.

Les anticorps primaires utilisés sont de 2 types :
- Souris anti-lamine A/C (côté N-terminal), clone 4A7, don de G. Morris (Oswestry, UK)
- Chèvre anti-prélamine A (15 aa extrémité C-terminale), produit SC6214, Santa Cruz Biotechnology, Inc.

3 rinçages dans 50 µl de PBS 1X sont effectués pendant 15 min chacun.

L'incubation avec l'anticorps secondaire a lieu pendant 1 h dans 50 ml de solution d'incubation à température ambiante en chambre humide. Les anticorps secondaires sont de deux types :
- Ane anti-souris, Jackson Immunoresearch, dilution au 1/100^{e}
- Ane anti-chèvre, Jackson Immunoresearch, dilution au 1/200^{e}

3 rinçages dans 50 µl de PBS 1X sont effectués pendant 15 min chacun.

Une incubation avec 100 µl de solution DAPI 50 ng/ml (SERVA, réf. 18860) est réalisée pendant 15 min à température ambiante en chambre humide.

3 rinçages dans du PBS 1X sont effectués dans des bacs porte-lames pendant 5 min chacun.

Un ultime rinçage est effectué pendant 5 min dans une solution de Tween20 à 0,1 % dans du PBS.

### A.5.3 Montage

Les cellules sont immergées dans une goutte de VectaShield (Vector), recouvertes d'une lamelle couvre-objet et observées sur un microscope à fluorescence (Leica DMR, Leica Microsystems), équipé d'un système de caméra cooISNAP (Princeton).

### B. RESULTATS

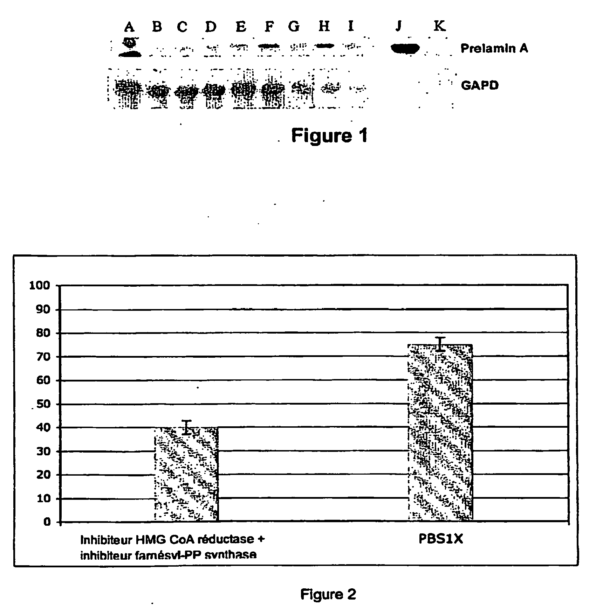

### B.1. Western blot (Figure 1)

Des fibroblastes témoins "normaux" ont été traités avec une statine hydrosoluble (pravastatine P, 20 à 100 µM), et avec un aminobiphosphonate (NBP zolédronate Z, 20 à 100 µM) en association (Pistes A à I, respectivement P20/Z20, P20/Z60, P20/Z100, P60/Z20, P60/Z60, P60/Z100, P100/Z20, P100/Z60, P100/Z100). Le western-blot montre "l'apparition" d'une bande correspondant à la taille de la prélamine A non mature (non tronquée) en fonction de l'augmentation de concentration des deux molécules, ce qui confirme que la farnésylation est nécessaire à la maturation de la lamine A. Ce résultat montre que le blocage de la synthèse du farnésyl-PP en 2 points de la voie métabolique est plus efficace qu'un blocage en un seul point sur l'inhibition de la farnésylation de la prélamine A, au moins *ex-vivo*.

### B.2. Dose- et durée-réponse en immunohistochimie (Figure 2)

Des courbes dose-réponse et durée-réponse ont permis de déterminer une efficacité maximale en mesurant 2 paramètres sur des cellules témoins saines d'une part, puis sur des cellules de patients HGPS.

L'association pravastatine (hydrosoluble) / zolédronate (NBP) la plus efficace a été obtenue pour une administration de 1 µM de pravastatine pendant 24 h, de zolédronate pendant 12 h sur les cellules saines. Aucune toxicité n'a été observée. Sur les cellules HGPS (Cellules avec anomalies nucléaires), en utilisant le même protocole d'administration, le nombre de noyaux "déformés" a baissé de 75 à 40 %. La mesure du taux de prélamine A obtenu sur cellules saines a été effectuée. Cette mesure a montré que ce taux est maximal.

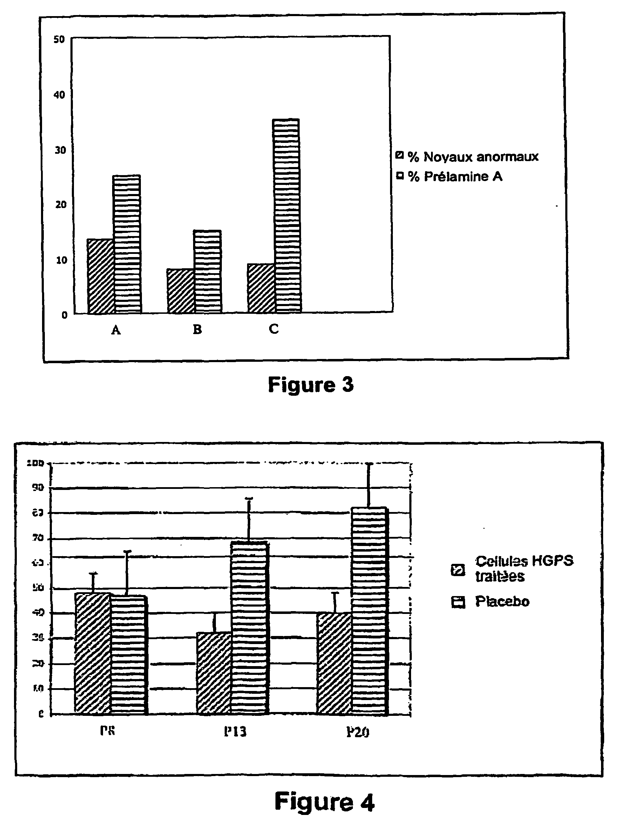

### B.3. Effet du traitement en immunohistochimie (Figure 3)

L'action combinée de la pravastatine et du zolédronate, traitement: Pravastatine 100 µM pendant 12h, Zolédronate 20 µM pendant 6h, montre une meilleure efficacité, puisque le taux de prélamine A produit dans des cellules saines traitées (estimé à 35%) est beaucoup plus élevé en association que si les molécules sont ajoutées seules (respectivement 25 et 15 %). De plus, le taux de noyaux déformés (signe d'une toxicité sur les cellules saines) est minimal (inférieur à 10%), et inférieur à ce qu'il est sur des cellules traitées avec de la pravastatine seule (environ 12 %).

### B.4. Action sur des cellules âgées en immunohistochimie (Figure 4)

En fonction du nombre de "passages" (nombre de repiquage des cellules), donc de l'âge des cellules, la proportion de noyaux anormaux augmente. Cette caractéristique est typique des cellules HGPS non traitées. Si on traite les cellules HGPS avec la Pravastatine 1 µM pendant 24h et Zolédronate 1 µM pendant 12h., cette proportion se maintient, et diminue même un peu (moins de 40% contre plus de 80% chez les cellules traitées avec un placébo).

### B.5. Conclusion

L'association pravastatine/zolédronate est efficace à des doses pour lesquelles quasiment aucun effet n'est observé avec les molécules administrées séparément.

L'effet physiologique de blocage de la voie de prénylation est donc obtenu avec des doses bien inférieures à celles utilisées en traitement unique dans des articles publiés sur des cultures de cellules (Kusuyama & al. 2006 **(27),** 10 µM de pravastatine seule sur progéniteurs de cellules vasculaires ; Flint & al. 1997 **(13),** 25 µM de pravastatine seule sur cellules de muscle de rat néonatal).

### EXEMPLE 2 : Effet d'une composition selon l'invention comprenant un inhibiteur de l'hydroxyméthylglutaryl-coenzyme A (HMG-CoA) réductase hydrosoluble et d'un inhibiteur de la farnésyl-pyrophosphate synthase sur la division de fibroblaste humains âgés et sur des fibroblastes humains jeunes

### A. OBJET DE L'EXEMPLE

Dans le présent exemple, l'évaluation de l'effet *in vitro* d'une composition selon l'invention comprenant un inhibiteur de l'hydroxyméthylglutaryl-coenzyme A (HMG-CoA) réductase hydrosoluble et d'un inhibiteur de la farnésyl-pyrophosphate synthase sur le taux de division cellulaire (index mitotique) de fibroblastes a été mesuré. Une comparaison de l'effet de la composition sur des fibroblastes humains âgés par rapport à des fibroblastes humains jeunes a également été effectuée. Le nombre d'actifs utilisés dans cette expérience est de quatre, les produits ayant été employés en combinaisons deux par deux. Les actifs utilisés sont:
A1 : Zolendronate
A2 : Alendronate
B1 : Pravastatine
B2 : Simvastatine

Les associations particulières qui ont été utilisées dans cet exemple sont : A1B1, A1B2, A2B1, A2B2.

### B. PROTOCOLE

Dans cet exemple, deux lots de fibroblastes, fibroblastes âgés (n° de lot : 9052) et jeunes (n° de lot : 7080), ont été mis en culture dans un milieu RPMI (Invitrogen) contenant 10 % de sérum de veau foetal sans antibiotiques durant 24 h après trypsination de boîtes fournies.

Les différents actifs ont été ajoutés à une concentration de 1 µM finale chacun durant 24 h (une dilution 1000 d'une solution stock dans l'eau pour les composés A1, A2 et B1, ou dans l'Ethanol 100% pour le composé B2, a été effectuée).

L'index mitotique a été évalué par incorporation de Bromo-déoxy-uridine (BrdU) sur 45 minutes après 24 h d'incubation des cellules avec l'une des combinaisons d'actifs. Une révélation immunohistochimique a permis de révéler les cellules en phase de synthèse d'ADN (cellule en pré-division). Une coloration des noyaux (du matériel génétique) a été réalisée par incorporation de di-amino phényl indol (DAPI).

La saisie de 6 champs microscopiques (OLYMPUS IX 70) a permis une mesure de l'index mitotique par analyse d'image (OLYMPUS AnalySIS). L'index mitotique correspond au rapport du nombre de noyaux ayant incorporé le BrdU sur le nombre de noyaux ayant incorporé le DAPI. Un index moyen est calculé statistiquement avec une déviation standard (écart-type) comprise entre 0,005 et 0,061.

Un test de Student bilatéral a permis de mesurer la signification statistique des résultats obtenus.

### C. RESULTATS

### C.1. Observation visuelle générale des cellules

Ces résultats montrent une très faible capacité de division des fibroblastes âgés en l'absence de tout traitement, préalablement à l'étude.

Les fibroblastes jeunes ont montré une capacité de division supérieure à celle des fibroblastes âgés. La capacité de division des fibroblastes âgés était inférieure à 5 %, tandis que la capacité de division des fibroblastes jeunes était de 15,6 %. La différence de capacité de division entre les fibroblastes âgés non traités et les fibroblastes jeunes non traités était donc égale à 3.

Au cours de la réalisation de cet exemple, aucune toxicité n'a été observée visuellement après 24h d'incubation des fibroblastes avec les combinaisons d'actifs testées.

Au cours de la réalisation de cet exemple, aucun effet toxique de l'éthanol (0.1 % final) n'a été observé après 24h d'incubation.

### D. EVALUATION DE L'INDEX MITOTIQUES (Figure 5)

D'une manière générale, le nombre de fibroblastes âgés sans aucun traitement en phase de synthèse d'ADN était extrêmement faible : moins de 5% (voir figure 5, colonne 1).

L'index mitotique n'était pas non plus très élevé pour les fibroblastes jeunes : de l'ordre de 15 % (voir figure 5, colonne 6).

Par comparaison avec les fibroblastes âgés témoins sans aucun traitement, les fibroblastes témoins exposés à l'éthanol (0.1% - 24 h), ne présentent pas une différence significative (p = 0.11, n=6) de leur index mitotique. Les valeurs ont été alors regroupées (Témoin, n=12).

Les résultats présentés dans la figure 5, colonne 2, montrent, sur l'index mitotique des fibroblastes âgés un effet activateur de A1B1 - Zolendronate-Pravastatine par rapport au témoin (stimulation d'un facteur 2 maximum) (p < 0.001, n≥6).

Cet exemple montre donc que l'application de la combinaison Zolendronate-Pravastatine a un effet activateur de la division cellulaire de fibroblastes de sujet âgé.

### Exemple 3: Effet de l'association d'un inhibiteur de l'hydroxyméthylglutaryl-coenzyme A (HMG-CoA) réductase hydrosoluble et d'un inhibiteur de la farnésyl-pyrophosphate synthase sur un modèle de souris présentant un syndrome progéroïde

Les souris KO Zmpste24-/ utilisées ici sont celles décrites dans l'article cité de Varela & al. 2005 (49). Une preuve d'efficacité de l'association des 2 molécules (pravastatine et zolédronate) a été apportée en collaboration avec un laboratoire espagnol (Pr C. Lopez-Otin). L'efficacité est obtenue à des doses combinées qui n'ont pas d'effet lorsque les produits sont utilisés séparément, démontrant un effet additif

Les 2 molécules (Acide zolédronique (Zometa (marque déposée)) 100 µg/kgrour et Pravastatine 100 mg/kg/jour) ont été diluées dans du PBS 1X et injectées par voie intrapéritonéale, quotidiennement, sur des souris âgées de 1 mois et jusqu'à leur décès. Les contrôles sont des souris sauvages de même portée, traitées au PBS 1X seul.

La survie des souris traitées a été grandement améliorée, elle est maximale notamment pour les femelles, avec un allongement de la durée de vie moyenne de 80% environ. Les symptômes cliniques de la maladie sont tous considérablement réduits par rapport aux individus traités avec le PBS seul. Notamment, il a été observé un effet du traitement sur la peau et la repousse des poils de ces souris par rapport aux souris traitées avec le PBS, qui montraient de larges zones glabres.

### EXEMPLE 4: Effets de l'association d'un inhibiteur de l'hydroxyméthylglutaryl-coenzyme A (HMG-CoA) réductase hydrosoluble et d'un inhibiteur de la farnésyl-pyrophosphate synthase sur des extraits ex-vivo de peau humaine.

### A. Protocole

Dans le présent exemple, les tests sont réalisés sur une peau provenant d'un donneur âgé d'environ 60 ans. Une préparation de 21 explants de peau humaine est réalisée et mise en survie en milieu BEM (BIO-EC's Explants Médium).

Les explants sont répartis en 3 lots de six explants et un lot témoin T0 de 3 explants, comme suit :
- **T0** Témoin Plastie : 3 explants
- **T** Témoin non traité : 6 explants
- **R** Contrôle positif : 6 explants
- **P** Explants traités par la composition de l'invention : 6 explants

### A.1. Traitement

Le traitement est réalisé à différent jour, au premier jour (J0), 2 heures après la préparation des explants, puis à J+1 jour, J+2 jours, J+4 jours, J+6 jours, J+8 jours et J10+ jours.

Les produits sont appliqués sur les explants comme suit :
- **T** les explants ne reçoivent aucun traitement,
- **R** les explants reçoivent chacun à J0, J+2 et J+4, 1 mg du contrôle positif (crème au rétinol)
- **P1** les explants reçoivent chacun et à chaque temps de traitement 2mg du produit P,

Le traitement est réalisé par application topique de la composition de l'invention. La composition est ensuite répartie sur toute la surface de l'explant, à l'aide d'une spatule. La moitié du milieu de culture est renouvelée tous les deux jours et les explants sont remis en survie à 37 °C en atmosphère humide enrichie de 5% de CO₂.

### A.2. Prélèvement pour l'histologie

A J0, les 3 explants du lot T0 sont prélevés.

A J+6 jours et J+11 jours, 3 explants de chaque lot sont prélevés. Les prélèvements sont coupés en deux, une moitié est fixée dans du formol et l'avitre moitié est congelée à -80°C, suivant la procédure BIO-EC « P006-PPEH ».

### B. Etude histologique

Après 24 heures de fixation dans le formol, les prélèvements sont déshydratés, imprégnés et enrobés en paraffine. Des coupes de 5µm sont réalisées pour l'observation morphologique.

### B.1. Première étape : étude morphologique

L'étude morphologique des structures épidermiques et dermiques est réalisée sur les coupes colorées au trichrome de Masson, variante de Goldner.

### B.2. Deuxième étape :

### B.2.1 Immunomarquage du KI67 :

L'immunomarquage des cellules en mitoses est réalisé sur coupes congelées avec l'anti-corps polyclonal anti-KI 67 (Novo Castra) révélé en DAB. Les cellules positives sont comptées sur toute la longueur épidermique et les moyennes ramenées au nombre de cellules marquées par cm.

### B.2.2 Immunomarquage du collagène I:

L'immunomarquage du collagène I sera réalisé sur coupes congelées avec l'anticorps polyclonal anti-collagène I révélé en FITC. Les noyaux sont contre-colorés à l'iodure de propidium.

### B.2.3 Immunomarquage du collagène III :

L'immunomarquage du collagène III est réalisé sur coupes congelées avec l'anti-corps polyclonal anti-collagène III révélé en DAB. Les noyaux sont contre-colorés à l'hémalun de Masson.

### B.2.4 Immunomarquage du collagène IV :

L'immunomarquage du collagène IV est réalisé sur coupes congelées avec l'anti-corps polyclonal anti-collagène IV (Cliniscience) révélé en FITC. Les noyaux sont contre colorés à l'iodure de propidium.

### B.2.5 Immunomarquage du collagène VII :

L'immunomarquage du collagène VII est réalisé sur coupes congelées avec l'anti-corps monoclonal anti-collagène VII révélé en FITC. Les noyaux sont contre- colorés à l'iodure de propidium.

### B.2.6 Immunomarquage de PECAM1 :

La visualisation des cellules endothéliales est réalisée grâce à l'immunomarquage de PECAM-1, réalisé sur coupes congelées avec l'anticorps monoclonal anti-PECAM-1 révélé en Fast-red.

### EXEMPLE 5: Effet de l'association d'un inhibiteur de l'hydroxyméthylglutaryl-coenzyme A (HMG-CoA) réductase hydrosoluble et d'un inhibiteur de la farnésyl-pyrophosphate synthase sur des cultures in-vitro de cellules constitutive de la peau.

Cet exemple est réalisé avec les mêmes combinaisons d'actifs présentés dans l'exemple 2 ci-dessus. Ces différentes combinaisons d'actifs sont utilisées *in vitro* afin d'évaluer leur effet sur des paramètres physiologiques intervenant dans le vieillissement cutané.

Les combinaisons utilisées dans le présent exemple sont A1B1, A1B2, A2B1, A2B2, respectivement. Ces quatre combinaisons sont testées à plusieurs concentrations, les expériences étant faites en triple (ce qui représente au moins 36 points expérimentaux).

Les concentrations des 4 combinaisons sont proposées par le demandeur et il n'est donc pas envisagé à ce stade d'étude (sauf à titre de contrôle simple) de cytotoxicité *in vitro.* L'expérimentation est faite sur des cultures de cellules d'une lignée de fibroblastes tels que présentées dans l'exemple 1. Ce test est également appliqué à des cultures de kératinocytes. Les paramètres suivants sont examinés pour les 4 combinaisons d'actifs aux concentrations indiquées.
- Mesure de l'index mitotique.
- Mesure du remodelage de la matrice extracellulaire par contraction de lattices de collagène.
- Mesure de la réparation d'ADN génomique après irradiation aux UVB (Stress photoinduit s'approchant des conditions du bain de soleil)

La mesure de l'index mitotique est réalisée après exposition des cellules aux actifs durant un temps unique. L'index est évalué par comptage en analyse d'image des noyaux cellulaires ayant incorporé un analogue de la thymidine rendu fluorescent, sur le nombre de noyaux totaux. Plusieurs champs sont analysés. Des photos sont archivées pour iconographie.

Le remodelage de la matrice extracellulaire induite par les fibroblastes exposés aux actifs est évalué par incorporation de ces cellules dans des lattices de collagène et en quantifiant leur capacité à rétracter ces lattices. L'évaluation de la surface rétractée donne un index de remodelage. Des photos sont archivées pour iconographie.

La mesure de la réparation d'ADN génomique est réalisée après irradiation des cellules à une dose d'UV-B mimant les conditions d'un coup de soleil. Il est envisagé, dans un premier temps, d'évaluer l'effet des actifs au cours de la réparation d'ADN suivie sur une cinétique de 3 temps. La quantification est réalisée par détection et analyse d'image des dimères de pyrimidine cyclobutane induits par l'irradiation UVB à l'aide d'une technique immunohistochimique.

Des photos sont archivées pour iconographie.

### Exemple 6 : Objectif principal : Mesure de l'impact du virus VIH et des thérapeutiques antirétrovirales sur les marqueurs nucléaires, mitochondriaux et cytosoliques du vieillissement cellulaire

### ▪ Objectifs secondaires

- Analyser la prévalence des altérations des fonctions nucléaires, mitochondriales et cytosoliques chez des patients infectés par le VIH
- Mesurer l'incidence d'apparition de ces anomalies
- Analyser le type et la fréquence de ces anomalies en fonction de la durée d'exposition aux antirétroviraux, de façon globale, par classe et par molécules (durée cumulée d'exposition).
- Analyser le type et la fréquence de ces anomalies en fonction de la charge virale VIH intracellulaire
- Analyser le type et la fréquence de ces anomalies en fonction de la durée de suivi de l'infection par le VIH

### A. PLAN EXPERIMENTAL

### ▪ Choix du plan expérimental

Cette étude observationnelle comprend 3 groupes de patients :
- Groupe A: Patients infectés par le VIH1 naifs de traitement antirétroviral
- Groupe B: Patients infectés par le VIH1 sous traitement antirétroviral depuis au moins 12 mois.
- Groupe C : Témoins séronégatifs pour le VIH appariés sur l'âge et le sexe.

### B. CRITERES D'ELIGIBILITE :

### ▪ Critères d'inclusion :

- âge > 18 ans et <65 ans:
- ayant signé un formulaire de consentement éclairé
- séropositif pour le VIH confirmé en Elisa et Western Blott depuis au moins 5 ans
- séronégatif pour le VIH2
- N'ayant jamais reçu de traitement antirétroviral ou en première ligne de traitement depuis au moins 12 mois.

### ▪ Critères de non inclusion:

- âge <18 ans et > 65 ans
- n'ayant pas signé le formulaire de consentement éclairé
- séropositif pour le VIH2
- Patients traités par statines ou amino-biphosphonates
- Traitements concomittants non autorisés : testostérone, traitement d'un diabète insulino-dépendant

### C. DEROULEMENT DE L'ETUDE

### ▪ Nombre de patients évalués:

200 patients dont :
- Groupe A : n= 50
- Groupe B : n= 100
- Groupe C : n= 50

Les patients du groupe A nécessitant au cours de leur suivi la mise en route d'une thérapeutique antirétrovirale ont une évaluation supplémentaire lors de l'instauration du traitement et sont analysés à partir de cette date avec les patients du groupe B.

Les données mesurées au cours de la période pendant laquelle ils ont été inclus dans le Groupe A permettent de mesurer le rôle direct éventuel du virus VIH sur les fonctions nucléaires et mitochondriales.

Les patients du groupe B nécessitant une modification de traitement antirétroviral en cours d'études, ont une évaluation clinique et paraclinique identique à celles prévues dans le cadre du suivi lors du changement de traitement. Les données de suivi de ces patients sont prises en compte pour le calcul de l'incidence des perturbations des fonctions nucléaires et mitochondriales observées chez les patients exposés aux antirétroviraux.

### ▪ Durée de l'étude :

La durée de l'étude est de 36 mois

### Pour les patients des Groupes A et B

### ▪ Historique de l'infection par le VIH:

- Date de diagnostic de l'infection par le VIH
- Mode de contamination
- Stade CDC
- Pour les stades C : diagnostic de l'affection classante SIDA
- Traitements antirétroviraux en cours (Date d'introduction, Molécules administrées)

### ▪ Examen Clinique :

- Poids, Taille, Indice de masse corporelle
- Tour de taille, tour de hanche, calcul du rapport Taille/ hanche

### ▪ Bilan sanguin :

- Mesure de la charge virale du VIH (seuil de détection 40 copies/ml)
- Dosage du taux des lymphocytes CD4 et des CD8
- Glycémie, Insulinémie , calcul du HOMA
- Cholestérol Total, LDL, HDL Cholestérol
- Triglycérides
- Prélèvement de 3 tubes EDTA de 7,5 ml pour analyse des fonctions nucléaires et mitochondriales, mesure de l'ADN proviral du VIH et cellulothèque

### ▪ Analyse des protéines nucléaires, mitochondriales et cytosoliques cibles des antirétroviraux :

- Western blot et immunornarquages :
- La production de NF□B et de I□B comme témoins de l'activité du protéasome
- la maturation des lamines A et B, modèles de protéines nucléaires
- la production des isoformes de SREBP et leur importation nucléaire
- la N- et O-glycosylation de CD36 purifié et déglycosylé (± 30 kDa de sucres) (Abcam)
- l'importation dans la mitochondrie de Hsp70 possédant un signal d'adressage clivé, comme mesure de l'activité des protéases mitochondriales (Abcam)
- les fonctions « respiratoires » mitochondriales : production de ROS, étude des sous-unités II et IV de la cytochrome oxydase (Molecular Probes)

La recherche d'une éventuelle susceptibilité aux traitements antirétroviraux est réalisée en génotypant certaines des cibles, comme cela a été montré pour la sensibilité de la protéase virale aux PI (Baxter et al., 2006):
- ZMPSTE24 et Rce1 impliquées dans la maturation des prélamines A et B1/B2 (en routine au Laboratoire de génétique Moléculaire).
- Les protéases S1-P et S2-P permettant la production du domaine de SREBP actif sur la transcription des gènes du métabolisme lipidique
- Les transporteurs mitochondriaux des désoxynucléosides

### Pourle groupe témoin C :

### ▪ Examen Clinique :

- Poids, Taille, Indice de masse corporelle
- Tour de taille, tour de hanche, calcul du rapport Taille/ hanche
- Prélèvement de 3 tubes EDTA de 7,5 ml pour analyse des protéines nucléaires, mitochondriales et cytosoliques et mise en DNAthèque, cellulothèque

### Visites de suivi

L'évaluation comprend :

### ▪ Un Examen Clinique :

- Poids, Taille, Indice de masse corporelle
- Tour de taille, tour de hanche, calcul du rapport Taille/ hanche

### ▪ Un Bilan sanguin :

- Mesure de la charge virale du VIH (seuil de détection : 40 copies/ml)
- Dosage du taux des lymphocytes CD4 et des CD8
- Glycémie, Insulinémie, calcul du HOMA
- CholestérolTotal, LDL, HDL Cholestérol
- Triglycérides
- Prélèvement de 3 tubes EDTA de 7,5 ml pour analyse des protéines nucléaires, mitochondriales et cytosoliques, mesure de l'ADN proviral du VIH et mise en DNAthèque, cellulothèque

### ▪ Analyse des protéines nucléaires, mitochondriales et cytosoliques cibles des antirétroviraux (voir ci-dessus)

### Pour les patients du Groupe A :

Les patients nécessitant la mise en route d'un traitement antirétroviral ont une évaluation clinique et paraclinique identique à celle prévue dans le cadre du suivi au moment de l'instauration du traitement. Les données de suivi de ces patients sont analysées à compter de cette date dans le groupe des patients traités (groupe B).

### Pour les patients du Groupe B :

En cas de modification de traitement antirétroviral en cours d'études, une évaluation clinique et paraclinique identique à celles prévues dans le cadre du suivi est réalisée lors du changement de traitement. Les données de suivi de ces patients sont prises en compte pour le calcul de l'incidence des perturbations des fonctions nucléaires et mitochondriales observées chez les patients exposés aux antirétroviraux.

### D. Résultats attendus, perspectives

Confirmer *in vivo* chez des patients infectés par le virus du SIDA et soumis à des traitements anti-rétroviraux les résultats obtenus *in vitro* dans des cultures cellulaires : ces traitements, en particulier les inhibiteurs de la protéase, induisent un vieillissement accéléré selon les mêmes mécanismes que les laminopathies génétiques (avec production de prélamine A farnésylée ou de progérine) ou que le vieillissement « physiologique » (avec production de progérine).

Conforter l'hypothèse selon laquelle la combinaison de drogues (statine et aminobiphosphonate) utilisée dans la progéria pourrait être utilisée pour lutter contre le viellissement accéléré de patients infectés par le virus du SIDA et soumis à un traitement anti-rétroviral et permettre la mise en place d'un essai thérapeutique.

### Exemple 7 : Un traitement associant une statine et un aminobiphosphonate augmente la durée de vie d'un modèle de souris reproduisant un syndrome humain de vieillissement prématuré.

Cet exemple est également publié dans Varela et al, Nature Medicine 2008, 7, 767 **(54 bis)**.

### Matériel et méthodes

### Souris :

La production des souris *Zmpste24*^{-/-} et *Lmna*^{-/-} a été décrite (Pendas et al. 2002 **(38)**; Sullivan et al., 1999 **(285)**. La microtomographie informatisée osseuse des souris a été réalisée à l'aide du micro-CT SkyScan 1172 system (SkyScan - marque de commerce)). Toutes les expérimentations sur les souris obéissent aux règles édictées par le Comité de l'Expérimentation Animale de l'Université d'Oviédo (Espagne). La pravastatine (100 mg/kg/jour) et le zolédronate (100 mg/kg/jour) dilués dans du PBS sont administrés aux souris tous les jours. Les souris recevant le traitement pravastatine-zolédronate ou les souris témoins recevant uniquement du PBS ne présentent aucun dommage apparent ni stress.

### Culture cellulaire

Les fibroblastes dermiques d'un sujet témoin (GM00038) et de patients atteints de progeria et porteurs de la mutation G608G (AG11498 et AG01972) ont été obtenus du Coriell Cell Repository. Les cellules HeLa proviennent de l'American Type Culture Collection. Les cellules sont cultivées dans du DMEM (Gibco) supplementé avec 10% FBS (Gibco) et 1% antibiotic-antimycotic (Gibco). Les fibroblastes proviennent de queues de souris âgées de 12 jours (Varela et al., 2005). La concentration et la durée du traitement avec la statine et l'aminobiphosphonate sont indiquées dans la légende des figures. Lors du traitement combiné statine+aminobiphosphonate en présence de farnésol et/ou de géranylgéraniol, 1mM de pravastatine et 1mM de zolédronate ont été ajoutés au milieu de culture avec des concentrations croissantes de farnésol et/ou de géranylgéraniol. Le pourcentage de noyaux anormaux est mesuré 48 h après le début du traitement.

### Immunocytochimie

Les fibroblastes sont cultivés dans des chambres Lab Tek (Nalgen Nunc International), lavés dans le PBS, puis fixés dans du paraformaldéhyde 4% pendant 15 min. Les cellules sont déshydratées dans des bains d'éthanol en concentration croissante et sont perméabilisées 5 min à 25 °C dans du PBS contenant du Triton X-100 (0,5%), 5% de sérum (chèvre ou lapin). Les lames sont préincubées à 25°C dans du PBS pendant 5 min.

La dilution des anticorps primaires est de 1:100 pour l'anticorps polyclonal de chèvre anti-prélamine A (Sc-6214, Santa Cruz Biotechnologies), 1:40 pour l'anticorps anti-lamine A/C (4A7 fourni par G. Morris), 1:200 pour l'anticorps de lapin anti-calréticuline (Stressgen) et 1:100 pour l'anticorps anti-lamine B1 (Abcam). Après lavage dans le PBS, les lames sont incubées 1 h à 25°C avec les anticorps secondaires dilués dans la solution d'incubation. La dilution des anticorps secondaires est la suivante : 1:100 pour l'IgG d'âne anti-souris couplé au FITC (Jackson ImmunoResearch), 1:400 pour l'IgG d'âne anti-chèvre couplé à l'Alexa 488, 1:800 pour l'IgG d'âne anti-chèvre couplé à l'Alexa 568 (Molecular Probes) et 1:100 pour l'IgG d'âne anti-lapin couplé au tétraméthylrhodamine isothiocyanate (Sigma). Les cellules sont ensuite lavées, les noyaux colorés pendant 15 min à 25°C au DAPI (100 ng/ml, Sigma-Aldrich), enfin lavées 3 fois avec du PBS contenant 0,5% de Tween 20. Les lames sont montées dans du Vectashield (Vector). Des images numériques sont enregistrées avec un microscope Leica DMR équipé d'une caméra CoolSnap ou avec un microscope confocal Leica TCS SP5. Les noyaux sont observés dans des cellules après marquage de la lamine A/C. Plus de 100 noyaux ont été analysés dans des fibroblastes témoins pour chacun des traitements. Le nombre de noyaux de cellules de patients atteints de progeria analysé est de 250 (passage 8), 198 (passage 13) et 150 (passage 20).

### Irradiation X et étude de l'histone H2AX phosphorylée.

Les cellules de patients progeria et les cellules témoins 1 BR3 sont irradiées avec un appareillage X Philips. Le faisceau X est produit par une anode de tungstène soumise à une tension de 200 kV sous une intensité de 20 mA avec un filtre de cuivre de 0,1 mm de diamètre. Le débit de dose est de 1,243 Gy/min. L'histone phosphorylée H2AX est détectée avec des anticorps reconnaissant spécifiquement la sérine 139 phosphorylée (Upstate Biotechnology-Euromedex, Mundolsheim, France) à la dilution 1:800 et des anticorps anti-souris conjugués au FITC (1:100, Sigma-Aldrich). Le nombre des cassures double brin (DSB) en fonction de la durée de la réparation a été ajusté avec la formule Nt = N0 (1 / 1 + βt)^{α}, où α et β sont des paramètres ajustables et Nt et N0 le nombre de DSB au temps t et au temps 0.

### Western blot

Les cellules sont homogénéisées dans le milieu suivant : 50 mM Tris (pH 7,4), 150 mM NaCl, 1% NP-40, 50 mM NaF, 1 mM dithiothreitol, 2 mg/ml pepstatin A, en présence des inhibiteurs de protéases (Complete inhibitor cocktail, Roche) et d'inhibiteurs de phosphatases (Phosphatase Inhibitor Cocktails I and II, Sigma). Après électrophorèse, les protéines sont transférées sur des membranes de nitrocellulose bloquées avec 5% de poudre de lait délipidée, à l'aide du tampon TBS-T (20 mM Tris pH 7,4, 150 mM NaCl et 0,05% Tween-20), et incubées 1 h avec soit un anticorps anti-lamine A/C spécifique (4A7, 1:500), soit un anti-lamine A spécifique (C20, Santa Cruz Biotechnology, 1:250) ou un anti-bêta actine (A5441, Sigma, 1:5000). Les anticorps sont dilués dans du TBS-T contenant 3% de poudre de lait délipidée. Les blots sont ensuite incubés avec un anticorps couplé à la peroxydase (chèvre anti-souris ou anti-lapin, Jackson ImmunoResearch) dans du TBS-T contenant 1,5% de poudre de lait délipidée, puis lavés, enfin révélés par chémiluminescence (Immobilon Western chemiluminescent HRP substrate, Millipore - marque de commerce).

### Analyse parspectrométrie de masse

Les fibroblastes de souris témoins et *Zmpste24*^{-/-} ainsi que les cellules lymphoblastoïdes de patients progeria ont été homogénéisées, les noyaux isolés par ultracentrifugation et les protéines nucléaires obtenues comme décrit dans Blobel et Potter, V.R. Nuclei from rat liver : isolation method that combines purity with high yield, Science 154, 1662-1665, 1966. Les protéines de la lamina nucléaire ont été séparées par électrophorèse SDS-PAGE, et les bandes contenant la lamine A, la prélamine A et la progérine ont été excisées. Les fragments du gel ont été lavés deux fois avec 180 ml d'un mélange ammonium bicarbonatelacétonitrile (70:30, 25 mM), séchés (15 min, 90°C) et digérés (1 h, 60°C) avec de la trypsine (12 ng/ml, Promega) dans le bicarbonate d'ammonium 25 mM. Dans une expérimentation typique, 1 ml de CHCA (acide a-cyano-4-hydroxycinnamic, Waters) est placé dans un spectromètre MALDI-ToF. Une fois séché, 1 ml de la solution de peptides et 1 ml de la matrice (CHCA) sont placés dans le spectromètre équipé avec une source laser (Voyager-DE STR (marque de commerce), Applied Biosystems). Les données collectées à partie de 200 tirs laser produisent des spectres analysés avec le programme Data Explorer (version 4.0.0.0, Applied Biosystems).

### PCR quantitative en temps réel

Le taux d'expression de gènes cibles de p53 (*Atf3, Gadd45g* et *Cdkn1a,* qui code p21) a été mesuré avec l'appareil ABI PRISM 7900HT Sequence détection system (Applied Biosystems).

### Analyse statistique

La différence entre les différents groupes de souris et de cellules, traitées ou non, a été analysée à l'aide du teste de Student. Les calculs ont été réalisés à l'aide du programme Microsoft Excel. Les données sont exprimées en moyenne ± erreur standard de la moyenne (SEM).

### Résultats

Des cellules HeLa sont cultivées en présence d'inhibiteurs de la farnésyl-transférase (FTI-277, Sigma-Aldrich) et/ou de la géranyl-géranyl transférase de type I (GGTI-2147, Sigma-Aldrich) aux concentrations indiquées. Seule la combinaison des deux inhibiteurs conduit à l'accumulation dans les cellules d'une quantité importante de prélamine A non prénylée par rapport à l'effet de chacun des inhibiteurs utilisés seuls.

Ces résultats sont montrés sur la figure 10 qui est une photographie d'un Western blot obtenu montrant la Détection de la lamine A/C dans des cellules HeLa traités par inhibiteurs de la farnésyl-transférase et/ou de la géranylgénamyl transférase de type I. LA = lamine A, LC = lamine C, Pre = prélamine A.

Ces résultats confirment que le blocage de la prénylation de la prélamines A nécessite à la fois l'inhibition de la farnésyl-transférase et de la géranyl-géranyl-transférase de type I conformément à la présente invention.

### L'inhibiteur de la farnésyl-transférase (FTI) induit la géranyl-géranylation compensatoire de la progérine (dans des cellules de patients atteints de progeria) et dans des fibroblastes de souris Zmpste24^{-/-}

L'analyse par spectrométrie de masse montre comme attendu la présence de peptides tryptiques de la prélamine A farnésylée et carboxyméthylée dans les cellules de souris *Zmpste24*^{-/-} mais pas dans les cellules de souris témoins. Ces résultats sont montrés sur la Figure 13a. Le peptide farnésylé est dépourvu des 3 résidus SIM ce qui montre que Zmpste24 n'est pas indispensable au premier clivage au cours de la maturation de la prélamine A. Une diminution de la quantité de prélamine A farnésylée est observée dans les cellules de souris traitées par FTI. Lors de l'observation de la partie du spectre correspondant aux peptides géranylgéranylés, aucun peptide dérivé de la prélamine A n'a pu être détecté dans les cellules de souris *Zmpste24*^{*-*/*-*} non traitées. Mais un peptide dont la masse est compatible avec un fragment géranylgéranylée de la prélamines A.est détecté après traitement avec le FTI . Ces résultats sont montrés sur la Figure 13b.

Dans les cellules de patients progeria, des peptides correspondant à la progérine farnésylée et carboxyméthylée sont détectés en l'absence de tout traitement, comme indiqué que la Figure 11 a. Le traitement de ces cellules par FTI-127 provoque l'apparition de peptides dont la masse correspond à celle de peptides géranylgéranylés de la progérine, comme cela apparaît sur la Figure 11b.

L'ensemble de ces données montre que la progérine et la prélamine A sont géranylgéranylées de manière alternative sous l'effet des FTI et fournit une explication à la faible efficacité des traitements par FTI dans les modèles murins de syndrome progéroïdes.

Les cellules de patients progeria et de souris *Zmpste24*^{*-*/*-*} ont été utilisées pour évaluer des stratégies thérapeutiques destinées à prévenir la prénylation croisée de la prélamine A et de la progérine. Nous avons fait l'hypothèse que la farnésylation des variants anormaux de la lamine A pourraient être inhibée par des drogues agissant sur la voie de biosynthèse du farnésyl-pyrophosphate, substrat de la farnésyl-transférase et précurseur du géranylgéranyl pyrophosphate, substrat de la géranyl-géranyl transférase de type I. Nous avons donc testé l'effet de deux drogues, une statine et un aminobiphosphonate, connues pour agir à deux étapes de cette voie métabolique, sur des cellules de patients progeria. L'analyse par spectrométrie de masse montre que l'association pravastatine (statine) zolédronate (aminobiphosphonate) provoque l'apparition d'un peptide correspondant à l'extrémité C-terminale non prénylée de la progérine, peptide non détectable dans les cellules traitées par FTI, alors que ni les peptides farnésylés, ni les peptides géranylgéranylés ne sont pas non plus détectés, comme cela apparaît sur la Figure 11c). Le traitement statine+aminobiphosphonate inhibe bien la prénylation de la progérine. La même observation a été réalisée pour la prélamines A, comme cela apparaît sur la Figure 12. Son peptide C-terminal, non prénylé, est détecté dans les cellules traitées par le mélange statine+aminobiphosphonate, alors qu'il est absent des cellules non traitées, dans lesquelles on retrouve le peptide farnésylé et carboxyméthylé Enfin, le traitement pravastatine+zolédronate ne fait pas apparaître la prélamine A géranylgéranylée, à la différence du FTI.

Légende de la figure 13: La lamine A (cellules témoins) et la prélamine A (cellules de souris *Zmpste24*^{-/-}) ont été analysées par spectrométrie de masse (MALDI-ToF). **a**, **b** : portions du spectre correspondant aux peptide tryptiques farnésylés (**a**) et géranylgéranylés (**b**). Chacun des pics est marqué avec la masse théorique (entre parenthèses) du peptide provenant de la digestion par la trypsine de la lamine A ou de la prélamine A Le numéro des résidus est noté en bleu. La séquence des peptides et leur masse sont notées en rouge. Farn = farnesylé ; CM = carboxyméthylé ; Ger = géranylgéranylé.

Légende de la figure 11: Analyse par spectrométrie de masse de protéines extraites de l'enveloppe nucléaire de cellules non traitées (a), de cellules de patients progeria traitées par FTI (2,5 µM, **b**) ou traitées par le mélange pravastatine+zolédronate (1 µM chacun, **c**). La portion des spectres correspondant aux protéines non modifiées, famésylées et géranylgéranylées est montrée en haut, au milieu et en bas de la figure. Chaque pic correspond au peptide tryptique de la progérine et est marqué avec la masse monoisotopique mesurée dans l'expérience et par la masse théorique (entre parenthèses). Le numéro des résidus d'acides aminés est noté en bleu. La séquence des peptides et leur masse sont notées en rouge.

La progérine est majoritairement farnésylée (F) et carboxyméthylée (Cm) dans les cellules non traitées (**a**, panneau du milieu), alors que sous l'effet des FTI ce pic est très réduit et la progérine apparaît géranylgéranylée et phosphorylée (**b**, panneau du bas). Après traitement pravastatine+zolédronate, la progérine non modifiée est la forme prédominante.

Légende de la figure 12: Analyse par spectrométrie de masse de protéines extraites de l'enveloppe nucléaire de fibroblastes non traitées (a), ou traitées par le mélange pravastatine+zolédronate (1 µM chacun, **b**) provenant de souris *Zmpste24*^{-/-}*.* La portion des spectres correspondant aux protéines non modifiées, famésylées et géranylgéranylées est montrée en haut, au milieu et en bas de la figure. Chaque pic correspond au peptide tryptique de la progérine et est marqué avec la masse monoisotopique mesurée dans l'expérience et avec la masse théorique (entre parenthèses). Le numéro des résidus d'acide aminés est noté en bleu. La séquence des peptides et leur masse sont notées en rouge.

Sur cette figure, on remarque que la prélamine A est majoritairement farnésylée (F) et carboxyméthylée (Cm) dans les cellules non traitées (**a**, panneau du milieu), alors les formes non modifiées ou géranylgéranylées ne sont pas détectées. Après traitement par pravastatine+zolédronate, les peptides prénylés ne sont plus détectables et la forme non modifiée de la prélamine A est prédominante (**b**, panneau du haut).

**Le traitement par pravastatine+zolédronate corrige les anomalies nucléaire de cellules de patients progeria et de souris *Zmpste24*^{-/-} en culture, et restaure en partie les mécanismes de réparation des lésions de l'ADN induites par irradiation X (****Figures 14****,** **15****,** **16** **et** **17****).**

Le traitement pravastatine+zolédronate provoque l'apparition de prélamine A dans le noyau de cellules témoins (Fig. 14a), comme dans le noyau de cellules de patients progeria, mais avec une nette amélioration de la morphologie nucléaire dans ces dernières (Fig. 14b). L'analyse quantitative montre une augmentation des anomalies nucléaires dans les cellules de patients progeria avec le nombre de passages, nombre d'anomalies qui diminuent sous l'effet du traitement pravastatine+zolédronate (Fig. 14c). Observées au microscope confocal, les cellules de patient progeria contiennent des agrégats de lamine A/C, des invaginations profondes de la face nucléoplasmique de l'enveloppe nucléaire dans le nucléoplasme (le réticulum nucléaire) marquées par des anticorps anti-calréticuline (Fig. 15a-f). Ces agrégats de lamine A/C sont absents de cellules de sujets témoins (Fig. 14j-l) et disparaissent des cellules de patients progeria sous l'effet du traitement pravastatine+zolédronate (Fig. 14g-i). La localisation de la lamine B1, un constituant farnésylé de la lamina nucléaire, est modifiée sous l'effet du traitement, ce qui confirme que le traitement bloque la prénylation des lamines.

Nous avons vérifié que l'amélioration de la forme des noyaux par le traitement pravastatine+zolédronate est bien liée au blocage de la prénylation de la progérine, en incubant les cellules avec du farnésol et/ou du géranylgénaniol. La supplémentation des cellules par le farnésol et le géranylgéraniol permet aux cellules de synthétiser le farnésyl pyriphosphate et le géranylgéranyl pyrophosphate et donc prényler la progérine même en présence de pravastatine+zolédronate (Fig. 14d). Le farnésol abolit l'effet du traitement pravastatine+zolédronate, ce qui est une autre preuve que l'effet du traitement passe par l'inhibition de la synthèse du farnésyl pyrophosphate. Il est à noter que le géranylgéraniol bloque aussi l'effet du traitement, ce qui prouve que la forme géranylgéranylée de la progérine est elle aussi toxique pour les cellules (Fig. 14d). Les mêmes effets sont observés dans les cellules *Zmpste24*^{*-*/*-*} (Fig. 16a) ce qui suggère que les données concernant la progérine peuvent être étendues à la prélamine A, la protéine accumulée dans les cellules *Zmpste24*^{*-*/}*⁻.* Ni le farnésol, ni le géranylgeraniol ne produisent d'effet sur des fibroblastes témoins, ce qui élimine l'éventualité d'un artefact induit par ces molécules (Fig. 16b).

Enfin, le traitement pravastatine+zolédronate provoque une réduction du nombre de foci de l'histone H2AX phosphorylée, foci directement corrélés avec le nombre de cassures double brin de l'ADN non réparées (Fig. 17).

En conclusion, les données acquises *in vitro* montrent que la combinaison pravastatine+zolédronate inhibe partiellement la farnésylation et la géranylgéranylation, et provoque les modifications attendues de localisation au niveau de la lamina et la redistribution dans le nucléoplasme, de la prélamine A et de la progérine non prénylées dans les cellules *Zmpste24*^{-/-} et de patients progeria. De plus, la diminution de la quantité de la progérine farnésylée au niveau de la lamina et sa relocalisation dans le nucléoplasme explique les effets bénéfiques du traitement sur les cellules des patients progeria.

Légende de la figure 14: Effet synergique de la combinaison pravastatine+zolédronate sur l'accumulation de prélamine A dans des cellules témoins et de patients progeria.
(**a**) Détection immunocytochimique de la lamine A/C et de la prélamine A dans des fibroblastes humains normaux, non traités, traités par pravastatine (60 µM, 12 h), par zolédronate (60 µM, 6 h) seuls ou associés.
(**b**) Détection immunocytochimique de la lamine A/C et de la prélamine A dans des fibroblastes humains normaux et de patients progeria traités pendant 24 h par la combinaison pravastatine+zolédronate (1 µM chacun).
(c) Analyse quantitative de l'effet du traitement pravastatine+zolédronate (1 µM chacun) sur la morphologie nucléaire de cellules de patients progeria. Les cellules traitées ou non ont été immunomarquées avec un anticorps anti-lamine A/C aux passages 8 (p8), 13 (p13) et 20 (p20). Les flèches blanches montrent les noyaux anormaux. (**d**) Analyse quantitative de l'effet du traitement pravastatine+zolédronate (1 µM chacun) sur la morphologie nucléaire de cellules de patients progeria en présence de farnésol, de géranylgéraniol ou des deux composés. Barres d'erreur = moyenne ± erreur standard de la moyenne. Barre d'échelle = 10 µm.

Légende de la figure 15 : Le traitement par pravastatine+zolédronate corrige la morphologie nucléaire et induit une relocalisation partielle des isoformes de la lamine A/C et de la lamine B1 de la lamina nucléaire dans le nucléoplasme, dans des cellules de patients progeria.
(A) la colocalisation de la lamine A/C et de la calréticuline dans ces cellules progeria traitées ou non. Immunofluorescence et microscopie confocale (Leica TCS SP5, pile tridimensionnelle d'images 2048 x 2048 pixels, pas de 0,2 µm, moyenne de 3 lignes, accumulation de 3 images, zoom x 1,7). Les images **a** à **c** de chaque panneau sont des projections de l'intensité moyenne de 27 images de la pile et montrent les tubules du réticulum nucléaire marqués par la calréticuline dans les cellules progeria incubées avec le PBS. Images **d** à l : coupes confocales isolées de 0,2 µm d'épaisseur. Le traitement pravastatine+zolédronate corrige la forme des noyaux des cellules progeria, diminue le nombre des tubules du réticulum nucléaire **(g)** et l'épaisseur de la lamina nucléaire **(h)*.***
(**B**) Colocalisation de la lamine B1 et de la calréticuline. Le traitement pravastatine+zolédronate augmente le signal de marquage nucléoplasmique par la lamine B1, ce qui indique que la farnésylation de cette protéine est en partie inhibée. Barre d'échelle = 5 µm.

Légende de la figure 16 : Les précurseurs du farnésyl-pyrophosphate et du géranyl-géranyl pyrophosphate abolissent l'effet du traitement pravastatine+zolédronate dans les cellules de souris *Zmpste24*^{*-*/*-*} en culture.

Quantification de l'effet de la pravastatine (1 µM) et du zolédronate (1 µM) associés ou non sur la morphologie nucléaire de cellules de souris *Zmpste24*^{*-*/*-*} **(a)** et de souris témoins **(b)** en culture, en présence de farnésol, de géranylgéraniol ou des deux molécules.

Farnésol, géranylgéraniol seuls ou associés abolissent l'effet du traitement pravastatine+zolédronate sur la morphologie nucléaire de cellules *Zmpste24*^{*-*/}*⁻.*

Légende de la figure 17 : Le traitement pravastatine+zolédronate corrige en partie les anomalies de la réparation des cassures double-brin (DSB) de l'ADN dans les cellules de patients progeria.

Des fibroblastes témoins et de patients progeria ont été incubés avec le mélange pravastatine+zolédonate (1 µM chacun) ou avec le PBS et ont été irradiés avec des rayons X (2 Gy). Immunodétection des foci de l'histone H2AX phosphorylée détectés 24 h après irradiation, foci correspondant aux cassures double brin non réparées (images du haut). Marquage nucléaire du DAPI (images du bas).

Courbes du bas : évolution du nombre de foci d'histone H2AX phosphorylée en fonction du temps après irradiation dans les cellules témoins (carré plein) et les cellules progeria (cercle vide) incubées avec le PBS ou traités avec pravastatine+zolédronate. Chaque courbe représente la moyenne ± erreur standard de la moyenne d'au moins 3 expériences.

### Le traitement associant pravastatine+zolédronate améliore le phénotype progéroïde de souris Zmpste24^{-/-} (Figures 18, 19 et 20) :

Les souris *Zmpste24*^{*-*/*-*} et les souris témoins ont été traitées quotidiennement avec la pravastatine, le zolédronate ou la combinaison des deux drogues à une dose qui avait été montrée précédemment comme étant dépourvue de toxicité chez la souris. Comme déjà observé pour les cellules de patient progeria, chacune des drogues isolément, pravastatine ou zolédronate, n'améliore pas la durée de vie des souris *Zmpste24*^{*-*/*-*} (Fig. 19). Au contraire, la combinaison des deux drogues améliore de manière significative le phénotype progéroïde des souris *Zmpste24*^{*-*/}*⁻ :* le traitement améliore la prise de poids, augmente la quantité de graisse sous-cutanée, réduit l'importance de la cyphose et de l'alopécie et augmente la durée de vie. La valeur moyenne de survie passe de 101 à 179 jours et la survie maximum passe de 151 à 222 jours (P < 0,001, Fig. 18c). Il est à remarquer que tous les signes phénotypiques corrigés par le traitement chez les souris sont aussi les caractéristiques de la progeria chez l'homme. Le traitement combiné corrige la diminution de la densité osseuse qui est l'une des caractéristiques des souris *Zmpste24*^{*-*/*-*} et de patients atteints de progeria ou d'un syndrome progéroïde apparenté. La microtomographie osseuse informatisée montre une augmentation de la minéralisation de l'os et de l'épaisseur de la corticale tibiale chez les souris traitées (Fig. 18d). De plus, la quantification des anomalies morphologiques nucléaires dans le foie de souris *Zmpste24*^{*-*/}*⁻, Zmpste24*^{*-*/*-*} et *Zmpste24*^{*-*/*-*} traitées montre que le traitement pravastatine+zolédronate normalise la forme des noyaux de cellules *Zmpste24*^{-/-}) (Fig. 18e). Le traitement corrige en outre l'augmentation de la transcription des gènes cibles de la protéine p53, augmentation qui avait été décrite dans les cellules de souris *Zmpste24*^{*-*/*-*} (Varela et al., 2005 (54 bis)) (Fig. 18f). Enfin, nous avons recherché si le traitement pouvait avoir un effet sur les souris *Lmna*^{*-*/*-*} qui ne peuvent pas accumuler de prélamines A. Le traitement pravastatine+zolédronate n'a aucun effet sur la durée de vie de ces souris (Fig. 20), ce qui est une preuve supplémentaire que ce traitement ne peut agir que chez des souris qui accumulent de la prélamine A farnésylée à l'enveloppe nucléaire.

Légende de la figure 18 : Le traitement pravastatine+zolédronate améliore le phénotype progéroïde de souris Zmpste24^{*-*/*-*} :
**(a)** Photographies représentatives de souris âgées de 3 mois *Zmpste24*^{+/+}, *Zmpste24*^{*-*/*-*} et *Zmpste24*^{*-*/*-*} traitées par la combinaison pravastatine (100 mg/kg/jour) et zolédronate (100 mg/kg/jour). Barre d'échelle = 1 cm. **(b)** Poids de souris âgées de 3 mois *Zmpste24*^{*+*/*+*} (n = 12), *Zmpste24*^{*-*/*-*} (n = 13) et *Zmpste2*^{*-*/*-*} traitées (n = 15). **(c)** Courbes de Kaplan-Meier montrant une augmentation significative de la dure de vie des souris *Zmpste24*^{*-*/}*⁻)* traitées (n = 15) comparée à celle de souris non traitées (n = 13). **(d)** Représentation tridimensionnelle par microtomographie informatisée du tibia de souris *Zmpste24*^{*-*/*-*} traitée et non traitée (image du haut). Le panneau du bas représente le volume osseux relatif (volume du tissu osseux/volume du tibia) et le nombre de trabécules osseux dans les souris *Zmpste24*^{*-*/*-*} non traitées (n = 6) et traitées (n = 5). **(e)** Quantification des anomalies nucléaires des hépatocytes de souris *Zmpste24*^{*+*/}*⁺, Zmpste24*^{*-*/*-*} et *Zmpste24*^{*-*/*-*} traitées. Les flèches blanches montrent les noyaux anormaux. Barre d'échelle = 10 µm. **(f)** Expression relative des gènes cibles de la p53 dans le foie et le coeur de souris *Zmpste24*^{*+*/}*⁺. Zmpste24*^{*-*/*-*} et *Zmpste24*^{*-*/*-*} traitées, analysée par RT-PCR quantitative. *P < 0,05 ; ** P < 0,01 ; *** P < 0,001. Les barres d'erreur représentent la moyenne ± erreur standard de la moyenne.

Légende de la figure 19: Ni la pravastatine seule, ni le zolédronate seul n'augmente la durée de vie des souris Zmpste24^{*-*/*-*} :

Courbes de Kaplan-Meier montrant que la pravastine seule (n = 5) (a), et le zolédronate seul (n = 5) **(b)** ne corrigent pas la durée de vie des souris *Zmpste24*^{*-*/*-*} traitées (diamant vide) et non traitées (cercles pleins, n = 11).

Légende de la figure 20 : Le traitement pravastatine+zolédronate ne corrige pas la durée de vie de souris *Lmna*^{*-*/*-*} :
Courbes de Kaplan-Meier montrant que durée de vie de souris *Lmna*^{*-*/*-*} traitées par pravastine+zolédronate (n= 12, diamant vide), comparée à celle de souris non traitées (cercles pleins, n = 11). Le traitement pravastatine+zolédronate est sans effet sur des souris dépourvues de lamine A/C.

### Résumé/conclusion/perspectives

Plusieurs syndromes progéroïdes humains, dont la progeria de Hutchinson-Gilford, sont causés par l'accumulation au niveau de l'enveloppe nucléaire d'une forme farnésylée de prélamine A délétée (progérine) ou non. La progérine est aussi produite au cours du vieillissement physiologique. Des études précédentes menées avec des cellules de patients atteints de progeria ont montré que les inhibiteurs de la farnésyltransférase (FTI) améliorent la morphologie des noyaux, suggérant que ces inhibiteurs pourraient représenter un traitement pour ces syndromes dévastateurs.

Les inventeurs montrent ici que la prélamine A et la progérine subissent une prénylation alternative par la géranylgéranyltransférase lorsque la farnésyltransférase est inhibée, ce qui pourrait expliquer la faible efficacité des FTI pour améliorer le phénotype de modèles murins de ces syndromes progéroïdes.

Ils montrent aussi que la combinaison d'une statine et d'un aminobiphosphonate inhibe de manière efficace à la fois la farnésylation et la géranylgéranylation de la prélamines A et de la progérine, améliore de manière significative le phénotype de vieillissement de souris chez lesquelles a été inactivée le gène codant la métalloprotéase Zmpste24 impliquée dans la maturation de la prélamines A. L'amélioration du phénotype inclut celle de la courbe de croissance, du poids, la lipodystrophie, la chute des poils et les anomalies osseuses.

De plus, la durée de vie de ces souris est augmentée de manière substantielle.

Ces données ouvrent une nouvelle approche thérapeutique des syndromes progéroïdes humains avec accumulation de protéines prénylées à l'enveloppe nucléaire.

Le traitement pravastatine+aminobiphosphonate est en cours d'application à Marseille pour les 3 années à venir sur des enfants atteints de progéria dans le cadre d'une essai thérapeutique européen (15 enfants) placé sous la responsabilité du Pr. Nicolas Lévy, financé par le Ministère de la Santé (PHRC 2008) et l'Association Française contre les Myopathies (AFM) et qui a reçu l'autorisation de l'AFSSAPS et du CCP Sud-Méditerranée.

Le même traitement va être prochainement donné à Rome, sous la responsabilité du Pr. Giuseppe Novelli, à des patients atteints de dysplasie acromandibulaire, un autre syndropme progéroïde avec accumulation de prélamine A farnésylée.

### Liste des références

(1) Basso AD, Kirschmeier P, Bishop WR. Farnesyl transferase inhibitors. J Lipid Res 47:15-31, 2006.
(2) Biamonti G, Giacca M, Perini G, Contreas G, Zentilin L, Weighardt F, Guerra M, Della Valle G, Saccone S, Riva S et al. The gene for a novel human lamin maps at a highly transcribed locus of chromosome 19 which replicates at the onset of S-phase. Mol Cell Biol 12:3499-3506, 1992.
(3) Bishop WR, Kirschmeier P, Baun C. Famesyl transferase inhlbitors: mechanism of action, translational studies and clinical évaluation. Cancer Biol Ther 2:S96-104, 2003.
(4) Broers JL, Hutchinson CJ, Ramaekers FC. Laminopathies. J Pathol 204:478-488, 2004.
(5) Broers JLV, Ramaekers FCS, Bonne G, Ben Yaou R, Hutchinson CJ. Nuclear lamins: laminopathies and their role in premature, aging. Physiol Rev 86:967-1008, 2006.
(6) Capell BC, Erdos MR, Madigan JP, Fiordalisi JJ, Varga R, Conneely KN, Gordon LB, Der CJ, Cox AD, Collins FS. Inhibiting farnesylation of progerin prevents the characteristic nuclear blebbing of Hutchinson-Gilford progeria syndrome. Proc Natl Acad Sci USA 102:12879-12884, 2005.
(7) De Sandre-Giovannoli A, Bernard R, Cau P, Navarro C, Amiel J, Boccaccio I, Lyonnet S, Stewart CL, Munnich A, Le Merrer M, Levy N. Lamin A truncation in Hutchinson-Gilford progeria. Science 300:2055, 2003.
(8) Demyanets S, Kaun C, Pfaffenberger S, Philipp J. Hohensinner PJ, Rega G, Pammer J, Maurer G, Huber K, Wojta J. Hydroxymethylglutaryl-coenzyme A reductase inhibitors induce apoptosis in human cardiac myocytes in vitro. Biochem Pharmacol 71:1324-1330, 2006.
(9) Duque G, Rivas D. Age-related changes in Lamin A/C expression in the osteoarticular system: laminopathies as a potentiel new aging mechanism. Mech Aging Dev 127:378-383, 2006.
(**10**) Efuet ET, Keyomarsi K. Farnesyl and geranylgeranyl transferase inhibitors induce G1 arrest by targeting the proteasome. Cancer Res 66:1040-1051, 2006.
(11) Eriksson M, Brown WT, Gordon LB, Glynn MW, Singer J, Scott L, Erdos MR, Robbins CM, Moses TY, Berglund P, Dutra A, Pak E, Durkin S, Csoka AB, Boehnke M, Glover TW, Collins FS. Recurrent de novo point mutation in lamin A cause Hutchinson-Gilford progerià syndrome. Nature 423:293-298, 2003.
(12) Evans M, Rees A The myotoxicity of statins. Cur Op Lipid, 13:415-420, 2002.
(13) Flint OP, Masters BA, Gregg RE, Durham SK. HMG CoA reductase inhibitor-induced myotoxicity: pravastatin and lovastatin inhibit the geranylgeranylation of low-molecular-weight proteins in neonatal rat muscle cell culture. Tox Appl Pharmacol 145:99-110, 1997.
(14) Fong LG, Frost D, Meta M, Qiao X, Yang SH, Coffinier C, Young SG. A protein famesyltransferase inhibitor ameliorates disease in a mouse model of progeria. Science,311 : 1621-1623, 2006.
(15) Fong LG, Ng JK, Lammerding J, Vickers TA, Meta M, Coté N, Gavino B, Qiao X, Chang SY, Young SR, Yang SH, Stewart CL, Lee RT, Bennett CF, Bergo MO, Young SG. Prelamin A and Lamin A appear to be dispensable in the nuclear lamina. J Clin Invest 116:743-752, 2006.
(16) Fong LG, Ng JK, Meta M, Cote N, Yang SH, Stewart CL, Sullivan T, Burghardt A, Majumdar S, Reue K, Bergo MO, Young SG. Heterozygosity for Lmna deficiency eliminates the progeria-like phenotypes in Zmpste24-deficient mice. Proc Natl Acad Sci USA 101:18111-18116, 2004.
(17) Glynn MW, Glover TW. Incomplete processing of mutant lamin A In Hutchison-Gilford progeria leads to nuclear abnormalities, which are reversed by farnesyltransferase inhibition. Hum Mol Genet 14:2959-2969, 2005.
(18) Goldman RD, Shumaker DK, Erdos MR, Eriksson M, Goldman AE, Gordon LB, Gruenbaum Y, Khuon S, Mendez M, Varga R, Collins FS. Accumulation of mutant lamin A causes progressive changes in nuclear architecture in Hutchinson-Gilford progeria syndrome. Proc Natl Acad Sci USA 101:8963-8968, 2004.
(19) Gruenbaum Y, Margalit A, Goldman RD, Shumaker DK, Wilson KL. The nuclear lamina comes of age. Nat Mol Cell Biol 6:21-31, 2005.
(20) Hampton R, Dimster-Denk D, Rine J. The biology of HMG-CoA reductase: the pros of contra-regulation. Trends Biochem Sci 21:140-145,1996.
(21) Harborth J, Elbashir SM, Bechert K, Tuschl T, Weber K. Identification of essential genes in cultured mammalian cells using small interfering RNAs. J Cell Sci 114:4557-4565, 2001.
(22) Hegele RA, Cao H, Liu DM, Costain GA, Charkon-Menys V, Rodger NW, Durrington PN. Sequencing of reannotated LMNB2 gene reveals novel mutations in patients with acquired partial lipodystrophy. Am J Hum Genet 79:383-389, 2006.
(23) Hildebrand T, Ruegsegger P. A new method for the model independent assessment of thickness in three dimensional images. J Microsc 185:67-75, 1997.
(24) Hoffmann GF, Charpentier C, Mayatepek E, Mancini J, Leichsenring M, Gibson KM, Divry P, Hrebicek M, Lehnert W, Sartor K. Clinical and biochemical phenotype in 19 patients with mevalonic aciduria. Pediatrics 91:915-921, 1993.
(25) Huang S, Chen L, Libina N, Janes J, Martin GM, Campisi J, Oshima J._Correction of cellular phenotypes of Hutchinson-Gilford Progeria cells by RNA interference. Hum Genet. 2005 Oct 6;: 1-7
(26) Hutchinson CJ, Worman HJ. A-type lamins: guardians of the soma? Nat Cell Biol 6:1062-1067, 2004.
(27) Kusuyama T, Omura T, Nishiya D, Enomoto S, Matsumoto R, Murata T, Takeuchi K, Yoshikawa J, Yoshiyama M. The effects of HMG-CoA reductase inhibitor on vascular progenitor cells. J Pharmacol Sci 1001:344-349, 2006.
(28) Leung KF, Baron R, Seabra MC. Geranylgeranylation of Rab GTPases. J Lipid Res 47:467-475, 2006.
(29) Lévy N, Cau P. Anomalies du noyau et maladies. Pour la Science 313:2-7, 2003.
(30) Lin F, Worman HJ. Structural organization of the human gene (LMNB1) encoding nuclear lamin B1. Genomics 27:230-236, 1995.
(31) Lin F, Worman HJ. Structural organization of the human gene encoding nuclea lamin A and nuclear lamin C. J Biol Chem 268:16321-16326, 1993.
(32) Liu Y, Wang Y, Rusinol AE, Sinensky MS, Liu J, Shell SM, Zou Y. Involvement of xeroderma pigmentosum group A (XPA) in progeria arising from defective maturation of prelamin A. FASEB J. 2007 Sep 11;
(33) Mattout A, Dechat T, Adam SA, Goldman RD, Gruenbaum Y. Nuclear lamins, diseases and aging. Cur Op Cell Biol 18:335-341, 2006.
(34) McClintock D, Ratner D, Lokuge M, Owens DM, Gordon LB, Collins FS, Djhabali K. The mutatnt form of lamin A that causes Hutchinbson-Gilford progeria is a biomarker of cellular aging in human skin. PloS One, 2007, 2, e1269
(35) Navarro CL, Cadinanos J, De Sandre-Giovannoli A, Bernard R, Courrier S, Boccaccio I, Boyer A, Kleijer WJ, Wagner A, Giuliano F, Beemer FA, Freije JM, Cau P, Hennekam RC, Lopez-Otin C, Badens C, Levy N. Loss of ZMPSTE24 (FACE-1) causes autosomal recessive restrictive dermopathy and accumulation of Lamin A precursors. Hum Mol Genet 14:1503-1513, 2005.
(36) Navarro CL, De Sandre-Giovannoli A, Bernard R, Boccaccio I, Boyer A, Genevieve D, Hadj-Rabia S, Gaudy-Marqueste C, Smitt HS, Vabres P, Faivre L, Verloes A, Van Essen T, Flori E, Hennekam R, Beemer FA, Laurent N, Le Merrer M, Cau P, Levy N. Lamin A and ZMPSTE24 (FACE-1) defects cause nuclear disorganization and identify restrictive dermopathy as a lethal neonatal laminopathy. Hum Mol Genet 13:2493-2503,2004.
(37) Padiath QS, Saigoh K, Schiffmann R, Asahara H, Yamada T, Koeppen A, Hogan K, Ptacek LJ, Fu YH. Lamin B1 duplications cause autosomal dominant leukodystrophy. Nature Genet 38:1114-1123, 2006.
(38) Pendas AM, Zhou Z, Cadinanos J, Freije JM, Wang J, Hultenby K, Astudillo A, Wernerson A, Rodriguez F, Tryggvason K, Lopez-Otin C. Defective prelamin A processing and muscular and adipocyte alterations in Zmpste24 metalloproteinase-deficient mice. Nat Genet 31:94-99, 2002.
(39) Reid TS, Terry KL, Casey PJ, Beese LS. Crystallographic analysis of CaaX prenyltransferases complexed with substrates defines rules of protein substrate selectivity. J Mol Biol 343:417-433, 2004.
(40) Scaffidi P, Misteli T. Lamin A-dependent nuclear defects in human aging. Sciencexpress, 27 avril 2006.
(41) Scaffidi P, Misteli T. Reversal of the cellular phenotype in the premature aging disease Hutchinson-Gilford progeria syndrome. Nat Med 11:440-445, 2005.
(42) Scaffidi P, Misteli T. Reversal of the cellular phenotype in the premature aging disease Hutchinson-Gilford progeria syndrome. Nature Med 11:440-445, 2005.
(43) Shelton KR, Egle PM, Cochran DL. Nuclear envelope proteins: identfication of lamin B subtypes. Biochem Biophys Res Comm 103:975-981, 1981.
(44) Shumaker DK, Kuczmarski ER, Goldman RD. The nucleoskeleton: lamins an actin are major players in essential nuclear functions. Curr Op Cell Biol 15:358-366, 2003.
(45) Stewart C, Burke B. Teratocarcinoma stem cells and early mouse embryos contain only a single major lamin polypeptide closely resembling lamin B. Cell 51:383-392, 1987.
(46) Takedaa M, Noshiroa R, Onozatob ML, Tojob A, Hasannejada H, Huangc XL, Narikawac S, Endoua H. Evidence for a role of human organic anion transporters in the muscular side effects of HMG-CoA reductase inhibitors. Eur J Pharm 483:133- 138, 2004.
(47) Toth JI, Yang SH, Qiao X, Beigneux AP, Gelb MH, Moulson CL, Miner JH, Young SG, Fong LG. Blocking protein farnesyltransferase improves nuclear shape in fibroblasts from humans with progeroid syndromes Proc Natl Acad Sci USA 102:12873-12878, 2005.
(48) Tsai MY, Wang S, Heidinger JM, Shumaker DK, Adam SA, Goldman RD, Zheng Y. A mitotic lamin B matrix induced by RanGTP required for spindle assembly. Science 311:1887-1893, 2006.
(49) Varela I, Cadinanos J, Pendas AM, Gutierrez-Femandez A, Folgueras AR, Sanchez LM, Zhou Z, Rodriguez FJ, Stewart CL, Vega JA, Tryggvason K, Freije JM, Lopez-Otin C. Accelerated ageing in mice deficient in Zmpste24 protease is linked to p53 signalling activation. Nature 437:564-568, 2005.
(50) Vergnes L, Peterfy M, Bergo MO, Young SG, Reue K. Lamin B1 is required for mouse development and nuclear integrity. Proc Natl Acad Sci USA 101:10428-10433, 2004.
(51) Winter-Vann AM, Casey PJ. Post-prenylation-processing enzymes as new targets in oncogenesis. Nat Rev Cancer 5:405-412, 2005.
(52) Wydner KL, McNeil JA, Lin F, Worman HJ, Lawrence JB. Chromosomal assignment of human nuclear envelope protein genes LMNA, LMNA1 and LBR by fluorescence in situ hybridization. Genomics 32:474-478,1996.
(53) Young SG, Meta M, Yang SH, Fong LG. Prelamin A farnesylation and progeroid syndromes. J Biol Chem 281:39741-39745, 2006.
(54) Zastrow MS, Vlcek S, Wilson KL. Proteins that bind A-type lamins: integrating isolated clues. J Cell Sci-117:979-987, 2004.
(54 bis) Varela, I. Pereira, S. Ugalde, A. P. Navarro, C. L. Suarez, M. F. Cau, P. Cadinanos, J. Osorio, F. G. Foray, N. Cobo, J. de Carlos, F. Levy; N. Freije, .J. M. Lopez-Otin, C. Combined treatment with statins and aminobisphosphonates extends longevity in a mouse model of human premature aging. Nature Medicine 14: 767-772, 2008.

### VIH

( 55 ) Achanta, G., R. Sasaki, L. Feng, J.S. Carew, W. Lu, H. Pelicano, M.J. Keating, and P. Huang. 2005. Novel role of p53 in maintaining mitochondrial genetic stability through interaction with DNA Pol gamma. Embo J. 24:3482-92.
( 56 ) Adler, A.S., S. Sinha, T.L. Kawahara, J.Y. Zhang, E. Segai, and H.Y. Chang. 2007. Motif module map reveals enforcement of aging by continual NF-{kappa}B activity. Genes Dev. 21:3244-57.
( 57 ) Agarwal, A.K., J.P. Fryns, R.J. Auchus, and A. Garg. 2003. Zinc metalloproteinase, ZMPSTE24, is mutated in mandibuloacral dysplasia. Hum Mol Genet. 12:1995-2001.
( 58 ) Azzam, R., L. Lal, S.L. Goh, K. Kedzierska, A. Jaworowski, E. Naim, C.L. Cherry, S.L. Wesselingh, J. Mills, and S.M. Crowe. 2006. Adverse effects of antiretroviral drugs on HIV-1-infected and - uninfected human monocyte-derived macrophages. J Acquir Immune Defic Syndr. 42:19-28.
( 59 ) Balaban, R.S., S. Nemoto, and T. Finkel. 2005. Mitochondria, oxidants, and aging. Cell, 120:483-95.
( 60 ) Barbaro, G. 2003. Pathogenesis of HIV-associated cardiovascular disease. Adv Cardiol. 40:49-70.
( 61 ) Bartke, A. 2005. Minireview: role of the growth hormone/insulin-like growth factor system in mammalian aging. Endocrinology. 146:3718-23.
( 62 ) Bartoli, M., and I. Richard. 2005. Calpains in muscle wasting. Int J Biochem Cell Biol. 37:2115-33.
( 63 ) Bauer, J.H., and S.L. Helfand. 2006. New tricks of an old molecule: lifespan regulation by p53. Aging Cell. 5:437-40.
( 64 ) Baxter, J.D., J.M. Schapiro, C.A. Boucher, V.M. Kohlbrenner, D.B. Hall, J.R. Scherer, and D.L. Mayers. 2006. Genotypic changes in human immunodeficiency virus type 1 protease associated with reduced susceptibility and virologic response to the protease inhibitor tipranavir. J Virol. 80:10794-801.
( 65 ) Ben-Porath, I., and R.A. Weinberg. 2005. The signais and pathways activating cellular senescence. Int J Biochem Cell Biol. 37:961-76.
( 66 ) Bender, A., K.J. Krishnan, C.M. Morris, G.A. Taylor, A.K. Reeve, R.H. Perry, E. Jaros, J.S. Hersheson, J. Betts, T. Klopstock, R.W. Taylor, and D.M. Turnbull. 2006. High levels of mitochondrial DNA deletions in substantia nigra neurons in aging and Parkinson disease. Nat Genet. 38:5.15-7.
( 67 ) Benesic; A., M. Zilly, F. Kluge, B., Weissbrich, R. Winzer, H. Klinker, and P. Langmann. 2004. Lipid lowering therapy with fluvastatin and pravastatin in patients with HIV infection and antiretroviral therapy: comparison of efficacy and interaction with indinavir. Infection. 32:229-33.
( 68 ) Bensaad, K., and K.H. Vousden. 2005. Savior and slayer: the two faces of p53. Nat Med. 11:1278-9.
( 69 ) Bordone, L., and L. Guarente. 2005. Calorie restriction, SIRT1 and metabolism: understanding longevity. Nat Rev Mol Cell Biol. 6:298-305.
( 70 ) Bourlier, V., A. Zakaroff-Girard, S. De Barros, C. Pizzacalla, V.D. de Saint Front, M. Lafontan, A. Bouloumie, and J. Galitzky. 2005. Protease inhibitor treatments reveal specific involvement of matrix metalloproteinase-9 in human adipocyte differentiation. J Pharmacol Exp Ther. 312:1272-9.
( 71 ) Broers, J.L., F.C. Ramaekers, G. Bonne, R.B. Yaou, and C.J. Hutchison. 2006. Nuclear lamins: laminopathies and their role in premature ageing. Physiol Rev. 86:967-1008.
( 72 ) Brokstad, K.A., K.H. Kalland, W.C. Russell, and D.A. Matthews. 2001. Mitochondrial protein p32 can accumulate in the nucleus. Biochem Biophys Res Commun. 281:1161-9.
( 73 ) Brosh, R.M., Jr., P. Karmakar, J.A. Sommers, Q. Yang, X.W. Wang, E.A. Spillare, C.C. Harris, and V.A. Bohr. 2001. p53 Modulates the exonuclease activity of Werner syndrome protein. J Biol Chem. 276:35093-102.
( 74 ) Brown, T.T., and R.B. Qaqish. 2006. Antiretroviral therapy and the prevalence of osteopenia and osteoporosis: a meta-analytic review. Aids. 20:2165-74.
( 75 ) Bukrinsky, M., and D. Sviridov. 2006. Human immunodeficiency virus infection and macrophage cholesterol metabolism. J Leukoc Biol. 80:1044-51.
( 76 ) Calvo, S., M. Jain, X. Xie, S.A. Sheth, B. Chang, O.A. Goldberger, A. Spinazzola, M. Zeviani, S.A. Carr, and V.K. Mootha. 2006. Systematic identification of human mitochondrial disease genes through integrative genomics. Nat Genet. 38:576-82.
( 77 ) Campisi, J. 2004. Fragile fugue: p53 in aging, cancer and IGF signaling. Nat Med. 10:231-2.
( 78 ) Cao, K., B.C. Capell, M.R. Erdos, K. Djabali, and F.S. Collins. 2007. A lamin A protein isoform overexpressed in Hutchinson-Gilford progeria syndrome interferes with mitosis in progeria and normal cells. Proc Natl Acad Sci U S A. 104:4949-54.
( 79 ) Capeau, J., J. Magre, O. Lascols, M. Caron, V. Bereziat, C. Vigouroux, and J.P. Bastard. 2005. Diseases of adipose tissue: genetic and acquired lipodystrophies. Biochem Soc Trans. 33:1073-7.
( 80 ) Caron, M., M. Auclair, B. Donadille, V. Bereziat, B. Guerci, M. Laville, H. Narbonne, C. Bodemer, O. Lascols, J. Capeau, and C. Vigouroux. 2007. Human lipodystrophies linked to mutations in A-type lamins and to HIV protease inhibitor therapy are both associated with prelamin A accumulation, oxidative stress and premature cellular senescence. Cell Death Differ. 14:1759-67.
( 81 ) Caron, M., M. Auclair, H. Sterlingot, M. Komprobst, and J. Capeau. 2003. Some HIV protease inhibitors alter lamin A/C maturation and stability, SREBP-1 nuclear localization and adipocyte differentiation. Aids. 17:2437-44.
( 82 ) Caron, M., M. Auclair, C. Vigouroux, M. Glorian, C. Forest, and J. Capeau. 2001. The HIV protease inhibitor indinavir impairs sterol regulatory element-binding protein-1 intranuclear localization, inhibits preadipocyte differentiation, and induces insulin resistance. Diabetes. 50:1378-88.
( 83 ) Casau, N.C. 2005. Perspective on HIV infection and aging: emerging research on the horizon. Clin Infect Dis. 41:855-63.
( 84 ) Cawthon, R.M. 2002. Telomere measurement by quantitative PCR. Nucleic Acids Res. 30:e47.
( 85 ) Chattopadhyay, C., D. Hawke, R. Kobayashi, and S.N. Maity. 2004. Human p32, interacts with B subunit of the CCAAT-binding factor, CBF/NF-Y, and inhibits CBF-mediated transcription activation in vitro. Nucieic Acids Res. 32:3632-41.
( 86 ) Chen, C., X.H. Lu, S. Yan, H. Chai, and Q. Yao. 2005. HIV protease inhibitor ritonavir increases endothelial monolayer permeability. Biochem Biophys Res Commun. 335:874-82.
( 87 ) Chen, C.D., S. Podvin, E. Gillespie, S.E. Leeman, and C.R. Abraham. 2007. Insulin stimulates the cleavage and release of the extracellular domain of Klotho by ADAM10 and ADAM17. Proc Natl Acad Sci U S A. 104:19796-801.
( 88 ) Coffinier, C., S.E. Hudon, E.A. Farber, S.Y. Chang, C.A. Hrycyna, S.G. Young, and L.G. Fong. 2007. HIV protease inhibitors block the zinc metalloproteinase ZMPSTE24 and lead to an accumulation of prelamin A in cells. Proc Natl Acad Sci U S A. 104:13432-7.
( 89 ) Cohan, G.R. 2006. HIV-associated hypogonadism. AIDS Read. 16:341-5, 348, 352-4.
( 90 ) Colgan, S.M., D. Tang, G.H. Werstuck, and R.C. Austin. 2007. Endoplasmic reticulum stress causes the activation of sterol regulatory element binding protein-2. Int J Biochem Cell Biol. 39:1843-51.
( 91 ) Costelli, P., and F.M. Baccino. 2003. Mechanisms of skeletal muscle depletion in wasting syndromes: role of ATP-ubiquitin-dependent proteolysis. Curr Opin Clin Nutr Metab Care. 6:407-12.
(92) Costelli, P., P. Reffo, F. Penna, R. Autelli, G. Bonelli, and F.M. - Baccino. 2005. Ca(2+)-dependent proteolysis in muscle wasting: Int J Biochem Cell Biol. 37:2134-46.
( 93 ) Csoka, A.B., S.B. English, C.P. Simkevich, D.G. Ginzinger, A.J. Butte, G.P. Schatten, F.G. Rothman, and J.M. Sedivy. 2004. Genome-scale expression profiling of Hutchinson-Gilford progeria syndrome reveals widespread transcriptional misregulation leading to mesodermal/mesenchymal defects and accelerated atherosclerosis. Aging Cell. 3:235-43.
( 94 ) De Barros, S., A. Zakaroff-Girard, M. Lafontan, J. Galitzky, and V. Bourlier. 2007. Inhibition of human preadipocyte proteasomal activity by HIV protease inhibitors or specific inhibitor lactacystin leads to a defect in adipogenesis, which involves matrix metalloproteinase-9. J Pharmacol Exp Ther. 320:291-9.
( 95 ) de Noronha, C.M., M.P. Sherman, H.W. Lin, M.V. Cavrois, R.D. Moir, R.D. Goldman, and W.C. Greene. 2001. Dynamic disruptions in nuclear envelope architecture and integrity induced by HIV-1 Vpr. Science. 294:1105-8.
( 96 ) de Oliveira, R.M. 2006. Klotho RNAi induces premature senescence of human cells via a p53/p21 dependent pathway. FEBS Lett. 580:5753-8.
( 97 ) de Saint Martin, L., O. Vandhuick, P. Guillo, V. Bellein, L. Bressollette, N. Roudaut, A. Amaral, and E. Pasquier. 2006. Premature atherosclerosis in HIV positive patients and cumulated time of exposure to antiretroviral therapy (SHIVA study). Atherosclerosis. 185:361-7.
( 98 ) De Sandre-Giovannoli, A., R. Bernard, P. Cau, C. Navarro, J. Amiel, I. Boccaccio, S. Lyonnet, C.L. Stewart, A. Munnich, M. Le Merrer, and N. Levy. 2003. Lamin a truncation in Hutchinson-Gilford progeria. Science. 300:2055.
( 99 ) Dechat, T., T. Shimi, S.A. Adam, A.E. Rusinol, D.A. Andres, H.P. Spielmann, M.S. Sinensky, and R.D. Goldman. 2007. Alterations in mitosis and cell cycle progression caused by a mutant lamin A known to accelerate human aging. Proc Natl Acad Sci U S A. 104:4955-60.
( 100 ) Dixit, V., N. Hariparsad, F. Li, P. Desai, K.E. Thummel, and J.D. Unadkat. 2007. Cytochrome P450 enzymes and transporters induced by anti-human immunodeficiency virus protease inhibitors in human hepatocytes: implications for predicting clinical drug interactions. Drug Metab Dispos. 35:1853-9.
( 101 ) Dressman, J., J. Kincer, S.V. Matveev, L. Guo, R.N. Greenberg, T. Guerin, D. Meade, X.A. Li, W. Zhu, A. Uittenbogaard, M.E. Wilson, and E.J. Smart. 2003. HIV protease inhibitors promote atherosclerotic lesion formation independent of dyslipidemia by increasing CD36-dependent cholesteryl ester accumulation in macrophages. J Clin Invest. 111:389-97.
( 102 ) Dunn, B.M., M.M. Goodenow, A. Gustchina, and A. Wlodawer. 2002. Retroviral proteases. Genome Biol. 3:REVIEWS3006.
( 103 ) Effros, R.B. 2000. Telomeres and HIV disease. Microbes Infect. 2:69-76.
( 104 ) Eriksson, M., W.T. Brown, L.B. Gordon, M.W. Glynn, J. Singer, L. Scott, M.R. Erdos, C.M. Robbins, T.Y. Moses, P. Berglund, A. Dutra, E. Pak, S. Durkin, A.B. Csoka, M. Boehnke, T.W. Glover, and F.S. Collins. 2003. Recurrent de novo point mutations in lamin A cause Hutchinson-Gilford progeria syndrome. Nature. 423:293-8.
( 105 ) Ferreira, C.E., A.M. Pinto-Neto, D.M. Conde, L. Costa-Paiva, S.S. Morais, and J. Magalhaes. 2007. Menopause symptoms in women infected with HIV: prevalence and associated factors. Gynecol Endocrinol. 23:198-205.
( 106 ) Fuster, J.J., S.M. Sanz-Gonzalez, U.M. Moll, and V. Andres. 2007. Classic and novel roles of p53: prospects for anticancer therapy. Trends Mol Med. 13:192-9.
( 107 ) Gharakhanian, S., F. Boccara, and J. Capeau. 2006. Statins in HIV-associated lipodystrophy and metabolic syndrome: is there a missing link? Aids. 20:1061-3.
( 108 ) Ghibelli, L., F. Mengoni, M. Lichtner, S. Coppola, M. De Nicola, A. Bergamaschi, C. Mastroianni, and V. Vullo. 2003. Anti-apoptotic effect of HIV protease inhibitors via direct inhibition of calpain. Biochem Pharmacol. 66:1505-12.
( 109 ) Gil, M.E., and T.L. Coetzer. 2004. Real-time quantitative PCR of telomere length. Mol Biotechnol. 27:169-72.
( 110 ) Gilson, E., and V. Geli. 2007. How telomeres are replicated. Nat Rev Mol Cell Biol. 8:825-38.
( 111 ) Giorgio, M., E. Migliaccio, F. Orsini, D. Paolucci, M. Moroni, C. Contursi, G. Pelliccia, L. Luzi, S. Minucci, M. Marcaccio, P. Pinton, R. Rizzuto, P. Bernardi, F. Paolucci, and P.G. Pelicci. 2005. Electron transfer between cytochrome c and p66Shc generates reactive oxygen species that trigger mitochondrial apoptosis. Cell. 122:221-33.
(112) Giorgio, M., M. Trinei, E. Migliaccio, and P.G. Pelicci. 2007. Hydrogen peroxide: a metabolic by-product or a common mediator of ageing signais? Nat Rev Mol Cell Biol. 8:722-8.
( 113 ) Glass, D.J. 2003. Signalling pathways that mediate skeletal muscle hypertrophy and atrophy. Nat Cell Biol. 5:87-90.
( 114 ) Goldman, R.D., D.K. Shumaker, M.R. Erdos, M. Eriksson, A.E. Goldman, L.B. Gordon, Y. Gruenbaum, S. Khuon, M. Mendez, R. Varga, and F.S. Collins. 2004. Accumulation of mutant lamin A causes progressive changes in nuclear architecture in Hutchinson-Gilford progeria syndrome. Proc Natl Acad Sci U S A. 101:8963-8.
( 115 ) Granfors, M.T., J.S. Wang, L.I. Kajosaari, J. Laitila, P.J. Neuvonen, and J.T. Backman. 2006. Differential inhibition of cytochrome P450 3A4, 3A5 and 3A7 by five human immunodeficiency virus (HIV) protease inhibitors in vitro. Basic Clin Pharmacol Toxicol. 98:79-85.
( 116 ) Graziewicz, M.A., B.J. Day, and W.C. Copeland. 2002. The mitochondrial DNA polymerase as a target of oxidative damage. Nucleic Acids Res. 30:2817-24.
( 117 ) Gredilla, R., and G. Barja. 2005. Minireview: the role of oxidative stress in relation to caloric restriction and longevity. Endocrinology. 146:3713-7.
( 118 ) Grillari, J., H. Katinger, and R. Voglauer. 2006. Aging and the ubiquitinome: traditional and non-traditional functions of ubiquitin in aging cells and tissues. Exp Gerontol. 41:1067-79.
( 119 ) Guarente, L., and F. Picard. 2005. Calorie restriction-the SIR2 connection. Cell. 120:473-82.
(120) Gupta, A.K., G.J. Cerniglia, R. Mick, W.G. McKenna, and R.J. Muschel. 2005. HIV protease inhibitors block Akt signaling and radiosensitize tumor cells both in vitro and in vivo. Cancer Res. 65:8256-65.
( 121 ) Hamel, F.G., J. Fawcett, B.T. Tsui, R.G. Bennett, and W.C. Duckworth. 2006. Effect of nelfinavir on insulin metabolism, proteasome activity and protein degradation in HepG2 cells. Diabetes Obes Metab. 8:661-8.
( 122 ) Hardy, K., L. Mansfield, A. Mackay, S. Benvenuti, S. Ismail, P. Arora, M.J. O'Hare, and P.S. Jat. 2005. Transcriptional networks and cellular senescence in human mammary fibroblasts. Mol Biol Cell. 16:943-53.
( 123 ) Hayden, M.S., and S. Ghosh. 2004. Signaling to NF-kappaB. Genes Dev. 18:2195-224.
( 124 ) Heessen, S., and M. Fornerod. 2007. The inner nuclear envelope as a transcription factor resting place. EMBO Rep. 8:914-9.
( 125 ) Helton, E.S., and X. Chen. 2007. p53 modulation of the DNA damage response. J Cell Biochem. 100:883-96.
( 126 ) Hennekam, R.C. 2006. Hutchinson-Gilford progeria syndrome: Review of the phenotype. Am J Med Genet A*.*
( 127 ) Hertel, J., H. Struthers, C.B. Horj, and P.W. Hruz. 2004. A structural basis for the acute effects of HIV protease inhibitors on GLUT4 intrinsic activity. J Biol Chem. 279:55147-52.
( 128 ) Holzenberger, M., L. Kappeler, and C. De Magalhaes Filho. 2004. IGF-1 signaling and aging. Exp Gerontol. 39:1761-4.
( 129 ) Hruz, P.W. 2006. Molecular Mechanisms for Altered Glucose Homeostasis in HIV Infection. Am J Infect Dis. 2:187-192.
( 130 ) Huang, S., R.A. Risques, G.M. Martin, P.S. Rabinovitch, and J. Oshima. 2008. Accelerated telomere shortening and replicative senescence in human fibroblasts overexpressing mutant and wild-type lamin A. Exp Cell Res. 314:82-91.
( 131 ) Hudson, G., and P.F. Chinnery. 2006. Mitochondrial DNA polymerase-gamma and human disease. Hum Mol Genet. 15 Spec No 2:R244-52.
( 132 ) Hui, D.Y. 2003. Effects of HIV protease inhibitor therapy on lipid metabolism. Prog Lipid Res. 42:81-92.
( 133 ) Irminger-Finger, I. 2007. Science of cancer and aging. J Clin Oncol. 25:1844.-51.
( 134 ) Jacque, J.M., and M. Stevenson. 2006. The inner-nuclear-envelope protein emerin regulates HIV-1 infectivity. Nature. 441:641-5.
( 135 ) Jiang, B., V.Y. Hebert, Y. Li, J.M. Mathis, J.S. Alexander, and T.R. Dugas. 2007. HIV antiretroviral drug combination induces endothelial mitochondrial dysfunction and reactive oxygen species production, but not apoptosis. Toxicol Appl Pharmacol. 224:60-71.
( 136 ) Jiang, B., V.Y. Hebert, J.H. Zavecz, and T.R. Dugas. 2006. Antiretrovirals induce direct endothelial dysfunction in vivo. J Acquir Immune Defic Syndr. 42:391-5.
( 137 ) Jiang, J., Y. Zhang, A.R. Krainer, and R.M. Xu. 1999. Crystal structure of human p32, a doughnut-shaped acidic mitochondrial matrix protein. Proc Natl Acad Sci U S A. 96:3572-7.
( 138 ) John, M., C.B. Moore, I.R. James, D. Nolan, R.P. Upton, E.J. McKinnon, and S.A. Mallal. 2001. Chronic hyperlactatemia in HIV-infected patients taking antiretroviral therapy. Aids. 15:717-23.
( 139 ) Jones, R., S. Sawleshwarkar, C. Michailidis, A. Jackson, S. Mandalia, J. Stebbing, M. Bower, M. Nelson, B.G. Gazzard, and G.J. Moyle. 2005. Impact of antiretroviral choice on hypercholesterolaemia events: the role of the nucleoside reverse transcriptase inhibitor backbone. HIV Med. 6:396-402.
( 140 ) Kenyon, C. 2005. The plasticity of aging: insights from long-lived mutants. Cell. 120:449-60.
( 141 ) Kim, K.S., Y.B. Seu, S.H. Baek, M.J. Kim, K.J. Kim, J.H. Kim, and J.R. Kim. 2007. Induction of cellular senescence by insulin-like growth factor binding protein-5 through a p53-dependent mechanism. Mol Biol Cell. 18:4543-52.
( 142 ) Kim, R.J., C.G. Wilson, M. Wabitsch, M.A. Lazar, and C.M. Steppan. 2006. HIV protease inhibitor-specific alterations in human adipocyte differentiation and metabolism. Obesity (Silver Spring). 14:994-1002.
( 143 ) Kohler, J.J., and W. Lewis. 2007. A brief overview of mechanisms of mitochondrial toxicity from NRTIs. Environ Mol Mutagen. 48:166-72.
( 144 ) Krahn, M., A. Lopez de Munain, N. Streichenberger, R. Bernard, C. Pecheux, H. Testard, J.L. Pena-Segura, E. Yoldi, A. Cabello, N.B. Romero, J.J. Poza, S. Bouillot-Eimer, X. Ferrer, M. Goicoechea, F. Garcia-Bragado, F. Leturcq, J.A. Urtizberea, and N. Levy. 2006. CAPN3 mutations in patients with idiopathic eosinophilic myositis. Ann Neurol. 59:905-11.
(145) Kraytsberg, Y., E. Kudryavtseva, A.C. McKee, C. Geula, N.W. Kowall, and K. Khrapko. 2006. Mitochondrial DNA deletions are abundant and cause functional impairment in aged human substantia nigra neurons. Nat Genet. 38:518-20.
( 146 ) Kudlow, B.A., B.K. Kennedy, and R.J. Monnat, Jr. 2007. Werner and Hutchinson-Gilford progeria syndromes: mechanistic basis of human progeroid diseases. Nat Rev Mol Cell Biol. 8:394-404.
( 147 ) Kujoth, G.C., A. Hiona, T.D. Pugh, S. Someya, K. Panzer, S.E. Wohlgemuth, T. Hofer, A.Y. Seo, R. Sullivan, W.A. Jobling, J.D. Morrow, H. Van Remmen, J.M. Sedivy, T. Yamasoba, M. Tanokura, R. Weindruch, C. Leeuwenburgh, and T.A. Prolla. 2005. Mitochondrial DNA mutations, oxidative stress, and apoptosis in mammalian aging. Science. 309:481-4.
( 148 ) Kuro-o, M., Y. Matsumura, H. Aizawa, H. Kawaguchi, T. Suga, T. Utsugi, Y. Ohyama, M. Kurabayashi, T. Kaname, E. Kume, H. Iwasaki, A. lida, T. Shiraki-lida, S. Nishikawa, R. Nagai, and Y.I. Nabeshima. 1997. Mutation of the mouse klotho gene leads to a syndrome resembling ageing. Nature. 390:45-51.
( 149 ) Kurosu, H., Y. Ogawa, M. Miyoshi, M. Yamamoto, A. Nandi, K.P. Rosenblatt, M.G. Baum, S. Schiavi, M.C. Hu, O.W. Moe, and M. Kuro-o. 2006. Regulation of fibroblast growth factor-23 signaling by klotho. J Biol Chem. 281:6120-3.
( 150 ) Kurosu, H., M. Yamamoto, J.D. Clark, J.V. Pastor, A. Nandi, P. Gurnani, O.P. McGuinness, H. Chikuda, M. Yamaguchi, H. Kawaguchi, I. Shimomura, Y. Takayama, J. Herz, C.R. Kahn, K.P. Rosenblatt, and M. Kuro-o. 2005. Suppression of aging in mice by the hormone Klotho. Science. 309:1829-33.
( 151 ) Lactic Acidosis International Study Group. 2007. Risk factors for lactic acidosis and severe hyperlactataemia in HIV-1-infected adults exposed to antiretroviral therapy. Aids. 21:2455-64.
( 152 ) Lee, S., S.Y. Jeong, W.C. Lim, S. Kim, Y.Y. Park, X. Sun, R.J. Youle, and H. Cho. 2007. Mitochondrial fission and fusion mediators, hFis1 and OPA1, modulate cellular senescence. J Biol Chem. 282:22977-83.
( 153 ) Levy, J.A., M.G. Ory, and S. Crystal. 2003. HIV/AIDS interventions for midlife and older adults: current status and challenges. J Acquir Immune Defic Syndr. 33 Suppl 2:S59-67.
( 154 ) Lewis, W. 2003. Defective mitochondrial DNA replication and NRTIs: pathophysiological implications in AIDS cardiomyopathy. Am J Physiol Heart Circ Physiol. 284:H1-9.
( 155 ) Liang, S.L., H. Liu, and A. Zhou. 2006. Lovastatin-induced apoptosis in macrophages through the Rac1/Cdc42/JNK pathway. J Immunol. 177:651-6.
( 156 ) Lichtner, M., F. Mengoni, C.M. Mastroianni, I. Sauzullo, R. Rossi, M. De Nicola, V. Vullo, and L. Ghibelli. 2006. HIV protease inhibitor therapy reverses neutrophil apoptosis in AIDS patients by direct calpain inhibition. Apoptosis. 11:781-7.
( 157 ) Liu, B., J. Wang, K.M. Chan, W.M. Tjia, W. Deng, X. Guan, J.D. Huang, K.M. Li, P.Y. Chau, D.J. Chen, D. Pei, A.M. Pendas, J. Cadinanos, C. Lopez-Otin, H.F. Tse, C. Hutchison, J. Chen, Y. Cao, K.S. Cheah, K. Tryggvason, and Z. Zhou. 2005. Genomic instability in laminopathy-based premature aging. Nat Med. 11:780-5.
( 158 ) Liu, H., M.M. Fergusson, R.M. Castilho, J. Liu, L. Cao, J. Chen, D. Malide, Rovira, II, D. Schimel, C.J. Kuo, J.S. Gutkind, P.M. Hwang, and T. Finkel. 2007a. Augmented Wnt signaling in a mammalian model of accelerated aging. Science. 317:803-6.
( 159 ) Liu, Y., A. Rusinol, M. Sinensky, Y. Wang, and Y. Zou. 2006. DNA damage responses in progeroid syndromes arise from defective maturation of prelamin A. J Cell Sci. 119:4644-9.
( 160 ) Liu, Y., Y. Wang, A.E. Rusinol, M.S. Sinensky, J. Liu, S.M. Shell, and Y. Zou. 2007b. Involvement of xeroderma pigmentosum group A (XPA) in progeria arising from defective maturation of prelamin A. Faseb J.
( 161 ) Lizzi, A.R., A.M. D'Alessandro, A. Bozzi, B. Cinque, A. Oratore, and G. D'Andrea. 2007. Pattern expression of glycan residues in AZT-treated K562 cells analyzed by lectin cytochemistry. Mol Cell Biochem. 300:29-37.
( 162 ) Mallon, P.W., J. Miller, J.C. Kovacic, J. Kent-Hughes, R. Noms, K. Samaras, M.P. Feneley, D.A. Cooper, and A. Carr. 2006. Effect of pravastatin on body composition and markers of cardiovascular disease in HIV-infected men-a randomized, placebo-controlled study. Aids. 20:1003-10.
( 163 ) Manfredi, J.J. 2003. p53 and apoptosis: it's not just in the nucleus anymore. Mol Cell. 11:552-4.
( 164 ) Martin, F.M., and J.S. Friedman. 2004. Ticking fast or ticking slow, through Shc must you go? Sci Aging Knowledge Environ. 2004:pe32.
( 165 ) Martin, G.M., and L.A. Loeb. 2004. Ageing: mice and mitochondria. Nature. 429:357-9.
( 166 ) Masoro, E.J. 2004. Role of sirtuin proteins in life extension by caloric restriction. Mech Ageing Dev. 125:591-4.
( 167 ) Matarrese, P., L. Gambardella, A. Cassone, S. Vella, R. Cauda, and W. Malorni. 2003. Mitochondrial membrane hyperpolarization hijacks activated T lymphocytes toward the apoptotic-prone phenotype: homeostatic mechanisms of HIV protease inhibitors. J Immunol. 170:6006-15.
( 168 ) Matarrese, P., A. Tinari, L. Gambardella, E. Mormone, P. Narilli, M. Pierdominici, R. Cauda, and W. Malomi. 2005. HIV protease inhibitors prevent mitochondrial hyperpolarization and redox imbalance and decrease endogenous uncoupler protein-2 expression in gp 120-activated human T lymphocytes. Antivir Ther. 10 Suppl 2:M29-45.
( 169 ) Matoba, S., J.G. Kang, W.D. Patino, A. Wragg, M. Boehm, O. Gavrilova, P.J. Hurley, F. Bunz, and P.M. Hwang. 2006. p53 regulates mitochondrial respiration. Science. 312:1650-3.
( 170 ) Maurer, T.A. 2005. Dermatologie manifestations of HIV infection. Top HIV Med. 13:149-54.
( 171 ) Mehta, I.S., M. Figgitt, C.S. Clements, I.R. Kill, and J.M. Bridger. 2007. Alterations to nuclear architecture and genome behavior in senescent cells. Ann N Y Acad Sci. 1100:250-63.
( 172 ) Meissner, C. 2007. Mutations of mitochondrial DNA - cause or consequence of the ageing process? Z Gerontol Geriatr. 40:325-333.
( 173 ) Migliaccio, E., M. Giorgio, S. Mele, G. Pelicci, P. Reboldi, P.P. Pandolfi, L. Lanfrancone, and P.G. Pelicci. 1999. The p66shc adaptor protein controls oxidative stress response and life span in mammals. Nature. 402:309-13.
(174) Migliaccio, E., M. Giorgio, and P.G. Pelicci. 2006. Apoptosis and aging: role of p66Shc redox protein. Antioxid Redox Signal. 8:600-8.
( 175 ) Mihara, M., S. Erster, A. Zaika, O. Petrenko, T. Chittenden, P. Pancoska, and U.M. Moll. 2003. p53 has a direct apoptogenic role at the mitochondria. Mol Cell. 11:577-90.
( 176 ) Miserez, A.R., P.Y. Muller, and V. Spaniol. 2002. Indinavir inhibits sterol-regulatory element-binding protein-1c-dependent lipoprotein lipase and fatty acid synthase gene activations. Aids. 16:1587-94.
( 177 ) Moll, U.M., S. Wolff, D. Speidel, and W. Deppert. 2005. Transcription-independent pro-apoptotic functions of p53. Curr Opin Cell Biol. 17:631-6.
( 178 ) Mondy, K., W.G. Powderly, S.A. Claxton, K.H. Yarasheski, M. Royal, J.S. Stoneman, M.E. Hoffmann, and P. Tebas. 2005. Alendronate, vitamin D, and calcium for the treatment of osteopenia/osteoporosis associated with HIV infection. J Acquir Immune Defic Syndr. 38:426-31.
( 179 ) Mondy, K., and P. Tebas. 2007. Cardiovascular risks of antiretroviral therapies. Annu Rev Med. 58:141-55.
( 180 ) Moyle, G. 2007. Metabolic issues associated with protease inhibitors. J Acquir Immune Defic Syndr. 45 Suppl 1:S19-26.
( 181 ) Mujawar, Z., H. Rose, M.P. Morrow, T. Pushkarsky, L. Dubrovsky, N. Mukhamedova, Y. Fu, A. Dart, J.M. Orenstein, Y.V. Bobryshev, M. Bukrinsky, and D. Sviridov. 2006. Human immunodeficiency virus impairs reverse cholesterol transport from macrophages. PLoS Biol. 4:e365.
( 182 ) Mukhopadhyay, A., B. Wei, S.J. Zullo, L.V. Wood, and H. Weiner. 2002. In vitro evidence of inhibition of mitochondrial protease processing by HIV-1 protease inhibitors in yeast: a possible contribution to lipodystrophy syndrome. Mitochondrion. 1:511-8.
( 183 ) Muntoni, F., M. Brockington, S. Torelli, and S.C. Brown. 2004. Defective glycosylation in congenital muscular dystrophies. Curr Opin Neurol. 17:205-9.
( 184 ) Mylonis, I., V. Drosou, S. Brancorsini, E. Nikolakaki, P. Sassone-Corsi, and T. Giannakouros. 2004. Temporal association of protamine 1 with the inner nuclear membrane protein lamin B receptor during spermiogenesis. J Biol Chem. 279:11626-31.
( 185 ) Naujokat, C., D. Fuchs, and C. Berges. 2007. Adaptive modification and flexibility of the proteasome system in response to proteasome inhibition. Biochim Biophys Acta. 1773:1389-97.
( 186 ) Navarro, C.L., J. Cadinanos, A. De Sandre-Giovannoli, R. Bernard, S. Courrier, I. Boccaccio, A. Boyer, W.J. Kleijer, A. Wagner, F. Giuliano, F.A. Beemer, J.M. Freije, P. Cau, R.C. Hennekam, C. Lopez-Otin, C. Badens, and N. Levy. 2005. Loss of ZMPSTE24 (FACE-1) causes autosomal recessive restrictive dermopathy and accumulation of Lamin A precursors. Hum Mol Genet. 14:1503-13.
( 187 ) Navarro, C.L., P. Cau, and N. Levy. 2006. Molecular bases of progeroid syndromes. Hum Mol Genet. 15 Suppl 2:R151-61.
( 188 ) Navarro, C.L., A. De Sandre-Giovannoli, R. Bernard, I. Boccaccio, A. Boyer, D. Genevieve, S. Hadj-Rabia, C. Gaudy-Marqueste, H.S. Smitt, P. Vabres, L. Faivre, A. Verloes, T. Van Essen, E. Flori, R. Hennekam, F.A. Beemer, N. Laurent, M. Le Merrer, P. Cau, and N. Levy. 2004. Lamin A and ZMPSTE24 (FACE-1) defects cause nuclear disorganization and identify restrictive dermopathy as a lethal neonatal laminopathy. Hum Mol Genet. 13:2493-503.
( 189 ) Negredo, E., J. Molto, J. Puig, D. Cinquegrana, A. Bonjoch, N. Perez-Alvarez, R. Lopez-Blazquez, A. Blanco, B. Clotet, and C. Rey-Joly. 2006. Ezetimibe, a promising lipid-lowering agent for the treatment of dyslipidaemia in HIV-infected patients with poor response to statins. Aids. 20:2159-64.
( 190 ) Nemoto, S., C.A. Combs, S. French, B.H. Ahn, M.M. Fergusson, R.S. Balaban, and T. Finkel. 2006. The mammalian longevity-associated gene product p66shc regulates mitochondrial metabolism. J Biol Chem. 281:10555-60.
( 191 ) Neye, Y., M. Dufer, G. Drews, and P. Krippeit-Drews. 2006. HIV protease inhibitors: suppression of insulin secretion by inhibition of voltage-dependent K+ currents and anion currents. J Pharmacol Exp Ther. 316:106-12.
( 192 ) Nguyen, A.T., A. Gagnon, J.B. Angel, and A. Sorisky. 2000. Ritonavir increases the level of active ADD-1/SREBP-1 protein during adipogenesis. Aids. 14:2467-73.
( 193 ) Nisoli, E., and M.O. Carruba. 2006. Nitric oxide and mitochondrial biogenesis. J Cell Sci. 119:2855-62.
( 194 ) Nisoli, E., C. Tonello, A. Cardile, V. Cozzi, R. Bracale, L. Tedesco, S. Falcone, A. Valerio, O. Cantoni, E. Clementi, S. Moncada, and M.O. Carruba. 2005. Calorie restriction promotes mitochondrial biogenesis by inducing the expression of eNOS. Science. 310:314-7.
( 195 ) Njajou, O.T., R.M. Cawthon, C.M. Damcott, S.H. Wu, S. Ott, M.J. Garant, E.H. Blackburn, B.D. Mitchell, A.R. Shuldiner, and W.C. Hsueh. 2007. Telomere length is paternally inherited and is associated with parental lifespan. Proc Natl Acad Sci U S A. 104:12135-9.
( 196 ) North, B.J., and E. Verdin. 2004. Sirtuins: Sir2-related NAD-dependent protein deacetylases. Genome Biol. 5:224.
( 197 ) Novelli, G., A. Muchir, F. Sangiuolo, A. Helbling-Leclerc, M.R. D'Apice, C. Massart, F. Capon, P. Sbraccia, M. Federici, R. Lauro, C. Tudisco, R. Pallotta, G. Scarano, B. Dallapiccola, L. Merlini, and G. Bonne. 2002. Mandibuloacral dysplasia is caused by a mutation in LMNA-encoding lamin A/C. Am J Hum Genet. 71:426-31.
( 198 ) Oberdoerffer, P., and D.A. Sinclair. 2007. The role of nuclear architecture in genomic instability and ageing. Nat Rev Mol Cell Biol. 8:692-702.
( 199 ) Olivero, O.A. 2007. Mechanisms of genotoxicity of nucleoside reverse transcriptase inhibitors. Environ Mol Mutagen. 48:215-23.
(200) Olivero, O.A., G.M. Shearer, C.A. Chougnet, A.A. Kovacs, A.L. Landay, R. Baker, A.M. Stek, M.M. Khoury, L.A. Proia, H.A. Kessler, B.E. Sha, R.E. Tarone, and M.C. Poirier. 1999. Incorporation of zidovudine into leukocyte DNA from HIV-1-positive adults and pregnant women, and cord blood from infants exposed in utero. Aids. 13:919-25.
( 201 ) Olivero, O.A., A.M. Tejera, J.J. Fernandez, B.J. Taylor, S. Das, R.L. Divi, and M.C. Poirier. 2005. Zidovudine induces S-phase arrest and cell cycle gene expression changes in human cells. Mutagenesis. 20:139-46.
**(202)** Orsini, F., E. Migliaccio, M. Moroni, C. Contursi, V.A. Raker, D. Piccini, I. Martin-Padura, G. Pelliccia, M. Trinei, M. Bono, C. Puri, C. Tacchetti, M. Ferrini, R. Mannucci, I. Nicoletti, L. Lanfrancone, M. Giorgio, and P.G. Pelicci. 2004. The life span déterminant p66Shc localizes to mitochondria where it associates with mitochondrial heat shock protein 70 and regulates trans-membrane potential. J Biol Chem. 279:25689-95.
**( 203 )** Pacenti, M., L. Barzon, F. Favaretto, K. Fincati, S. Romano, G. Milan, R. Vettor, and G. Palu. 2006. Microarray analysis during adipogenesis identifies new genes altered by antiretroviral drugs. Aids. 20:1691-705.
( **204** ) Palmieri, F. 2004. The mitochondrial transporter family (SLC25): physiological and pathological implications. Pflugers Arch. 447:689-709.
**( 205 )** Ran, Y., A. Garg, and A.K. Agarwal. 2007. Mislocalization of prelamin A Tyr646Phe mutant to the nuclear pore complex in human embryonic kidney 293 cells. Biochem Biophys Res Commun. 355:78-84.
(206) Pandita, T.K., C.R. Hunt, G.G. Sharma, and Q. Yang. 2007. Regulation of telomere movement by telomere chromatin structure. Cell Mol Life Sci. 64:131-8.
**( 207 )** Papazoglu, C., and A.A. Mills. 2007. p53: at the crossroad between cancer and ageing. J Pathol. 211:124-33.
**( 208 )** Parker, R.A., O.P. Flint, R. Mulvey, C. Elosua, F. Wang, W. Fenderson, S. Wang, W.P. Yang, and M.A. Noor. 2005. Endoplasmic reticulum stress links dyslipidemia to inhibition of proteasome activity and glucose transport by HIV protease inhibitors. Mol Pharmacol. 67:1909-19.
**( 209 )** Pennings, M., I. Meurs, D. Ye, R. Out, M. Hoekstra, T.J. Van Berkel, and M. Van Eck. 2006. Regulation of cholesterol homeostasis in macrophages and consequences for atherosclerotic lesion development. FEBS Lett 580:5588-96.
**( 210 )** Percival, J.M., and S.C. Froehner. 2007. Golgi complex organization in skeletal muscle: a role for Golgi-mediated glycosylation in muscular dystrophies? Traffic. 8:184-94.
**( 211 )** Petersen-Mahrt, S.K., C. Estmer, C. Ohrmalm, D.A. Matthews, W.C. Russell, and G. Akusjarvi. 1999. The splicing factor-associated protein, p32, regulates RNA splicing by inhibiting ASF/SF2 RNA binding and phosphorylation. Embo J. 18:1014-24.
(212) Piccinini, M., M.T. Rinaudo, A. Anselmino, B. Buccinna, C. Ramondetti, A. Dematteis, E. Ricotti, L Palmisano, M. Mostert, and P.A. Tovo. 2005. The HIV protease inhibitors nelfinavir and saquinavir, but not a variety of HIV reverse transcriptase inhibitors, adversely affect human proteasome function. Antivir Ther. 10:215-23.
**( 213 )** Pilon, A.A., J.J. Lum, J. Sanchez-Dardon, B.N. Phenix, R. Douglas, and A.D. Badley. 2002. Induction of apoptosis by a nonnucleoside human immunodeficiency virus type 1 reverse transcriptase inhibitor. Antimicrob Agents Chemother. 46:2687-91.
**(214)** Porcu, M., and A. Chiarugi. 2005. The emerging therapeutic potential of sirtuin-interacting drugs: from cell death to lifespan extension. Trends Pharmacol Sci. 26:94-103.
**( 215 )** Quarrie, J.K., and K.T. Riabowol. 2004. Murine models of life span extension. Sci Aging Knowledge Environ. 2004:re5.
**( 216 )** Razzaque, M.S., and B. Lanske. 2006. Hypervitaminosis D and premature aging: lessons learned from Fgf23 and Klotho mutant mice. Trends Mol Med. 12:298-305.
**( 217 )** Restrepo, C.S., L. Diethelm, J.A. Lemos, E. Velasquez, T.A. Ovella, S. Martinez, J. Carrillo, and D.F. Lemos. 2006. Cardiovascular complications of human immunodeficiency virus infection. Radiographics. 26:213-31.
**( 218 )** Restrepo, C.S., D.F. Lemos, H. Gordillo, R. Odero, T. Varghese, W. Tiernann, F.F. Rivas, R. Moncada, and C.R. Gimenez. 2004. Imaging findings in musculoskeletal complications of AIDS. Radiographics. 24:1029-49.
**( 219 )** Richard, I., O. Broux, V. Allamand, F. Fougerousse, N. Chiannilkulchai, N. Bourg, L Brenguier, C. Devaud, P. Pasturaud, C. Roudaut, and et al. 1995. Mutations in the proteolytic enzyme calpain 3 cause limb-girdle muscular dystrophy type 2A. Cell. 81:27-40.
(220) Richter, C., J.W. Park, and B.N. Ames. 1988. Normal oxidative damage to mitochondrial and nuclear DNA is extensive. Proc Natl Acad Sci U S A. 85:6465-7.
**( 221 )** Riddle, T.M., D.G. Kuhel, L.A. Woollett, C.J. Fichtenbaum, and D.Y. Hui. 2001. HIV protease inhibitor induces fatty acid and sterol biosynthesis in liver and adipose tissues due to the accumulation of activated sterol regulatory element-binding proteins in the nucleus. J Biol Chem. 276:37514-9.
**( 222 )** Roche, R., I. Poizot-Martin, C.M. Yazidi, E. Compe, J.A. Gastaut, J. Torresani, and R. Planells. 2002. Effects of antiretroviral drug combinations on the differentiation of adipocytes. Aids. 16:13-20.
**( 223 )** Roehl White, S., and B. Lauring. 2007. AAA+ ATPases: Achieving Diversity of Function with Conserved Machinery. Traffic*.*
**( 224 )** Rota, M., N. LeCapitaine, T. Hosoda, A. Boni, A. De Angelis, M.E. Padin-Iruegas, G. Esposito, S. Vitale, K. Urbanek, C. Casarsa, M. Giorgio, T.F. Luscher, P.G. Pelicci, P. Anversa, A. Leri, and J. Kajstura. 2006. Diabetes promotes cardiac stem cell aging and heart failure, which are prevented by deletion of the p66shc gene. Circ Res. 99:42-52.
**( 225 )** Rudich, A., R. Ben-Romano, S. Etzion, and N. Bashan. 2005. Cellular mechanisms of insulin résistance, lipodystrophy and atherosclerosis induced by HIV protease inhibitors. Acta Physiol Scand. 183:75-88.
**( 226 )** Russell, S.J., and C.R. Kahn. 2007. Endocrine regulation of ageing. Nat Rev Mol Cell Biol. 8:681-91.
**( 227 )** Sablina, A.A., A.V. Budanov, G.V. Ilyinskaya, L.S. Agapova, J.E. Kravchenko, and P.M. Chumakov. 2005. The antioxidant function of the p53 tumor suppressor. Nat Med. 11:1306-13.
**( 228 )** Saint-Marc, T., M. Partisani, I. Poizot-Martin, F. Bruno, O. Rouviere, J.M. Lang, J.A. Gastaut, and J.L. Touraine. 1999. A syndrome of peripheral fat wasting (lipodystrophy) in patients receiving long-term nucleoside analogue therapy. Aids. 13:1659-67.
**( 229 )** Saint-Marc, T., M. Partisani, I. Poizot-Martin, O. Rouviers, F. Bruno, R. Avellaneda, J.M. Lang, J.A. Gastaut, and J.L. Touraine. 2000. Fat distribution evaluated by computed tomography and metabolic abnormalities in patients undergoing antiretroviral therapy: preliminary results of the LIPOCO study. Aids. 14:37-49.
**( 230 )** Scaffidi, P., and T. Misteli. 2006. Lamin A-dependent nuclear defects in human aging. Science. 312:1059-63.
**( 231 )** Schmid, G., M.P. Kramer, M. Maurer, S. Wandl, and J. Wesierska-Gadek. 2007. Cellular and organismal ageing: Role of the p53 tumor suppressor protein in the induction of transient and terminal senescence. J Cell Biochem. 101:1355-69.
**( 232 )** Schriner, S.E., N.J. Linford, G.M. Martin, P. Treuting, C.E. Ogburn, M. Emond, P.E. Coskun, W. Ladiges, N. Wolf, H. Van Remmen, D.C. Wallace, and P.S. Rabinovitch. 2005. Extension of murine life span by overexpression of catalase targeted to mitochondria. Science. 308:1909-11.
**( 233 )** Schutt, M., J. Zhou, M. Meier, and H.H. Klein. 2004. Long-term effects of HIV-1 protease inhibitors on insulin secretion and insulin signaling in INS-1 beta cells. J Endocrino/. 183:445-54.
(234) Seidah, N.G., A.M. Khatib, and A. Prat. 2006. The proprotein convertases and their implication in sterol and/or lipid metabolism. Biol Chem. 387:871-7.
(235) Sengupta, S., and C.C. Harris. 2005. p53: traffic cop at the crossroads of DNA repair and recombination. Nat Rev Mol Cell Biol. 6:44-55.
**( 236 )** Shaklai, S., N. Amariglio, G. Rechavi, and A.J. Simon. 2007. Gene silencing at the nuclear periphery. Febs J. 274:1383-92.
**( 237 )** Sharma, A., S. Awasthi, C.K Harrod, E.F. Matlock, S. Khan, L. Xu, S. Chan, H. Yang, C.K. Thammavaram, R.A. Rasor, D.K. Burns, D.J. Skiest, C. Van Lint, A.M. Girard, M. McGee, R.J. Monnat, Jr., and R. Harrod. 2007. The Werner syndrome helicase is a cofactor for HIV-1 long terminal repeat transactivation and retroviral replication. J Biol Chem. 282:12048-57.
**( 238 )** Sharpless, N.E., and R.A. DePinho. 2002. p53: good cop/bad cop. Cell. 110:9-12.
**( 239 )** Short, K.R., M.L. Bigelow, J. Kahl, R. Singh, J. Coenen-Schimke, S. Raghavakaimal, and K.S. Nair. 2005. Decline in skeletal muscle mitochondrial function with aging in humans. Proc Natl Acad Sci U S A. 102:5618-23.
(240) Simos, G., and S.D. Georgatos. 1994. The lamin B receptor-associated protein p34 shares sequence homology and antigenic determinants with the splicing factor 2-associated protein p32. FEBS Lett. 346:225-8.
**( 241 )** Sirkis, R., J.E. Gerst, and D. Fass. 2006. Ddi1, a eukaryotic protein with the retroviral protease fold. J Mol Biol. 364:376-87.
**( 242 )** Sommers, J.A., S. Sharma, K.M. Doherty, P. Karmakar, Q. Yang, M.K. Kenny, C.C. Harris, and R.M. Brosh, Jr. 2005. p53 modulates RPA-dependent and RPA-independent WRN helicase activity. Cancer Res. 65:1223-33.
**( 243 )** Stewart, S.A., and R.A. Weinberg. 2006. Telomeres: cancer to human aging. Annu Rev Cell Dev Biol. 22:531-57.
**( 244 )** Storz, P. 2006. Reactive oxygen species-mediated mitochondria-to-nucleus signaling: a key to aging and radical-caused diseases. Sci STKE. 2006:re3.
**( 245 )** Suzuki, Y., and R. Craigie. 2007. The road to chromatin - nuclear entry of retroviruses. Nat Rev Microbiol. 5:187-96.
**( 246 )** Tehranzadeh, J., R.R. Ter-Oganesyan, and L.S. Steinbach. 2004a. Musculoskeletal disorders associated with HIV infection and AIDS. Part I: infectious musculoskeletal conditions. Skeletal Radiol. 33:249-59.
**( 247 )** Tehranzadeh, J., R.R. Ter-Oganesyan, and L.S. Steinbach. 2004b. Musculoskeletal disorders associated with HIV infection and AIDS. Part II: non-infectious musculoskeletal conditions. Skeletal Radiol. 33:311-20.
**( 248 )** Thomas, C.M., and E.J. Smart. 2007. How HIV protease inhibitors promote atherosclerotic lesion formation. Curr Opin Lipidol. 18:561-5.
**( 249 )** Thomas, J., and S.M. Doherty. 2003. HIV infection--a risk factor for osteoporosis. J Acquir Immune Defic Syndr. 33:281-91.
(250) Torres, H.A., B.J. Bamett, and R.C. Arduino. 2007. Alopecia associated with ritonavir-boosted atazanavir therapy. Aids. 21:1391-2.
**( 251 )** Trifunovic, A., A. Hansson, A. Wredenberg, A.T. Rovio, E. Dufour, I. Khvorostov, J.N. Spelbrink, R. Wibom, H.T. Jacobs, and N.G. Larsson. 2005. Somatic mtDNA mutations cause aging phenotypes without affecting reactive oxygen species production. Proc Natl Acad Sci U S A. 102:17993-8.
**( 252 )** Trifunovic, A., A. Wredenberg, M. Falkenberg, J.N. Spelbrink, A.T. Rovio, C.E. Bruder, Y.M. Bohlooly, S. Gidlof, A. Oldfors, R. Wibom, J. Tornell, H.T. Jacobs, and N.G. Larsson. 2004. Premature ageing in mice expressing defective mitochondrial DNA polymerase. Nature. 429:417-23.
**( 253 )** Trinei, M., M. Giorgio, A. Cicalese, S. Barozzi, A. Ventura, E. Migliaccio, E. Milia, I.M. Padura, V.A. Raker, M. Maccarana, V. Petronilli, S. Minucci, P. Bernardi, L. Lanfrancone, and P.G. Pelicci. 2002. A p53-p66Shc signalling pathway controls intracellular redox status, levels of oxidation-damaged DNA and oxidative stress-induced apoptosis. Oncogene. 21:3872-8.
**( 254 )** Truscott, K.N., K. Brandner, and N. Pfanner. 2003. Mechanisms of protein import into mitochondria. Curr Biol. 13:R326-37.
**( 255 )** Tyner, S.D., S. Venkatachalam, J. Choi, S. Jones, N. Ghebranious, H. Igelmann, X. Lu, G. Soron, B. Cooper, C. Brayton, S. Hee Park, T. Thompson, G. Karsenty, A. Bradley, and L.A. Donehower. 2002. p53 mutant mice that display early ageing-associated phenotypes. Nature. 415:45-53.
**( 256 )** Unger, R.H. 2006. Klotho-induced insulin resistance: a blessing in disguise? Nat Med. 12:56-7.
**( 257 )** van Wijk, J.P., E.J. de Koning, M.C. Cabezas, J. Joven, J. op't Roodt, T.J. Rabelink, and A.M. Hoepelman. 2006. Functional and structural markers of atherosclerosis in human immunodeficiency virus-infected patients. J Am Coll Cardiol. 47:1117-23.
**( 258 )** Varela, I., J. Cadinanos, A.M. Pendas, A. Gutierrez-Femandez, A.R. Folgueras, L.M. Sanchez, Z. Zhou, F.J. Rodriguez, C.L Stewart, J.A. Vega, K. Tryggvason, J.M. Freije, and C. Lopez-Otin. 2005. Accelerated ageing in mice deficient in Zmpste24 protease is linked to p53 signalling activation. Nature. 437:564-8.
**( 259 )** Vidal, F., J.C. Domingo, J. Guallar, M. Saumoy, B. Cordobilla, R. Sanchez de la Rosa, M. Giralt, M.L. Alvarez, M. Lopez-Dupla, F. Torres, F. Villarroya, T. Cihlar, and P. Domingo. 2006. In vitro cytotoxicity and mitochondrial toxicity of tenofovir alone and in combination with other antiretrovirals in human renal proximal tubule cells. Antimicrob Agents Chemother. 50:3824-32.
**( 260 )** Vijg, J., and Y. Suh. 2006. Ageing: chromatin unbound. Nature. 440:874-5.
**( 261 )** Vicek, S., T. Dechat, and R. Foisner. 2001. Nuclear envelope and nuclear matrix: interactions and dynamics. Cell Mol Life Sci. 58:1758-65.
**( 262 )** Vlcek, S., and R. Foisner. 2006. A-type lamin networks in light of laminopathic diseases. Biochim Biophys Acta*.*
**( 263 )** Vlcek, S., and R. Foisner. 2007a. Lamins and lamin-associated proteins in aging and disease. Curr Opin Cell Biol. 19:298-304.
**( 264 )** Vlcek, S., and R. Foisner. 2007b. A-type lamin networks in light of laminopathic diseases. Biochim Biophys Acta. 1773:661-74.
**( 265 )** Wallace, D.C. 2005. A mitochondrial paradigm of metabolic and degenerative diseases, aging, and cancer: a dawn for evolutionary medicine. Annu Rev Genet. 39:359-407.
**( 266 )** Wallis, C.V., A.N. Sheerin, M.H. Green, C.J. Jones, D. Kipling, and R.G. Faragher. 2004. Fibroblast clones from patients with Hutchinson-Gilford progeria can senesce despite the presence of telomerase. Exp Gerontol. 39:461-7.
**( 267 )** Wang, X., H. Mu, H. Chai, D. Liao, Q. Yao, and C. Chen. 2007. Human immunodeficiency virus protease inhibitor ritonavir inhibits cholesterol efflux from human macrophage-derived foam cells. Am J Pathol. 171:304-14.
**( 268 )** Wang, X., and D.J: Rader. 2007. Molecular regulation of macrophage reverse cholesterol transport. Curr Opin Cardiol. 22:368-72.
**( 269 )** Williams, K., Y.P. Rao, R. Natarajan, W.M. Pandak, and P.B. Hylemon. 2004. Indinavir alters sterol and fatty acid homeostatic mechanisms in primary rat hepatocytes by increasing levels of activated sterol regulatory element-binding proteins and decreasing cholesterol 7alpha-hydroxylase mRNA levels. Biochem Pharmacol. 67:255-67.
**( 270 )** Wiwanitkit, V. 2004. Prevalence of dermatological disorders in Thai HIV-infected patients correlated with different CD4 lymphocyte count statuses: a note on 120 cases. Int J Dermatol. 43:265-8.
**( 271 )** Worman, H.J., and G. Bonne. 2007. "Laminopathies": a wide spectrum of human diseases. Exp Cell Res. 313:2121-33.
**( 272 )** Wright, L.P., and M.R. Philips. 2006. Thematic review series: lipid posttranslational modifications. CAAX modification and membrane targeting of Ras. J Lipid Res. 47:883-91.
**( 273 )** Wynford-Thomas, D. 1996. Telomeres, p53 and cellular senescence. Oncol Res. 8:387-98.
**( 274 )** Yamagishi, S., T. Matsui, T. Sato, and M. Takeuchi. 2006. Protective rôle of pravastatin in the pathogenesis of the metabolic syndrome. Med Hypotheses. 66:609-11.
**( 275 )** Yamaguchi, T., Y. Takayama, M. Saito. F. Ishikawa, and M. Saneyoshi. 2001. Telomerase-inhibitory effects of the triphosphate derivatives of some biologically active nucleosides. Nucleic Acids Res Suppl:211-2*.*
**( 276 )** Yamanaka, H., H. Gatanaga, P. Kosalaraksa, S. Matsuoka-Aizawa, T. Takahashi, S. Kimura, and S. Oka. 2007. Novel mutation of human DNA polymerase gamma associated with mitochondrial toxicity induced by anti-HIV treatment. J Infect Dis. 195:1419-25.
**( 277 )** Yeh, R.F., V.E. Gaver, K.B. Patterson, N.L. Rezk, F. Baxter-Meheux, M.J. Blake, J.J. Eron, Jr., C.E. Klein, J.C. Rublein, and A.D. Kashuba. 2006. Lopinavir/ritonavir induces the hepatic activity of cytochrome P450 enzymes CYP2C9, CYP2C19, and CYP1A2 but inhibits the hepatic and intestinal activity of CYP3A as measured by a phenotyping drug cocktail in healthy volunteers. J Acquir Immune Defic Syndr. 42:52-60.
**( 278 )** Yousefi, S., R. Perozzo, I. Schmid, A. Ziemiecki, T. Schaffner, L. Scapozza, T. Brunner, and H.U. Simon. 2006. Calpain-mediated cleavage of Atg5 switches autophagy to apoptosis. Nat Cell Biol. 8:1124-32.
**( 279 )** Zafon, C. 2007. Jekyll and Hyde, the p53 protein, pleiotropics antagonisms and the thrifty aged hypothesis of senescence. Med Hypotheses. 68:1371-7.
**( 280 )** Zhang, D., T.J. Chando, D.W. Everett, C.J. Patten, S.S. Dehal, and W.G. Humphreys. 2005. In vitro inhibition of UDP glucuronosyltransferases by atazanavir and other HiV protase inhibitors and the relationship of this property to in vivo bilirubin glucuronidation. Drug Metab Dispos. 33:1729-39.
**( 281 )** Zhong, D.S., X.H. Lu, B.S. Conklin, P.H. Lin, A.B. Lumsden, Q. Yao, and C. Chen. 2002. HIV protease inhibitor ritonavir induces cytotoxicity of human endothelial cells. Arterioscler Thromb Vasc Biol. 22:1560-6.
**( 282 )** Zhou, H., E.C. Gurley, S. Jarujaron, H. Ding, Y. Fang, Z. Xu, W.M. Pandak, Jr., and P.B. Hylemon. 2006. HIV protease inhibitors activate the unfolded protein response and disrupt lipid metabolism in primary hepatocytes. Am J Physiol Gastrointest Liver Physiol. 291:G1071-80.
**( 283 )** Zhou, H., W.M. Pandak, Jr., V. Lyall, R. Natarajan, and P.B. Hylemon. 2005. HIV protease inhibitors activate the unfolded protein response in macrophages: implication for atherosclerosis and cardiovascular disease. Mol Pharmacol. 68:690-700.
**( 284 )** Blobel et Potter, V.R. Nuclei from rat liver : isolation method that combines purity with high yield, Science 154, 1662-1665, 1966
**( 285 )** Sullivan, T. Escalante-Alcalde, D. Bhatt, H. Anver, M. Bhat, N. Nagashima, K. Stewart, C. L. Burke, B. Loss of A-type lamin Expression compromises nuclear envelope integrity leading to muscular dystrophy. J. Cell Biol. 147: 913-920, 1999

## Revendications

1. Composition comprenant :
- au moins un inhibiteur de l'hydroxyméthylglutaryl-coenzyme A (HMG-CoA) réductase ou l'un de ses sels physiologiquement acceptable, ledit inhibiteur étant une molécule de la famille des statines ou l'un de ses sels physiologiquement acceptables
- au moins un inhibiteur de la famésyl-pyrophosphate synthase ou de l'un de ses sels physiologiquement acceptable, ledit inhibiteur étant une molécule de la famille des aminobiphosphonates (NBP) ou l'un de ses sels physiologiquement acceptables, et
- au moins un agent anti-VIH, ledit agent anti-VIH étant .un inhibiteur de protéase ou un inhibiteur de la transcriptase inverse.

2. Composition selon la revendication 1, dans laquelle l'inhibiteur de HMG-CoA réductase est une statine hydrosoluble.

3. Composition selon la revendication 1 dans laquelle l'inhibiteur de HMG-CoA réductase est une statine liposoluble.

4. Composition la revendication 1, dans laquelle l'inhibiteur de la HMG-CoA réductase est choisi dans le groupe comprenant l'atorvastatine, la simvastatine, la pravastatine, la rivastatine, la mévastatine (ou compactine), la fluindostatine, la vélostatine, la fluvastatine, la dalvastatine, la cérivastatine, la pentostatine, la rosuvastatine, la lovastatine, la pitavastatine et leurs sels physiologiquement acceptables.

5. Composition selon la revendication selon l'une quelconque des revendications 1 à 4, dans laquelle que l'aminobiphosphonate peut être choisi parmi:
- l'acide alendronlque ou sa forme ionique, l'alendronate ;
- l'acide clodronique ou sa forme ionique, le clodronate ;
- l'acide étidronique ou sa forme ionique, l'étidronate ;
- l'acide-ibandronique, ou sa forme ionique, l'ibandronate ;
- l'acide médronique ou sa forme ionique, le médronate ;
- l'acide néridronique ou sa forme ionique, le néridronate ;
- l'acide olpadronique ou sa forme ionique, l'olpadronate ;
- l'acide pamidronique ou sa forme ionique, le pamidronate ;
- l'acide risédronique ou sa forme ionique, le risédronate;
- l'acide tiludronique ou sa forme ionique, le tiludronate ;
- l'acide zolédronique (ou zolendronique) ou sa forme ionique le zolédronate (ou zolendronate) ;
- l'acide 4-N,N-diméthylaminométhane diphosphonlque ou sa forme ionique le diméthylaminométhanediphosphonate ;
- l'α-amino-(4-hydroxybenzylidène) diphosphonate.

6. Composition selon l'une quelconque des revendications 1 à 5, dans laquelle l'inhibiteur de la farnésyl-pyrophosphate synthase est l'acide zolédronique ou sa forme ionique, le zolédronate.

7. Composition selon l'une quelconque des revendications 1 à 6, dans laquelle l'agent anti-VIH est un inhibiteur de protéase choisi dans le groupe comprenant fosamprenavir, lopinavir, ritonavir, amprenavir, atazanavir et indinavir.

8. Composition selon l'une quelconque des revendication 1 à 6, dans laquelle l'agent anti-VIH est un inhibiteur de la transcriptase inverse choisi dans le groupe comprenant zidovudine, lamivudine, didanosine et epzicom.

9. Composition selon l'une quelconque des revendications 1 à 8 pour son application comme médicament dans le traitement des effets secondaires de vieillissement précoce généré(s) chez un patient par un traitement anti-VIH.

10. Composition pour son application selon la revendication 9, dans laquelle la composition est une composition pour administration par voie orale ou par injection.

11. Composition pour son application selon l'une quelconque des revendications 9 à 10 dans laquelle le traitement est celui des effets secondaires de vieillissement précoce généré(s) chez un patient par un traitement anti-VIH utilisant un inhibiteur de protéase ou un inhibiteur de la transcriptase inverse.

12. Composition pour son application comme médicament dans le traitement du VIH comprenant dans un ordre quelconque les étapes suivantes :
i. administration d'un mélange comprenant au moins un inhibiteur de l'hydroxyméthylglutaryl-coenzyme A (HMG-CoA) réductase, ledit inhibiteur étant une molécule de la famille des statines ou l'un de ses sels physiologiquement acceptables, et au moins un inhibiteur de la farnésyl-pyrophosphate synthase, ledit inhibiteur étant une molécule de la famille des aminobiphosphonates (NBP) ou l'un de ses sels physiologiquement acceptables
ii. administration d'un agent anti-VIH, ledit agent anti-VIH étant .un inhibiteur de protéase ou un inhibiteur de la transcriptase inverse.
dans laquelle, les administrations sont concomitantes, successives ou alternatives.

## Patentansprüche

1. Zusammensetzung, umfassend:
- mindestens einen Hemmer der Hydroxymethylglutarylcoenzym A (HMG-CoA)-Reduktase oder eines ihrer physiologisch annehmbaren Salze, wobei der Hemmer ein Molekül der Familie der Statine oder eines ihrer physiologisch annehmbaren Salze ist,
- mindestens einen Hemmer der Farnesylpyrophosphatsynthase oder eines ihrer physiologisch annehmbaren Salze, wobei der Hemmer ein Molekül der Familie der Aminobiophosphonate (NBP) oder eines ihrer physiologisch annehmbaren Salze ist, und
- mindestens ein Anti-HIV-Mittel, wobei das Anti-HIV-Mittel ein Proteasehemmer oder ein Hemmer der inversen Transkriptase ist.

2. Zusammensetzung nach Anspruch 1, wobei der Hemmer der HMG-CoA-Reduktase ein wasserlösliches Statin ist.

3. Zusammensetzung nach Anspruch 1, wobei der Hemmer der HMG-CoA-Reduktase ein fettlösliches Statin ist.

4. Zusammensetzung nach Anspruch 1, wobei der Hemmer der HMG-CoA-Reduktase ausgewählt ist aus der Gruppe, bestehend aus Atorvastatin, Simvastatin, Pravastatin, Rivastatin, Mevastatin (oder Compactin), Fluindostatin, Velostatin, Fluvastatin, Dalvastatin, Cerivastatin, Pentostatin, Rosuvastatin, Lovastatin, Pitavastatin oder ihre physiologisch annehmbaren Salze.

5. Zusammensetzung nach einem der Ansprüche 1 bis 4, wobei nur das Aminobiphosphonat ausgewählt werden kann aus:
- Alendronsäure oder ihrer ionischen Form, Alendronat;
- Clodronsäure oder ihrer ionischen Form, Clodronat;
- Etidronsäure oder ihrer ionischen Form, Etidronat;
- Ibandronsäure oder ihrer ionischen Form, Ibandronat;
- Medronsäure oder ihrer ionischen Form, Medronat;
- Neridronsäure oder ihrer ionischen Form, Neridronat;
- Olpadronsäure oder ihrer ionischen Form, Olpadronat;
- Pamidronsäure oder ihrer ionischen Form, Pamidronat;
- Risedronsäure oder ihrer ionischen Form, Risedronat;
- Tiludronsäure oder ihrer ionischen Form, Tiludronat;
- Zoledronsäure (oder Zolendronsäure) oder ihrer ionischen Form, Zoledronat (oder Zolendronat);
- 4-N,N-Dimethylaminmethandiphosphonsäure oder ihrer ionischen Form, Dimethylaminmethandiphosphonat;
- α-Amino-(4-hydroxybenzyliden)-Diphosphonat.

6. Zusammensetzung nach einem der Ansprüche 1 bis 5, wobei der Hemmer der Farnesylpyrophosphatsynthase die Zoledronsäure oder ihre ionische Form, Zoledronat ist;

7. Zusammensetzung nach einem der Ansprüche 1 bis 6, wobei das Anti-HIV-Mittel ein Proteasehemmer ist, ausgewählt aus der Gruppe, bestehend aus Fosamprenavir, Lopinavir, Ritonavir, Amprenavir, Atazanavir und Indinavir.

8. Zusammensetzung nach einem der Ansprüche 1 bis 6, wobei das Anti-HIV-Mittel ein Hemmer der inversen Transkriptase ist, ausgewählt aus den Gruppe, bestehend aus Zidovudin, Lamivudin, Didanosin und Epzicom.

9. Zusammensetzung nach einem der Ansprüche 1 bis 8 für ihre Verwendung als Medikament bei der Behandlung der Nebenwirkungen der vorzeitigen Alterung, erzeugt bei einem Patienten durch eine Anti-HIV-Behandlung.

10. Zusammensetzung für ihre Anwendung nach Anspruch 9, wobei die Zusammensetzung eine Zusammensetzung zur Verabreichung auf oralem Weg oder durch Injektion ist.

11. Zusammensetzung für ihre Anwendung nach einem der Ansprüche 9 bis 10, wobei die Behandlung diejenige der Nebenwirkungen des vorzeitigen Alterns ist, erzeugt bei einem Patienten durch eine Anti-HIV-Behandlung unter Verwendung eines Proteasehemmers oder eines Hemmers der inversen Transkriptase.

12. Zusammensetzung für ihre Verwendung als Medikament bei der Behandlung von HIV, umfassend in einer beliebigen Reihenfolge die folgenden Schritte:
i. Verabreichen einer Mischung, umfassend mindestens einen Hemmer der Hydroxymethylglutarylcoenzym A (HMG-CoA)-Reduktase, wobei der Hemmer ein Molekül der Familie der Statine oder eines ihrer physiologisch annehmbaren Salze und mindestens ein Hemmer der Farnesylpyrophosphatsynthase ist, wobei der Hemmer ein Molekül der Familie der Aminobiphosphonate (NBP) oder eines ihrer physiologisch annehmbaren Salze ist
ii. Verabreichen eines Anti-HIV-Mittels, wobei das Anti-HIV-Mittel ein Proteasehemmer oder ein Hemmer der inversen Transkriptase ist,
wobei die Verabreichungen gleichzeitig, aufeinander folgend oder alternativ sind.

## Claims

1. Composition comprising:
- at least one hydroxymethylglutaryl-coenzyme A (HMG-CoA) reductase inhibitor or one of its physiologically acceptable salts, said inhibitor being a molecule of the family of statins or one of its physiologically acceptable salts.
- at least one farnesyl-pyrophosphate synthase inhibitor or one of its physiologically acceptable salts, said inhibitor being selected from the group comprising a molecule of the aminobisphosphonate (NBP) family or one of its physiologically acceptable salts, and
- at least one anti-HIV agent, said anti-HIV agent being a protease inhibitor or a reverse transcriptase inhibitor.

2. Composition according to claim 1 wherein the HMG-CoA reductase inhibitor is a hydrosoluble statin.

3. Composition according to claim 1 wherein the HMG-CoA reductase inhibitor is a liposoluble statin.

4. Composition according to claim 1 wherein the HMG-CoA reductase inhibitor is selected from the group comprising atorvastatin, simvastatin, pravastatin, rivastatin, mevastatin (or compactin), fluindostatin, velostatin, fluvastatin, dalvastatin, cerivastatin, pentostatin, rosuvastatin, lovastatin, pitavastatin, or their physiologically acceptable salts.

5. Composition according to any one of claims 1 to 4 wherein the aminobiphosphonate can be selected from:
- alendronic acid or its ionic form, alendronate;
- clodronic acid or its ionic form, clodronate;
- etidronic acid or its ionic form, etidronate;
- ibandronic acid or its ionic form, ibandronate;
- medronic acid or its ionic form, medronate;
- neridronic acid or its ionic form, neridronate;
- olpadronic acid or its ionic form, olpadronate;
- pamidronic acid or its ionic form, pamidronate;
- risedronic acid or its ionic form, risedronate;
- tiludronic acid or its ionic form, tiludronate;
- zoledronic (or zolendronic) acid or its ionic form, zoledronate (or zolendronate);
- 4-N,N-dimethylaminomethane diphosphonic acid or its ionic form, dimethylaminomethanediphosphonate;
- α-amino-(4-hydroxybenzylidene) diphosphonate.

6. Composition according to any one of claims 1 to 5, wherein the farnesyl-pyrophosphate synthase inhibitor is zoledronic acid or its ionic form, zoledronate.

7. Composition according to any one of claims 1 to 6, wherein the anti-HIV agent is a protease inhibitor selected from the group comprising fosamprenavir, lopinavir, ritonavir, amprenavir, atazanavir and indinavir.

8. Composition according to any one of claims 1 to 6 wherein the anti-HIV agent is a reverse transcriptase inhibitor selected from the group comprising zidovudine, lamivudine, didanosine and epzicom.

9. Composition according to any one of claims 1 to 8 for its use as medicament in the treatment of side effects of premature aging caused in a patient by an anti-HIV treatment.

10. Composition for its use according to claim 9, wherein the composition is a composition for oral or injection administration.

11. Composition for its use according to any one of claims 9 to 10, wherein the treatment is the treatment of side effects of premature aging caused in a patient by an anti-HIV treatment using a protease inhibitor or a reverse transcriptase inhibitor.

12. Composition for its use as a medicament in the treatment of HIV comprising in any order, the following steps:
i. administration of a mixture comprising at least one hydroxymethylglutaryl-coenzyme A (HMG-CoA) reductase inhibitor, said inhibitor being a molecule of the family of statins or one of its physiologically acceptable salts, and at least one farnesyl-pyrophosphate synthase inhibitor, said the farnesyl-pyrophosphate synthase inhibitor being selected from the group comprising a molecule of the aminobisphosphonate (NBP) family or one of its physiologically acceptable salts
ii. administration of an anti-HIV agent, said anti-HIV agent being a protease inhibitor or a reverse transcriptase inhibitor,
wherein the administrations are concomitant, successive or alternative.
